# EUROPEAN PATENT SPECIFICATION

(11) **EP 3 658 570 B1**
(45) Date of publication and mention of the grant of the patent: **01.12.2021**
(21) Application number: 18837280.9
(22) Date of filing: 19.07.2018
(51) Int. Cl.: C12N 15/113, C07K 14/00, A61K 31/7088, A61K 9/00

(54) **TYROSINASE ANTISENSE OLIGONUCLEOTIDES**
TYROSINASE-ANTISENSE-OLIGONUKLEOTIDE
OLIGONUCLÉOTIDES ANTISENS DE LA TYROSINASE

(30) Priority: 24.07.2017 KR 20170093605; 07.12.2017 KR 20170167558
(43) Date of publication of application: 03.06.2020
(73) Proprietor: Olipass Corporation, Yongin-si, Gyeonggi-do 17105 (KR)
(72) Inventor: JUNG, Daram, Hwaseong-si Gyeonggi-do 18477 (KR); PARK, Hye Mi, Seoul 04374 (KR); HAN, Seon-Young, Chuncheon-si Gangwon-do 24336 (KR); KIM, Soyoung, Seoul 08558 (KR)
(74) Representative: Boynton, Juliette Alice
(86) International application number: PCT/KR2018/008143
(87) International publication number: WO 2019/022434

(56) References cited:
- WO-A1-2010/123983
- WO-A1-2018/122610
- WO-A2-2005/060536
- WO-A2-2009/113828
- US-A1- 2004 215 006
- US-A1- 2015 274 782
- US-B1- 6 617 422
- SIWKOWSKI, A. M. et al.: "Identification and functional validation of PNAs that inhibit murine CD 40 expression by redirection of splicing", Nucleic Acids Research, vol. 32, no. 9, 17 May 2004 (2004-05-17), pages 2695-2706, XP055568402,

### Sequence Listing

<110> Jung, Daram
   Park, HyeMi
   Han, Seon-Young
   Kim, Soyoung
<120> Tyrosinase antisense oligonucleotides
<130> 2018op101pct 2018dp103
<150> KR10-2017-0093605
   <151> 2017-07-24
<150> KR10-2017-0167558
   <151> 2017-12-07
<160> 38
<170> PatentIn version 3.2
<210> 1
   <211> 13
   <212> DNA
   <213> Artificial Sequence
<220>
   <223> human tyrosinase targetted artificial sequence
<220>
   <221> misc_feature
   <222> (1)..(13)
   <223> PNA modified oligonucleotide
<220>
   <221> misc_feature
   <222> (1)
   <223> Carbamated N-terminal
<220>
   <221> misc_feature
   <222> (2)
   <223> modified base
<220>
   <221> misc_feature
   <222> (6)
   <223> modified base
<220>
   <221> misc_feature
   <222> (9)
   <223> modified base
<220>
   <221> misc_feature
   <222> (11)
   <223> modified base
<220>
   <221> misc_feature
   <222> (13)
   <223> modified base
<400> 1
   cngacnatnt ntn 13
<210> 2
   <211> 14
   <212> DNA
   <213> Artificial Sequence
<220>
   <223> human tyrosinase targetted artificial sequence
<220>
   <221> misc_feature
   <222> (1)..(14)
   <223> PNA modified oligonucleotide
<220>
   <221> misc_feature
   <222> (1)
   <223> Carbamated N-terminal
<220>
   <221> misc_feature
   <222> (2)
   <223> modified base
<220>
   <221> misc_feature
   <222> (3)
   <223> modified base
<220>
   <221> misc_feature
   <222> (5)
   <223> modified base
<220>
   <221> misc_feature
   <222> (8)
   <223> modified base
<220>
   <221> misc_feature
   <222> (12)
   <223> modified base
<220>
   <221> misc_feature
   <222> (13)
   <223> modified base
<400> 2
   anngncantc tnnc 14
<210> 3
   <211> 13
   <212> DNA
   <213> Artificial Sequence
<220>
   <223> human tyrosinase targetted artificial sequence
<220>
   <221> misc_feature
   <222> (1)..(13)
   <223> PNA modified oligonucleotide
<220>
   <221> misc_feature
   <222> (1)
   <223> Acetylated N-terminal
<220>
   <221> misc_feature
   <222> (2)
   <223> modified base
<220>
   <221> misc_feature
   <222> (6)
   <223> modified base
<220>
   <221> misc_feature
   <222> (9)
   <223> modified base
<220>
   <221> misc_feature
   <222> (11)
   <223> modified base
<220>
   <221> misc_feature
   <222> (13)
   <223> modified base
<400> 3
   cngacnatnt ntn 13
<210> 4
   <211> 13
   <212> DNA
   <213> Artificial Sequence
<220>
   <223> human tyrosinase targetted artificial sequence
<220>
   <221> misc_feature
   <222> (1)..(13)
   <223> PNA modified oligonucleotide
<220>
   <221> misc_feature
   <222> (1)
   <223> Acetylated N-terminal
<220>
   <221> misc_feature
   <222> (2)
   <223> modified base
<220>
   <221> misc_feature
   <222> (6)
   <223> modified base
<220>
   <221> misc_feature
   <222> (9)
   <223> modified base
<220>
   <221> misc_feature
   <222> (11)
   <223> modified base
<220>
   <221> misc_feature
   <222> (13)
   <223> modified base
<400> 4
   cngacnatnt ntn 13
<210> 5
   <211> 13
   <212> DNA
   <213> Artificial Sequence
<220>
   <223> human tyrosinase targetted artificial sequence
<220>
   <221> misc_feature
   <222> (1)..(13)
   <223> PNA modified oligonucleotide
<220>
   <221> misc_feature
   <222> (1)
   <223> Acetylated N-terminal
<220>
   <221> misc_feature
   <222> (2)
   <223> modified base
<220>
   <221> misc_feature
   <222> (6)
   <223> modified base
<220>
   <221> misc_feature
   <222> (9)
   <223> modified base
<220>
   <221> misc_feature
   <222> (11)
   <223> modified base
<220>
   <221> misc_feature
   <222> (13)
   <223> modified base
<400> 5
   cngacnatnt ntn 13
<210> 6
   <211> 13
   <212> DNA
   <213> Artificial Sequence
<220>
   <223> human tyrosinase targetted artificial sequence
<220>
   <221> misc_feature
   <222> (1)..(13)
   <223> PNA modified oligonucleotide
<220>
   <221> misc_feature
   <222> (1)
   <223> Mono-alkylated N-terminal
<220>
   <221> misc_feature
   <222> (2)
   <223> modified base
<220>
   <221> misc_feature
   <222> (6)
   <223> modified base
<220>
   <221> misc_feature
   <222> (9)
   <223> modified base
<220>
   <221> misc_feature
   <222> (11)
   <223> modified base
<220>
   <221> misc_feature
   <222> (13)
   <223> modified base
<400> 6
   cngacnatnt ntn 13
<210> 7
   <211> 13
   <212> DNA
   <213> Artificial Sequence
<220>
   <223> human tyrosinase targetted artificial sequence
<220>
   <221> misc_feature
   <222> (1)..(13)
   <223> PNA modified oligonucleotide
<220>
   <221> misc_feature
   <222> (1)
   <223> Mono-alkylated N-terminal
<220>
   <221> misc_feature
   <222> (2)
   <223> modified base
<220>
   <221> misc_feature
   <222> (6)
   <223> modified base
<220>
   <221> misc_feature
   <222> (9)
   <223> modified base
<220>
   <221> misc_feature
   <222> (11)
   <223> modified base
<220>
   <221> misc_feature
   <222> (13)
   <223> modified base
<400> 7
   cngacnatnt ntn 13
<210> 8
   <211> 13
   <212> DNA
   <213> Artificial Sequence
<220>
   <223> human tyrosinase targetted artificial sequence
<220>
   <221> misc_feature
   <222> (1) .. (13)
   <223> PNA modified oligonucleotide
<220>
   <221> misc_feature
   <222> (1)
   <223> Carbamated N-terminal
<220>
   <221> misc_feature
   <222> (2)
   <223> modified base
<220>
   <221> misc_feature
   <222> (6)
   <223> modified base
<220>
   <221> misc_feature
   <222> (9)
   <223> modified base
<220>
   <221> misc_feature
   <222> (11)
   <223> modified base
<220>
   <221> misc_feature
   <222> (13)
   <223> modified base
<400> 8
   cngacnatnt ntn 13
<210> 9
   <211> 13
   <212> DNA
   <213> Artificial Sequence
<220>
   <223> human tyrosinase targetted artificial sequence
<220>
   <221> misc_feature
   <222> (1)..(13)
   <223> PNA modified oligonucleotide
<220>
   <221> misc_feature
   <222> (1)
   <223> Sulfonylated N-terminal
<220>
   <221> misc_feature
   <222> (2)
   <223> modified base
<220>
   <221> misc_feature
   <222> (6)
   <223> modified base
<220>
   <221> misc_feature
   <222> (9)
   <223> modified base
<220>
   <221> misc_feature
   <222> (11)
   <223> modified base
<220>
   <221> misc_feature
   <222> (13)
   <223> modified base
<400> 9
   cngacnatnt ntn 13
<210> 10
   <211> 13
   <212> DNA
   <213> Artificial Sequence
<220>
   <223> human tyrosinase targetted artificial sequence
<220>
   <221> misc_feature
   <222> (1)..(13)
   <223> PNA modified oligonucleotide
<220>
   <221> misc_feature
   <222> (1)
   <223> Acetylated N-terminal
<220>
   <221> misc_feature
   <222> (2)
   <223> modified base
<220>
   <221> misc_feature
   <222> (6)
   <223> modified base
<220>
   <221> misc_feature
   <222> (9)
   <223> modified base
<220>
   <221> misc_feature
   <222> (11)
   <223> modified base
<220>
   <221> misc_feature
   <222> (13)
   <223> modified base
<400> 10
   cngacnatnt ntn 13
<210> 11
   <211> 13
   <212> DNA
   <213> Artificial Sequence
<220>
   <223> human tyrosinase targetted artificial sequence
<220>
   <221> misc_feature
   <222> (1)..(13)
   <223> PNA modified oligonucleotide
<220>
   <221> misc_feature
   <222> (1)
   <223> Acetylated N-terminal
<220>
   <221> misc_feature
   <222> (2)
   <223> modified base
<220>
   <221> misc_feature
   <222> (6)
   <223> modified base
<220>
   <221> misc_feature
   <222> (9)
   <223> modified base
<220>
   <221> misc_feature
   <222> (11)
   <223> modified base
<220>
   <221> misc_feature
   <222> (13)
   <223> modified base
<400> 11
   cngacnatnt ntn 13
<210> 12
   <211> 13
   <212> DNA
   <213> Artificial Sequence
<220>
   <223> human tyrosinase targetted artificial sequence
<220>
   <221> misc_feature
   <222> (1)..(13)
   <223> PNA modified oligonucleotide
<220>
   <221> misc_feature
   <222> (1)
   <223> Carbamated N-terminal
<220>
   <221> misc_feature
   <222> (2)
   <223> modified base
<220>
   <221> misc_feature
   <222> (6)
   <223> modified base
<220>
   <221> misc_feature
   <222> (9)
   <223> modified base
<220>
   <221> misc_feature
   <222> (11)
   <223> modified base
<220>
   <221> misc_feature
   <222> (13)
   <223> modified base
<400> 12
   cngacnatnt ntn 13
<210> 13
   <211> 13
   <212> DNA
   <213> Artificial Sequence
<220>
   <223> human tyrosinase targetted artificial sequence
<220>
   <221> misc_feature
   <222> (1)..(13)
   <223> PNA modified oligonucleotide
<220>
   <221> misc_feature
   <222> (1)
   <223> Acetylated N-terminal
<220>
   <221> misc_feature
   <222> (2)
   <223> modified base
<220>
   <221> misc_feature
   <222> (6)
   <223> modified base
<220>
   <221> misc_feature
   <222> (9)
   <223> modified base
<220>
   <221> misc_feature
   <222> (11)
   <223> modified base
<220>
   <221> misc_feature
   <222> (13)
   <223> modified base
<400> 13
   cngacnatnt ntn 13
<210> 14
   <211> 13
   <212> DNA
   <213> Artificial Sequence
<220>
   <223> human tyrosinase targetted artificial sequence
<220>
   <221> misc_feature
   <222> (1)..(13)
   <223> PNA modified oligonucleotide
<220>
   <221> misc_feature
   <222> (1)
   <223> Acetylated N-terminal
<220>
   <221> misc_feature
   <222> (2)
   <223> modified base
<220>
   <221> misc_feature
   <222> (6)
   <223> modified base
<220>
   <221> misc_feature
   <222> (9)
   <223> modified base
<220>
   <221> misc_feature
   <222> (11)
   <223> modified base
<220>
   <221> misc_feature
   <222> (13)
   <223> modified base
<400> 14
   cngacnatnt ntn 13
<210> 15
   <211> 13
   <212> DNA
   <213> Artificial Sequence
<220>
   <223> human tyrosinase targetted artificial sequence
<220>
   <221> misc_feature
   <222> (1)..(13)
   <223> PNA modified oligonucleotide
<220>
   <221> misc_feature
   <222> (1)
   <223> Mono-alkylated N-terminal
<220>
   <221> misc_feature
   <222> (2)
   <223> modified base
<220>
   <221> misc_feature
   <222> (6)
   <223> modified base
<220>
   <221> misc_feature
   <222> (9)
   <223> modified base
<220>
   <221> misc_feature
   <222> (11)
   <223> modified base
<220>
   <221> misc_feature
   <222> (13)
   <223> modified base
<400> 15
   cngacnatnt ntn 13
<210> 16
   <211> 13
   <212> DNA
   <213> Artificial Sequence
<220>
   <223> human tyrosinase targetted artificial sequence
<220>
   <221> misc_feature
   <222> (1)..(13)
   <223> PNA modified oligonucleotide
<220>
   <221> misc_feature
   <222> (1)
   <223> Mono-arylated N-terminal
<220>
   <221> misc_feature
   <222> (2)
   <223> modified base
<220>
   <221> misc_feature
   <222> (6)
   <223> modified base
<220>
   <221> misc_feature
   <222> (9)
   <223> modified base
<220>
   <221> misc_feature
   <222> (11)
   <223> modified base
<220>
   <221> misc_feature
   <222> (13)
   <223> modified base
<400> 16
   cngacnatnt ntn 13
<210> 17
   <211> 13
   <212> DNA
   <213> Artificial Sequence
<220>
   <223> human tyrosinase targetted artificial sequence
<220>
   <221> misc_feature
   <222> (1)..(13)
   <223> PNA modified oligonucleotide
<220>
   <221> misc_feature
   <222> (1)
   <223> Ureated N-terminal
<220>
   <221> misc_feature
   <222> (2)
   <223> modified base
<220>
   <221> misc_feature
   <222> (6)
   <223> modified base
<220>
   <221> misc_feature
   <222> (9)
   <223> modified base
<220>
   <221> misc_feature
   <222> (11)
   <223> modified base
<220>
   <221> misc_feature
   <222> (13)
   <223> modified base
<400> 17
   cngacnatnt ntn 13
<210> 18
   <211> 13
   <212> DNA
   <213> Artificial Sequence
<220>
   <223> human tyrosinase targetted artificial sequence
<220>
   <221> misc_feature
   <222> (1)..(13)
   <223> PNA modified oligonucleotide
<220>
   <221> misc_feature
   <222> (1)
   <223> Acetylated N-terminal
<220>
   <221> misc_feature
   <222> (2)
   <223> modified base
<220>
   <221> misc_feature
   <222> (6)
   <223> modified base
<220>
   <221> misc_feature
   <222> (9)
   <223> modified base
<220>
   <221> misc_feature
   <222> (11)
   <223> modified base
<220>
   <221> misc_feature
   <222> (13)
   <223> modified base
<400> 18
   cngacnatnt ntn 13
<210> 19
   <211> 13
   <212> DNA
   <213> Artificial Sequence
<220>
   <223> human tyrosinase targetted artificial sequence
<220>
   <221> misc_feature
   <222> (1)..(13)
   <223> PNA modified oligonucleotide
<220>
   <221> misc_feature
   <222> (1)
   <223> Acetylated N-terminal
<220>
   <221> misc_feature
   <222> (2)
   <223> modified base
<220>
   <221> misc_feature
   <222> (6)
   <223> modified base
<220>
   <221> misc_feature
   <222> (9)
   <223> modified base
<220>
   <221> misc_feature
   <222> (11)
   <223> modified base
<220>
   <221> misc_feature
   <222> (13)
   <223> modified base
<400> 19
   cngacnatnt ntn 13
<210> 20
   <211> 13
   <212> DNA
   <213> Artificial Sequence
<220>
   <223> human tyrosinase targetted artificial sequence
<220>
   <221> misc_feature
   <222> (1)..(13)
   <223> PNA modified oligonucleotide
<220>
   <221> misc_feature
   <222> (1)
   <223> Carbamated N-terminal
<220>
   <221> misc_feature
   <222> (2)
   <223> modified base
<220>
   <221> misc_feature
   <222> (5)
   <223> modified base
<220>
   <221> misc_feature
   <222> (7)
   <223> modified base
<220>
   <221> misc_feature
   <222> (9)
   <223> modified base
<220>
   <221> misc_feature
   <222> (11)
   <223> modified base
<400> 20
   cnganantnt nta 13
<210> 21
   <211> 13
   <212> DNA
   <213> Artificial Sequence
<220>
   <223> human tyrosinase targetted artificial sequence
<220>
   <221> misc_feature
   <222> (1)..(13)
   <223> PNA modified oligonucleotide
<220>
   <221> misc_feature
   <222> (1)
   <223> Carbamated N-terminal
<220>
   <221> misc_feature
   <222> (3)
   <223> modified base
<220>
   <221> misc_feature
   <222> (6)
   <223> modified base
<220>
   <221> misc_feature
   <222> (9)
   <223> modified base
<220>
   <221> misc_feature
   <222> (11)
   <223> modified base
<220>
   <221> misc_feature
   <222> (13)
   <223> modified base
<400> 21
   ctnacnatnt ntn 13
<210> 22
   <211> 13
   <212> DNA
   <213> Artificial Sequence
<220>
   <223> human tyrosinase targetted artificial sequence
<220>
   <221> misc_feature
   <222> (1) ..(13)
   <223> PNA modified oligonucleotide
<220>
   <221> misc_feature
   <222> (1)
   <223> Carbamated N-terminal
<220>
   <221> misc_feature
   <222> (2)
   <223> modified base
<220>
   <221> misc_feature
   <222> (6)
   <223> modified base
<220>
   <221> misc_feature
   <222> (9)
   <223> modified base
<220>
   <221> misc_feature
   <222> (11)
   <223> modified base
<220>
   <221> misc_feature
   <222> (13)
   <223> modified base
<400> 22
   cngatnatnt ntn 13
<210> 23
   <211> 13
   <212> DNA
   <213> Artificial Sequence
<220>
   <223> human tyrosinase targetted artificial sequence
<220>
   <221> misc_feature
   <222> (1)..(13)
   <223> PNA modified oligonucleotide
<220>
   <221> misc_feature
   <222> (1)
   <223> Carbamated N-terminal
<220>
   <221> misc_feature
   <222> (2)
   <223> modified base
<220>
   <221> misc_feature
   <222> (6)
   <223> modified base
<220>
   <221> misc_feature
   <222> (9)
   <223> modified base
<220>
   <221> misc_feature
   <222> (11)
   <223> modified base
<220>
   <221> misc_feature
   <222> (13)
   <223> modified base
<400> 23
   tngacnatnt ntn 13
<210> 24
   <211> 14
   <212> DNA
   <213> Artificial Sequence
<220>
   <223> human tyrosinase targetted artificial sequence
<220>
   <221> misc_feature
   <222> (1)..(14)
   <223> PNA modified oligonucleotide
<220>
   <221> misc_feature
   <222> (1)
   <223> Carbamated N-terminal
<220>
   <221> misc_feature
   <222> (3)
   <223> modified base
<220>
   <221> misc_feature
   <222> (6)
   <223> modified base
<220>
   <221> misc_feature
   <222> (7)
   <223> modified base
<220>
   <221> misc_feature
   <222> (10)
   <223> modified base
<220>
   <221> misc_feature
   <222> (12)
   <223> modified base
<220>
   <221> misc_feature
   <222> (14)
   <223> modified base
<400> 24
   tcngannatn tntn 14
<210> 25
   <211> 14
   <212> DNA
   <213> Artificial Sequence
<220>
   <223> human tyrosinase targetted artificial sequence
<220>
   <221> misc_feature
   <222> (1)..(14)
   <223> PNA modified oligonucleotide
<220>
   <221> misc_feature
   <222> (1)
   <223> Carbamated N-terminal
<220>
   <221> misc_feature
   <222> (2)
   <223> modified base
<220>
   <221> misc_feature
   <222> (6)
   <223> modified base
<220>
   <221> misc_feature
   <222> (9)
   <223> modified base
<220>
   <221> misc_feature
   <222> (11)
   <223> modified base
<220>
   <221> misc_feature
   <222> (13)
   <223> modified base
<400> 25
   cngacnatnt ntnc 14
<210> 26
   <211> 13
   <212> DNA
   <213> Artificial Sequence
<220>
   <223> human tyrosinase targetted artificial sequence
<220>
   <221> misc_feature
   <222> (1)..(13)
   <223> PNA modified oligonucleotide
<220>
   <221> misc_feature
   <222> (1)
   <223> Carbamated N-terminal
<220>
   <221> misc_feature
   <222> (2)
   <223> modified base
<220>
   <221> misc_feature
   <222> (5)
   <223> modified base
<220>
   <221> misc_feature
   <222> (8)
   <223> modified base
<220>
   <221> misc_feature
   <222> (10)
   <223> modified base
<220>
   <221> misc_feature
   <222> (12)
   <223> modified base
<400> 26
   anacnatntn tnc 13
<210> 27
   <211> 15
   <212> DNA
   <213> Artificial Sequence
<220>
   <223> human tyrosinase targetted artificial sequence
<220>
   <221> misc_feature
   <222> (1)..(15)
   <223> PNA modified oligonucleotide
<220>
   <221> misc_feature
   <222> (1)
   <223> Carbamated N-terminal
<220>
   <221> misc_feature
   <222> (2)
   <223> modified base
<220>
   <221> misc_feature
   <222> (5)
   <223> modified base
<220>
   <221> misc_feature
   <222> (8)
   <223> modified base
<220>
   <221> misc_feature
   <222> (10)
   <223> modified base
<220>
   <221> misc_feature
   <222> (12)
   <223> modified base
<220>
   <221> misc_feature
   <222> (14)
   <223> modified base
<400> 27
   anacnatntn tncna 15
<210> 28
   <211> 17
   <212> DNA
   <213> Artificial Sequence
<220>
   <223> human tyrosinase targetted artificial sequence
<220>
   <221> misc_feature
   <222> (1)..(17)
   <223> PNA modified oligonucleotide
<220>
   <221> misc_feature
   <222> (1)
   <223> Carbamated N-terminal
<220>
   <221> misc_feature
   <222> (2)
   <223> modified base
<220>
   <221> misc_feature
   <222> (5)
   <223> modified base
<220>
   <221> misc_feature
   <222> (8)
   <223> modified base
<220>
   <221> misc_feature
   <222> (12)
   <223> modified base
<220>
   <221> misc_feature
   <222> (14)
   <223> modified base
<220>
   <221> misc_feature
   <222> (15)
   <223> modified base
<220>
   <221> misc_feature
   <222> (17)
   <223> modified base
<400> 28
   anagncantc tntnnan 17
<210> 29
   <211> 17
   <212> DNA
   <213> Artificial Sequence
<220>
   <223> human tyrosinase targetted artificial sequence
<220>
   <221> misc_feature
   <222> (1)..(17)
   <223> PNA modified oligonucleotide
<220>
   <221> misc_feature
   <222> (1)
   <223> Carbamated N-terminal
<220>
   <221> misc_feature
   <222> (2)
   <223> modified base
<220>
   <221> misc_feature
   <222> (5)
   <223> modified base
<220>
   <221> misc_feature
   <222> (8)
   <223> modified base
<220>
   <221> misc_feature
   <222> (12)
   <223> modified base
<220>
   <221> misc_feature
   <222> (15)
   <223> modified base
<220>
   <221> misc_feature
   <222> (16)
   <223> modified base
<400> 29
   antgnctntc tntanna 17
<210> 30
   <211> 14
   <212> DNA
   <213> Artificial Sequence
<220>
   <223> human tyrosinase targetted artificial sequence
<220>
   <221> misc_feature
   <222> (1)..(14)
   <223> PNA modified oligonucleotide
<220>
   <221> misc_feature
   <222> (1)
   <223> Carbamated N-terminal
<220>
   <221> misc_feature
   <222> (2)
   <223> modified base
<220>
   <221> misc_feature
   <222> (5)
   <223> modified base
<220>
   <221> misc_feature
   <222> (9)
   <223> modified base
<220>
   <221> misc_feature
   <222> (11)
   <223> modified base
<220>
   <221> misc_feature
   <222> (12)
   <223> modified base
<220>
   <221> misc_feature
   <222> (14)
   <223> modified base
<400> 30
   gncantctnt nnan 14
<210> 31
   <211> 19
   <212> DNA
   <213> Artificial Sequence
<220>
   <223> human tyrosinase targetted artificial sequence
<220>
   <221> misc_feature
   <222> (1)..(19)
   <223> PNA modified oligonucleotide
<220>
   <221> misc_feature
   <222> (1)
   <223> Carbamated N-terminal
<220>
   <221> misc_feature
   <222> (2)
   <223> modified base
<220>
   <221> misc_feature
   <222> (5)
   <223> modified base
<220>
   <221> misc_feature
   <222> (8)
   <223> modified base
<220>
   <221> misc_feature
   <222> (12)
   <223> modified base
<220>
   <221> misc_feature
   <222> (14)
   <223> modified base
<220>
   <221> misc_feature
   <222> (16)
   <223> modified base
<220>
   <221> misc_feature
   <222> (18)
   <223> modified base
<400> 31
   anagncantc tntncnana 19
<210> 32
   <211> 13
   <212> DNA
   <213> Artificial Sequence
<220>
   <223> human tyrosinase targetted artificial sequence
<220>
   <221> misc_feature
   <222> (1)..(13)
   <223> PNA modified oligonucleotide
<220>
   <221> misc_feature
   <222> (1)
   <223> Carbamated N-terminal
<220>
   <221> misc_feature
   <222> (2)
   <223> modified base
<220>
   <221> misc_feature
   <222> (6)
   <223> modified base
<220>
   <221> misc_feature
   <222> (8)
   <223> modified base
<220>
   <221> misc_feature
   <222> (10)
   <223> modified base
<220>
   <221> misc_feature
   <222> (12)
   <223> modified base
<400> 32
   antctntncn ana 13
<210> 33
   <211> 19
   <212> DNA
   <213> Artificial Sequence
<220>
   <223> human tyrosinase targetted artificial sequence
<220>
   <221> misc_feature
   <222> (1)..(19)
   <223> PNA modified oligonucleotide
<220>
   <221> misc_feature
   <222> (1)
   <223> Carbamated N-terminal
<220>
   <221> misc_feature
   <222> (2)
   <223> modified base
<220>
   <221> misc_feature
   <222> (5)
   <223> modified base
<220>
   <221> misc_feature
   <222> (8)
   <223> modified base
<220>
   <221> misc_feature
   <222> (12)
   <223> modified base
<220>
   <221> misc_feature
   <222> (14)
   <223> modified base
<220>
   <221> misc_feature
   <222> (16)
   <223> modified base
<220>
   <221> misc_feature
   <222> (18)
   <223> modified base
<400> 33
   anagncantc tntncnana 19
<210> 34
   <211> 18
   <212> DNA
   <213> Artificial Sequence
<220>
   <223> human tyrosinase targetted artificial sequence
<220>
   <221> misc_feature
   <222> (1)..(18)
   <223> PNA modified oligonucleotide
<220>
   <221> misc_feature
   <222> (1)
   <223> Carbamated N-terminal
<220>
   <221> misc_feature
   <222> (2)
   <223> modified base
<220>
   <221> misc_feature
   <222> (5)
   <223> modified base
<220>
   <221> misc_feature
   <222> (8)
   <223> modified base
<220>
   <221> misc_feature
   <222> (15)
   <223> modified base
<220>
   <221> misc_feature
   <222> (17)
   <223> modified base
<400> 34
   gntanagnca atctntnc 18
<210> 35
   <211> 15
   <212> DNA
   <213> Artificial Sequence
<220>
   <223> human tyrosinase targetted artificial sequence
<220>
   <221> misc_feature
   <222> (1)..(15)
   <223> PNA modified oligonucleotide
<220>
   <221> misc_feature
   <222> (1)
   <223> Carbamated N-terminal
<220>
   <221> misc_feature
   <222> (2)
   <223> modified base
<220>
   <221> misc_feature
   <222> (4)
   <223> modified base
<220>
   <221> misc_feature
   <222> (8)
   <223> modified base
<220>
   <221> misc_feature
   <222> (13)
   <223> modified base
<400> 35
   gntncagnca atntg 15
<210> 36
   <211> 15
   <212> DNA
   <213> Artificial Sequence
<220>
   <223> human tyrosinase targetted artificial sequence
<220>
   <221> misc_feature
   <222> (1)..(15)
   <223> PNA modified oligonucleotide
<220>
   <221> misc_feature
   <222> (1)
   <223> Carbamated N-terminal
<220>
   <221> misc_feature
   <222> (2)
   <223> modified base
<220>
   <221> misc_feature
   <222> (4)
   <223> modified base
<220>
   <221> misc_feature
   <222> (7)
   <223> modified base
<220>
   <221> misc_feature
   <222> (10)
   <223> modified base
<220>
   <221>misc_feature
   <222> (13)
   <223> modified base
<400> 36
   gntncanacn atntg 15
<210> 37
   <211> 15
   <212> DNA
   <213> Artificial Sequence
<220>
   <223> human tyrosinase targetted artificial sequence
<220>
   <221> misc_feature
   <222> (1)..(15)
   <223> PNA modified oligonucleotide
<220>
   <221> misc_feature
   <222> (1)
   <223> Carbamated N-terminal
<220>
   <221> misc_feature
   <222> (2)
   <223> modified base
<220>
   <221> misc_feature
   <222> (4)
   <223> modified base
<220>
   <221> misc_feature
   <222> (8)
   <223> modified base
<220>
   <221> misc_feature
   <222> (10)
   <223> modified base
<220>
   <221> misc_feature
   <222> (13)
   <223> modified base
<400> 37
   gntncagncn atntg 15
<210> 38
   <211> 13
   <212> DNA
   <213> Artificial Sequence
<220>
   <223> mouse tyrosinase targetted artificial sequence
<220>
   <221> misc_feature
   <222> (1)..(13)
   <223> PNA modified oligonucleotide
<220>
   <221> misc_feature
   <222> (1)
   <223> Carbamated N-terminal
<220>
   <221> misc_feature
   <222> (2)
   <223> modified base
<220>
   <221> misc_feature
   <222> (4)
   <223> modified base
<220>
   <221> misc_feature
   <222> (7)
   <223> modified base
<220>
   <221> misc_feature
   <222> (9)
   <223> modified base
<220>
   <221> misc_feature
   <222> (11)
   <223> modified base
<400> 38
   cnantgntnt ntg 13

## Description

### Technical Field

The present invention relates to peptide nucleic acid derivatives complementarily targeting the human tyrosinase pre-mRNA for improvement of skin pigmentation mediated by tyrosinase.

### Background Art

Melanin collectively refers to dark natural and polymeric pigments produced in living organisms. There are three types of melanin, i.e., eumelanin, pheomelanin and neuromelanin. Eumelanin and pheomelanin are synthesized in the skin. In the meantime, Neuromelanin is a melanin found in the brain, and its physiological functions have yet to be elucidated.

Melanin in the skin effectively absorbs UV (ultra-violet) irradiation, and protects animals from the UV (ultra-violet) irradiation in natural sunlight. Thus sun tanning is a natural process that animals protect themselves from the UV irradiation in sunlight.

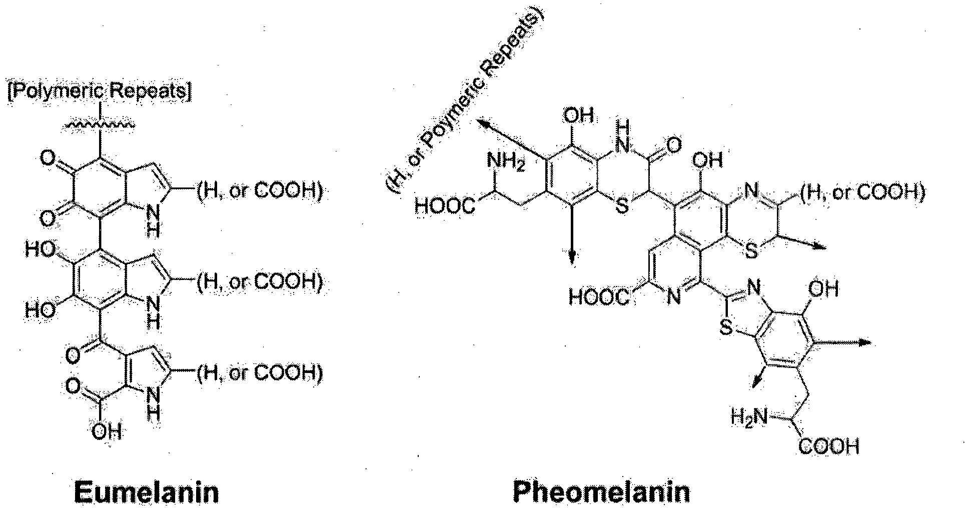

The amino acid tyrosine polymerizes to eumelanin following a series of complex reactions in the presence of tyrosinase. Eumelanin refers to polymers of 5,6-dihydroxyindole (DHI) and 5,6-dihydroxyindole-2-carboxylic acid (DHICA). Pheomelanin is formed when tyrosine is polymerized by tyrosinase in the presence of cysteine or glutathione.

Scheme 1 summarizes the reactions involving **TYR** (tyrosinase), **TYRP-1** (tyrosinase-related protein 1, i.e. DHICA oxidase) and **TYRP-2** (tyrosinase-related protein 2, i.e. DOPAchrome tautomerase) during the biosynthesis of eumelanin and pheomelanin. [Int. J. Mol. Sci. vol 10, 4066-4087 (2009)]

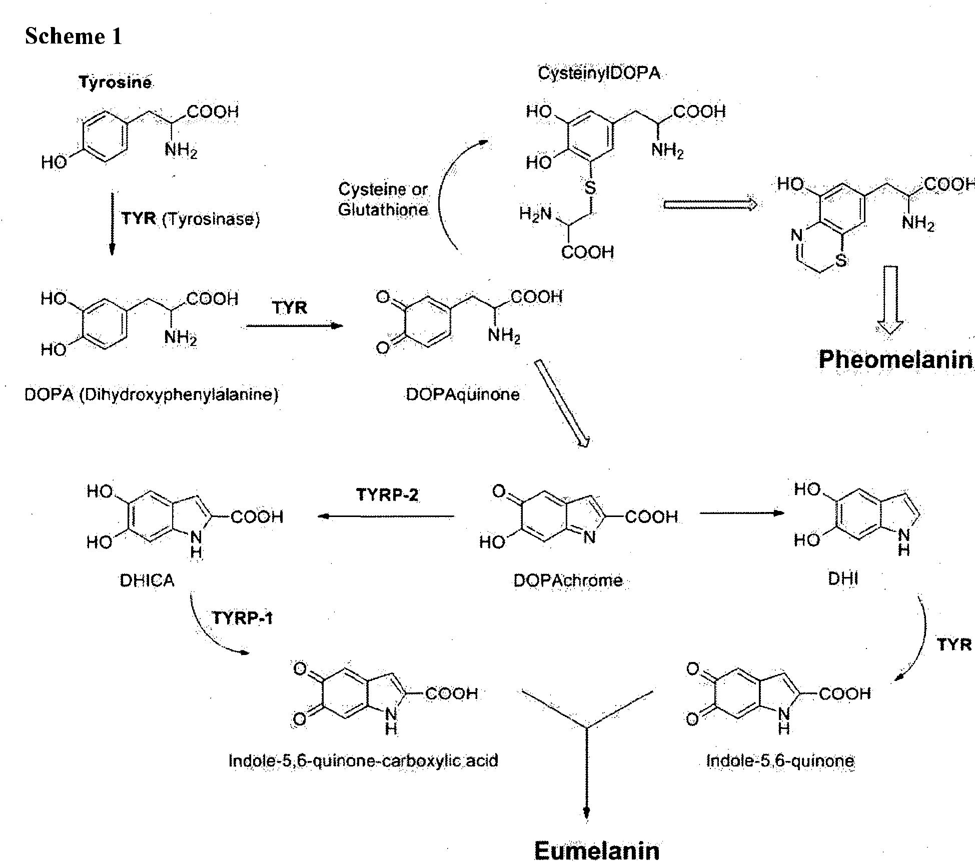

Given that tyrosinase (TYR) plays central roles in the melanin biosynthesis in the skin, TYR inhibitors have been used or developed as cosmetic ingredients to reduce excessive skin pigmentation, i.e. hyper-pigmentation. TYR inhibitors were reviewed for their effects against melanogenesis in several articles. [Int. J. Cosmetic Sci. vol 33, 210-221 (2011); Int. J. Mol. Sci. vol 10, 2440-2475 (2009)]

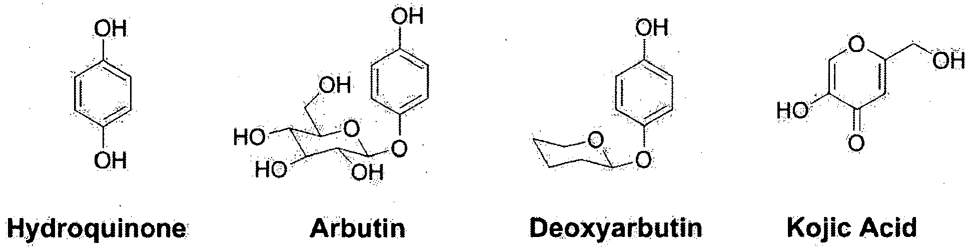

Hydroquinone (1,4-dihydroxybenzene) has been used to topically treat hyper-pigmentation for decades. [JAMA vol 194(9), 965-967 (1965)] Hydroquinone is known to inhibit the TYR activity by binding to the active site of tyrosinase. In 2006, the US FDA revoked the previous approval of all the OTC (over-the-counter) products containing hydroquinone due to safety concerns including potential carcinogenicity. Hydroquinone induces ROS (reactive oxygen species) damaging membrane lipids and proteins including tyrosinase. The oxidative damage of membrane lipids permanently deprives melanocytes.

Arbutin (hydroquinone-O-β-D-glucopyranoside) is a glycosylated hydroquinone extracted from the bearberry plant. Arbutin is also found in wheat and pear skins. Like hydroquinone, arbutin inhibits melanogenesis by competitively binding to the active site of TYR. *[*J. Pharmacol. Exp. Ther. vol 276(2), 765-769 (1996)] Arbutin is less cytotoxic to melanocytes than hydroquinone.

Deoxyarbutin is a synthetic derivative of arbutin, and is comparable to hydroquinone and arbutin in the TYR inhibitory activity. Deoxyarbutin is less cytotoxic to melanocytes than arbutin and hydroquinone as well. [J. Dermatol. Sci. vol 55(3), 179-184 (2009)]. Deoxyarbutin significantly improved skin lightness in human subjects topically treated for 12 weeks. [Exp. Dermatol. vol 14(8), 601-608 (2005)]

Kojic acid is a chelation agent produced during the fungal fermentation to manufacture soybean sauce and sake, i.e. Japanese rice wine. Kojic acid has been used to preserve or change colors in food and cosmetic products. Kojic acid chelates to the copper ions in the active site of TYR to inhibit melanogenesis. It also suppresses the tautomerization of DOPAchrome to 5,6-dihydroxyindole-2-carboxylic acid. [J. Pharm. Pharmacol. vol 46(12), 982-985 (1994)]. Kojic acid has been popularly used to treat melasma, although it may induce contact dermatitis, sensitization and erythema. [J. Drugs Dermatol. vol 6(1), 32-39 (2007)]

Although there are a variety of TYR inhibitors, their IC₅₀ values against TYR are in most cases micromolar (µM). [J. Enzyme Inhibition Med. Chem. vol 32(1), 403-425 (2017)] Such IC₅₀ values are considered to be poor considering their molecular sizes, and raise a possibility of cross reactivity with other molecular target(s). Since poor inhibitory activity is translated into large trans-dermal dose, the inhibitory potency needs to be markedly improved to meet the desired trans-dermal activity against skin pigmentation, and safety as well.

Pre-mRNA: Genetic information is carried on DNA (2-deoxyribose nucleic acid). DNA is transcribed to produce pre-mRNA (pre-messenger ribonucleic acid) in the nucleus. Mammalian pre-mRNA usually consists of exons and introns, and exon and intron are inter-connected to each other as schematically provided below. Exons and introns are numbered as exemplified in the drawing below.

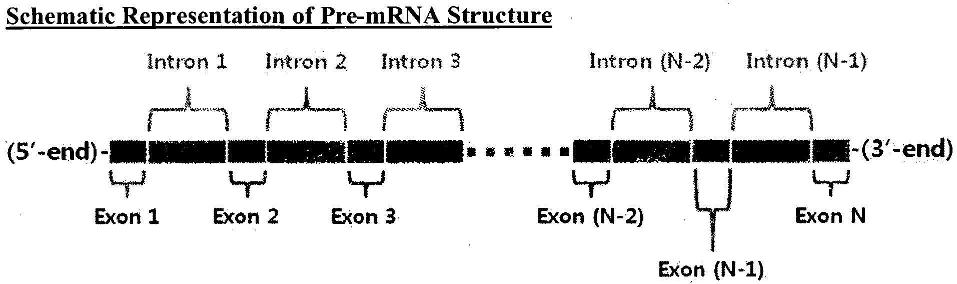

Splicing of Pre-mRNA: Pre-mRNA is processed into mRNA following deletion of introns by a series of complex reactions collectively called "splicing" which is schematically summarized in the diagram below. [Ann. Rev. Biochem. 72(1), 291-336 (2003); Nature Rev. Mol. Cell Biol. 6(5), 386-398 (2005); Nature Rev. Mol. Cell Biol. 15(2), 108-121 (2014)]

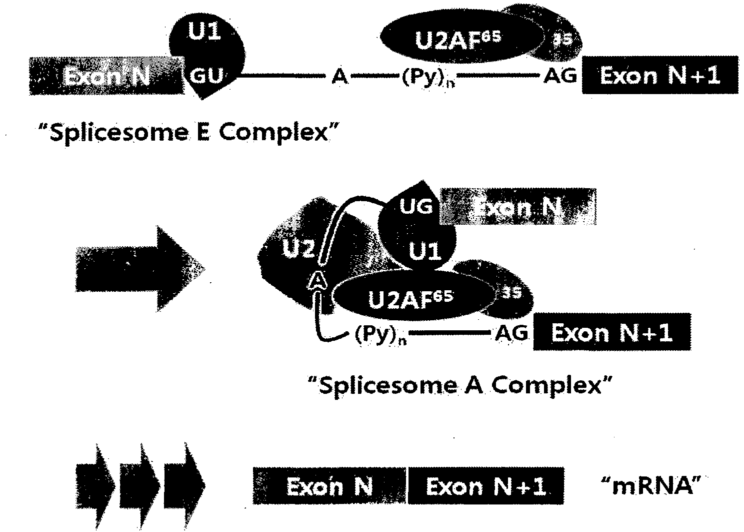

Splicing is initiated by forming "splicesome E complex" (i.e. early splicesome complex) between pre-mRNA and splicing adapter factors. In "splicesome E complex", U1 binds to the junction of exon N and intron N, and U2AF³⁵ binds to the junction of intron N and exon (N+1). Thus the junctions of exon/intron or intron/exon are critical to the formation of the early splicesome complex. "Splicesome E complex" evolves into "splicesome A complex" upon additional complexation with U2. The "splicesome A complex" undergoes a series of complex reactions to delete or splice out the intron to adjoin the neighboring exons.

Ribosomal Protein Synthesis: Proteins are encoded by DNA (2-deoxyribose nucleic acid). In response to cellular stimulation or spontaneously, DNA is transcribed to produce pre-mRNA (pre-messenger ribonucleic acid) in the nucleus. The introns of pre-mRNA are enzymatically spliced out to yield mRNA (messenger ribonucleic acid), which is then translocated into the cytoplasm. In the cytoplasm, a complex of translational machinery called ribosome binds to mRNA and carries out the protein synthesis as it scans the genetic information encoded along the mRNA. [Biochemistry vol 41, 4503-4510 (2002); Cancer Res. vol 48, 2659-2668 (1988)]

Antisense Oligonucleotide (ASO): An oligonucleotide binding to nucleic acid including DNA, mRNA and pre-mRNA in a sequence specific manner (i.e. complementarily) is called antisense oligonucleotide (ASO).

If an ASO tightly binds to an mRNA in the cytoplasm, for example, the ASO may be able to inhibit the ribosomal protein synthesis along the mRNA. ASO needs to be present within the cytoplasm in order to inhibit the ribosomal protein synthesis of its target protein.

Antisense Inhibition of Splicing: If an ASO tightly binds to a pre-mRNA in the nucleus, the ASO may be able to inhibit or modulate the splicing of pre-mRNA into mRNA. ASO needs to be present within the nucleus in order to inhibit or modulate the splicing of pre-mRNA into mRNA. Such antisense inhibition of splicing produces an mRNA or mRNAs lacking the exon targeted by the ASO. Such mRNA(s) is called "splice variant(s)", and encodes protein(s) smaller than the protein encoded by the full-length mRNA.

In principle, splicing can be interrupted by inhibiting the formation of "splicesome E complex". If an ASO tightly binds to a junction of (5' → 3') exon-intron, i.e. "5' splice site", the ASO blocks the complex formation between pre-mRNA and factor U1, and therefore the formation of "splicesome E complex". Likewise, "splicesome E complex" cannot be formed if an ASO tightly binds to a junction of (5' → 3') intron-exon, i.e. "3' splice site".

3' splice site and 5' splice site are schematically illustrated in the drawing provided below.

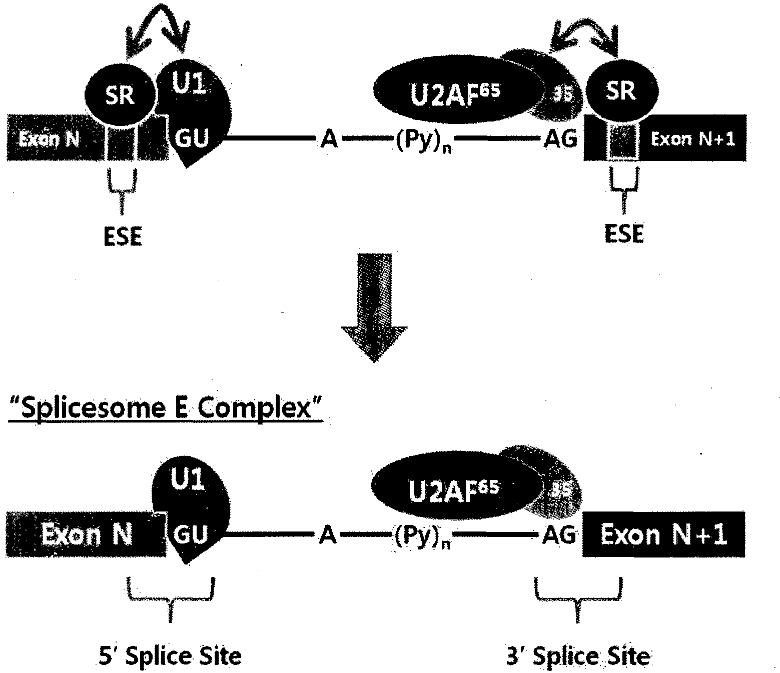

Unnatural Oligonucleotides: DNA or RNA oligonucleotides are susceptible to degradation by endogenous nucleases, limiting their therapeutic utility. To date, many types of unnatural (naturally non-occurring) oligonucleotides have been developed and studied intensively. [Clin. Exp. Pharmacol. Physiol. vol 33, 533-540 (2006)] Some of them show extended metabolic stability compared to DNA and RNA. Provided below are the chemical structures for a few of representative unnatural oligonucleotides. Such oligonucleotides predictably bind to a complementary nucleic acid as DNA or RNA does. 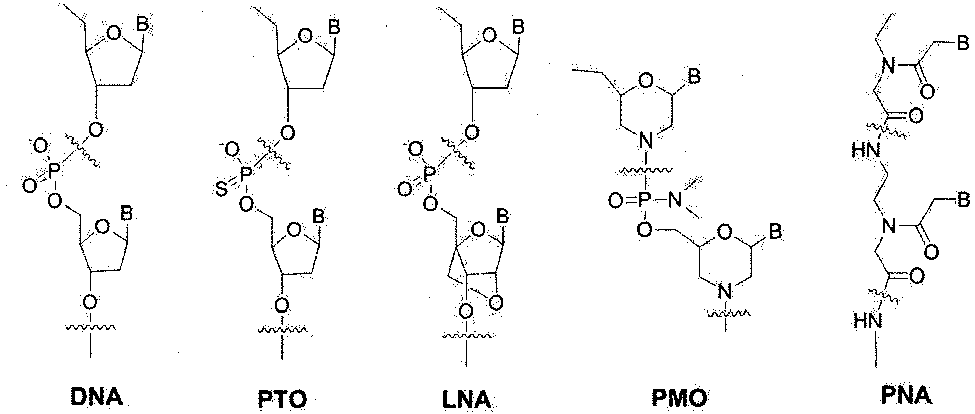 B :Nucleobase

Phosphorothioate Oligonucleotide: Phosphorothioate oligonucleotide (PTO) is a DNA analog with one of the backbone phosphate oxygen atoms replaced with a sulfur atom per monomer. Such a small structural change made PTO comparatively resistant to degradation by nucleases. [Ann. Rev. Biochem. vol 54, 367-402 (1985)]

Reflecting the structural similarity in the backbone of PTO and DNA, they both poorly penetrate the cell membrane in most mammalian cell types. For some types of cells abundantly expressing transporter(s) of DNA, however, DNA and PTO show good cell penetration. Systemically administered PTOs are known to readily distribute to the liver and kidney. [Nucleic Acids Res. vol 25, 3290-3296 (1997)]

In order to facilitate PTO's cell penetration in vitro, lipofection has been popularly practiced. However, lipofection physically alters the cell membrane, causes cytotoxicity, and therefore would not be ideal for long term in vivo therapeutic use.

Over the past 30 years, antisense PTOs and variants of PTOs have been clinically evaluated to treat cancers, immunological disorders, metabolic diseases, and so on. [Biochemistry vol 41, 4503-4510 (2002); Clin. Exp. Pharmacol. Physiol. vol 33, 533-540 (2006)] Many of such antisense drug candidates have not been successfully developed partly due to PTO's poor cell penetration. In order to overcome the poor cell penetration, PTO needs to be administered at high dose for therapeutic activity. However, PTOs are known to be associated with dose-limiting toxicity including increased coagulation time, complement activation, tubular nephropathy, Kupffer cell activation, and immune stimulation including splenomegaly, lymphoid hyperplasia, mononuclear cell infiltration. [Clin. Exp. Pharmacol. Physiol. vol 33, 533-540 (2006)]

Many antisense PTOs have been found to show due clinical activity for diseases with a significant contribution from the liver or kidney. Mipomersen is a PTO analog which inhibits the synthesis of apoB-100, a protein involved in LDL cholesterol transport. Mipomersen manifested due clinical activity in atherosclerosis patients most likely due to its preferential distribution to the liver. [Circulation vol 118(7), 743-753 (2008)] ISIS-113715 is a PTO antisense analog inhibiting the synthesis of protein tyrosine phosphatase 1B (PTP1B), and was found to show therapeutic activity in type II diabetes patients. [Curr. Opin. Mol. Ther. vol 6, 331-336 (2004)]

Locked Nucleic Acid: In locked nucleic acid (LNA), the backbone ribose ring of RNA is structurally constrained to increase the binding affinity for RNA or DNA. Thus, LNA may be regarded as a high affinity DNA or RNA analog. [Biochemistry vol 45, 7347-7355 (2006)]

Phosphorodiamidate Morpholino Oligonucleotide: In phosphorodiamidate morpholino oligonucleotide (PMO), the backbone phosphate and 2-deoxyribose of DNA are replaced with phosphoramidate and morpholine, respectively. [Appl. Microbiol. Biotechnol. vol 71, 575-586 (2006)] Whilst the DNA backbone is negatively charged, the PMO backbone is not charged. Thus the binding between PMO and mRNA is free of electrostatic repulsion between the backbones, and tends to be stronger than that between DNA and mRNA. Since PMO is structurally very different from DNA, PMO wouldn't be recognized by the hepatic transporter(s) recognizing DNA or RNA. Nevertheless, PMO doesn't readily penetrate the cell membrane.

Peptide Nucleic Acid: Peptide nucleic acid (PNA) is a polypeptide with N-(2-aminoethyl)glycine as the unit backbone, and was discovered by Dr. Nielsen and colleagues. [Science vol 254, 1497-1500 (1991)] The chemical structure and abbreviated nomenclature of PNA are illustrated in the drawing provided below. Like DNA and RNA, PNA also selectively binds to complementary nucleic acid. [Nature (London) vol 365, 566-568 (1992)] In binding to complementary nucleic acid, the N-terminus of PNA is regarded as equivalent to the "5'-end" of DNA or RNA, and the C-terminus of PNA as equivalent to the "3'-end" of DNA or RNA.

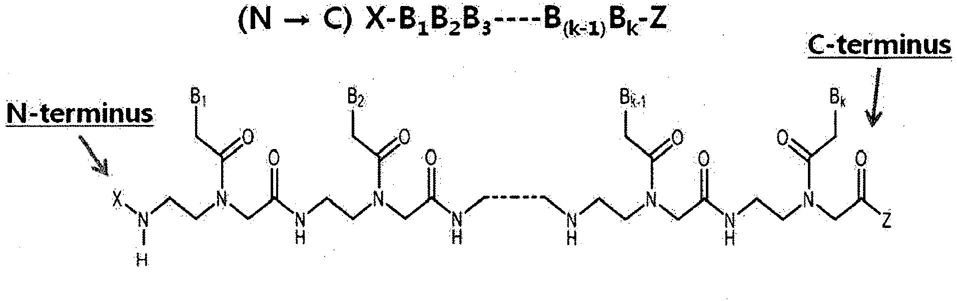

Like PMO, the PNA backbone is not charged. Thus the binding between PNA and RNA tends to be stronger than the binding between DNA and RNA. Since PNA is markedly different from DNA in the chemical structure, PNA wouldn't be recognized by the hepatic transporter(s) recognizing DNA, and would show a tissue distribution profile different from that of DNA or PTO. However, PNA also poorly penetrates the mammalian cell membrane. *(*Adv. Drug Delivery Rev. vol 55, 267-280, 2003)

Modified Nucleobases to Improve Membrane Permeability of PNA: PNA was made highly permeable to mammalian cell membrane by introducing modified nucleobases with a cationic lipid or its equivalent covalently attached thereto. The chemical structures of such modified nucleobases are provided below. Such modified nucleobases of cytosine, adenine, and guanine were found to predictably and complementarily hybridize with guanine, thymine, and cytosine, respectively. [PCT Appl. No. PCT/KR2009/001256; EP2268607; US8680253]

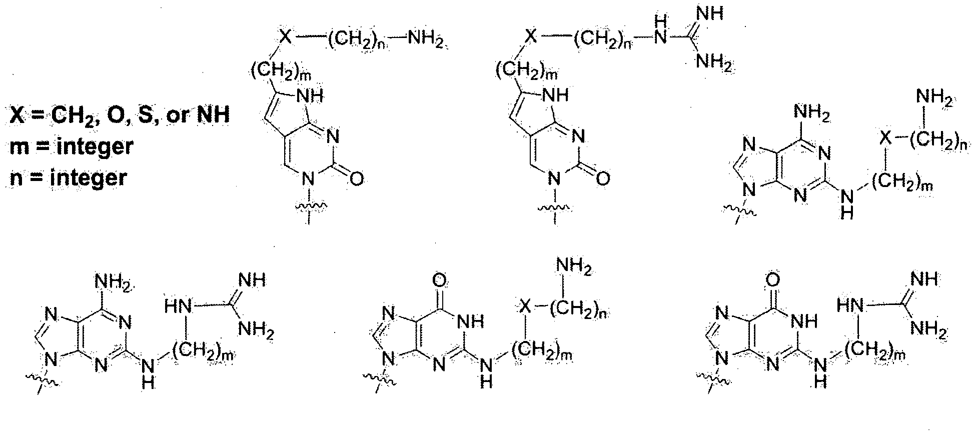

Incorporation of such modified nucleobases onto PNA resembles situations of lipofection. By lipofection, oligonucleotide molecules with phosphate backbone are wrapped with cationic lipid molecules such as lipofectamine, and such lipofectamine/oligonucleotide complexes tend to penetrate membrane rather easily as compared to naked oligonucleotide molecules.

In addition to good membrane permeability, those PNA derivatives were found to possess ultra-strong affinity for complementary nucleic acid. For example, introduction of 4 to 5 modified nucleobases onto 11- to 13-mer PNA derivatives easily yielded a Tₘ gain of 20°C or higher in duplex formation with complementary DNA. Such PNA derivatives are highly sensitive to a single base mismatch. A single base mismatch resulted in a Tₘ loss of 11 to 22°C depending on the type of modified base as well as PNA sequence.

Small Interfering RNA (siRNA): Small interfering RNA (siRNA) refers to a double stranded RNA of 20-25 base pairs. [Microbiol. Mol. Biol. Rev. vol 67(4), 657-685 (2003)] The antisense strand of siRNA somehow interacts with proteins to form an "RNA-induced Silencing Complex" (RISC). Then the RISC binds to a certain portion of mRNA complementary to the antisense strand of siRNA. The mRNA complexed with the RISC undergoes cleavage. Thus siRNA catalytically induces the cleavage of its target mRNA, and consequently inhibits the protein expression by the mRNA. The RISC does not always bind to the full complementary sequence within its target mRNA, which raises concerns relating to off-target effects of an siRNA therapy. Like other classes of oligonucleotide with DNA or RNA backbone, siRNA possesses poor cell permeability and therefore tends to show poor in vitro or in vivo therapeutic activity unless properly formulated or chemically modified to have good membrane permeability.

Tyrosinase siRNAs: A TYR siRNA targeting a 19-mer RNA sequence within the human TYR mRNA was found to inhibit the expression of the TYR mRNA and protein in human epidermal melanocytes (HEMs) following a lipofection at 20 nM. The siRNA down-regulated the TYR mRNA and protein, but didn't affect the expression of tyrosinase related proteins 1 and 2. The siRNA inhibited melanogenesis induced with UV irradiation. [BMB Reports, vol 42(3), 178-183 (2009)]

siRNAs designed to target the mouse TYR mRNA were screened for their ability to inhibit the expression of the TYR mRNA and protein, and melanogenesis in mouse B16 melanoma cells following a lipofection at 40 nM. The most effective siRNA was injected into the eyeball of C57BL/6 mouse and was found to inhibit the TYR mRNA in the retina by ca 60%. [Mol. Biol. International, vol 2010, Article ID 240472 (2010)]

### Disclosure of the Invention

### Problem to be solved

There are more examples for TYR siRNAs in the public domain. For example, a prior art disclosed siRNAs targeting the human TYR mRNA to treat hyper-pigmentation. [US 7504385] However, practicability of such siRNAs is questionable for use against hyper-pigmentation. siRNAs are too expensive to manufacture, and their cosmetic utility tends to be limited. Further they need to be delivered into the skin by trans-dermal administration for good user compliance. It should be addressed that trans-dermal delivery has been a huge technical challenge in the field of oligonucleotide.

### Solution to the Problem

The present invention provides a peptide nucleic acid derivative represented by **Formula I,** or a pharmaceutically acceptable salt thereof: 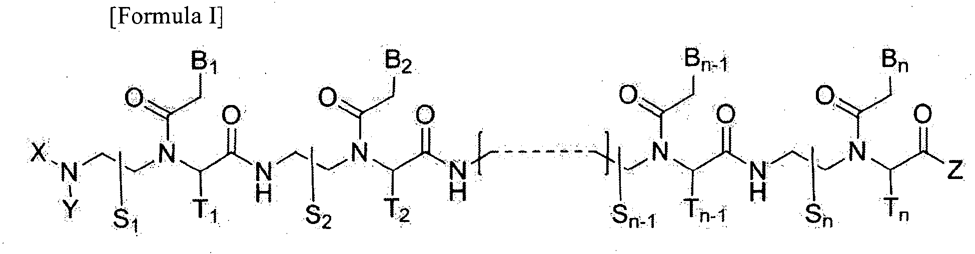 wherein,
n is an integer between 10 and 21;
the compound of **Formula I** possesses at least a 10-mer complementary overlap with the 14-mer pre-mRNA sequence of [(5' → 3') UGUACAGAUUGUCU] in the human TYR pre-mRNA;
the compound of **Formula I** is fully complementary to the human TYR pre-mRNA, or partially complementary to the human TYR pre-mRNA with one or two mismatches from the entire compound of Formula I;
S₁, S₂, ···, Sₙ₋₁, Sₙ, T₁, T₂, ···, Tₙ₋₁, and Tₙ independently represent deuterido, hydrido, substituted or non-substituted alkyl, or substituted or non-substituted aryl radical;
X and Y independently represent deuterido, hydrido [H], formyl [H-C(=O)-], aminocarbonyl [NH₂-C(=O)-], aminothiocarbonyl [NH₂-C(=S)-], substituted or non-substituted alkyl, substituted or non-substituted aryl, substituted or non-substituted alkylacyl, substituted or non-substituted arylacyl, substituted or non-substituted alkyloxycarbonyl, substituted or non-substituted aryloxycarbonyl, substituted or non-substituted alkylaminocarbonyl, substituted or non-substituted arylaminocarbonyl, substituted or non-substituted alkylaminothiocarbonyl, substituted or non-substituted arylaminothiocarbonyl, substituted or non-substituted alkyloxythiocarbonyl, substituted or non-substituted aryloxythiocarbonyl, substituted or non-substituted alkylsulfonyl, substituted or non-substituted arylsulfonyl, substituted or non-substituted alkylphosphonyl radical, or substituted or non-substituted arylphosphonyl radical;
Z represents hydrido, hydroxy, substituted or non-substituted alkyloxy, substituted or non-substituted aryloxy, substituted or non-substituted amino, substituted or non-substituted alkyl, or substituted or non-substituted aryl radical;
B₁, B₂, ···, Bₙ₋₁, and Bₙ are independently selected from natural nucleobases including adenine, thymine, guanine, cytosine and uracil, and unnatural nucleobases; and,
at least four of B₁, B₂, ···, Bₙ₋₁, and Bₙ are independently selected from unnatural nucleobases with a substituted or non-substituted amino radical covalently linked to the nucleobase moiety.

The compound of **Formula I** induces the skipping of "exon 2" in the human TYR pre-mRNA, yields the human TYR mRNA splice variant(s) lacking "exon 2", and therefore is useful to inhibit the functional activity of the gene transcribing the human TYR pre-mRNA.

The condition that "n is an integer between 10 and 21" literally means that n is an integer selectable from a group of integers of 11, 12, 13, 14, 15, 16, 17, 18, 19, and 20.

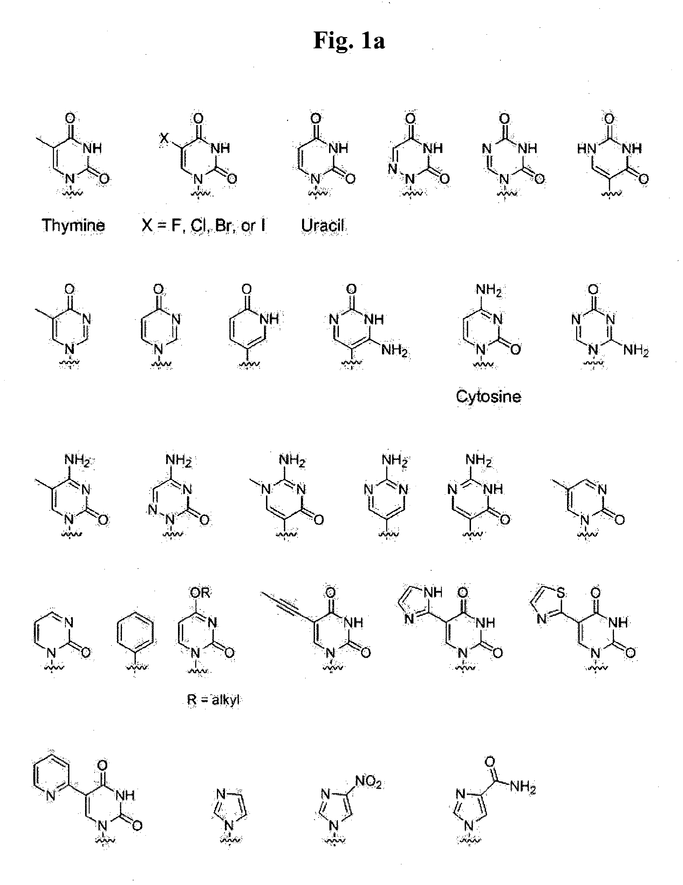

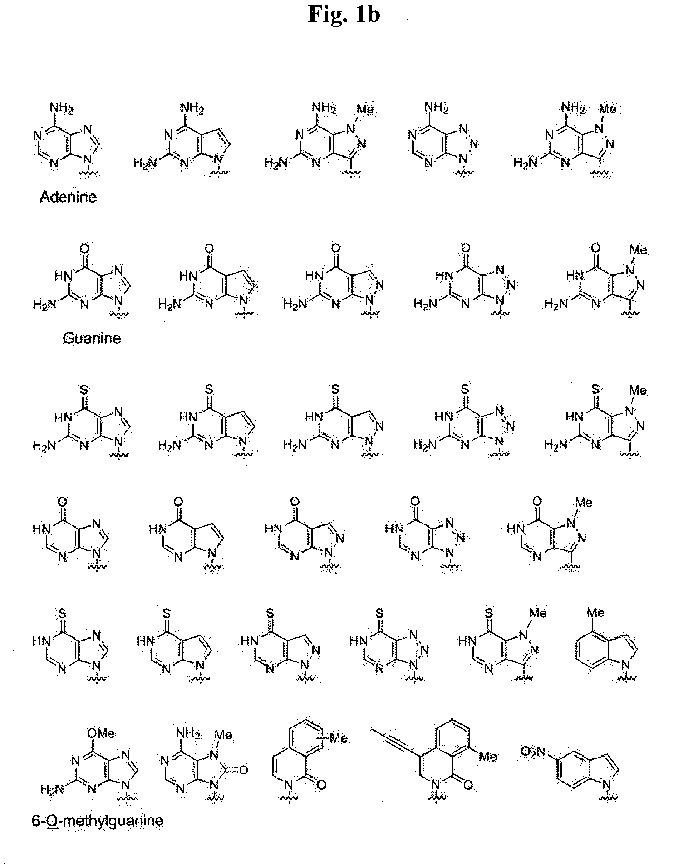

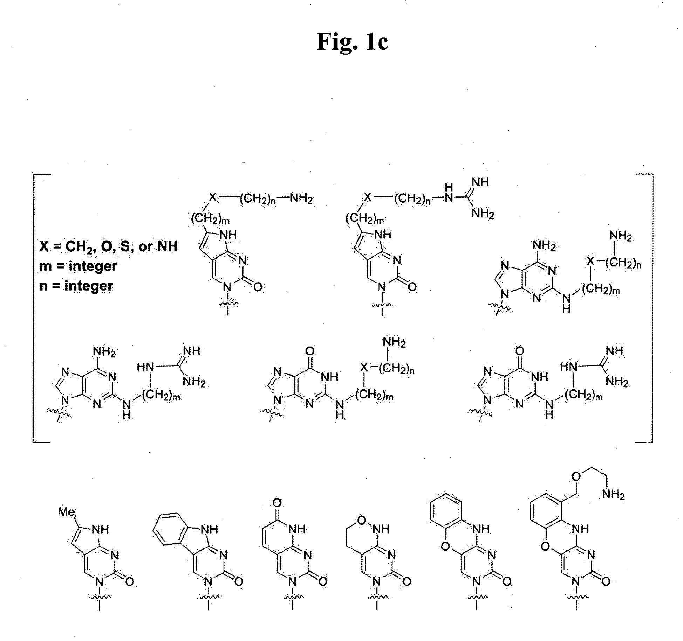

The chemical structures of natural or unnatural nucleobases in the PNA derivative of **Formula I** are exemplified in Figures 1a-1c. Natural (i.e. naturally occurring) or unnatural (naturally non-occurring) nucleobases of the present invention comprise but are not limited to the nucleobases provided in Figures 1a-1c. Provision of such unnatural nucleobases is to illustrate the diversity of allowable nucleobases, and therefore should not be interpreted to limit the scope of the present invention.

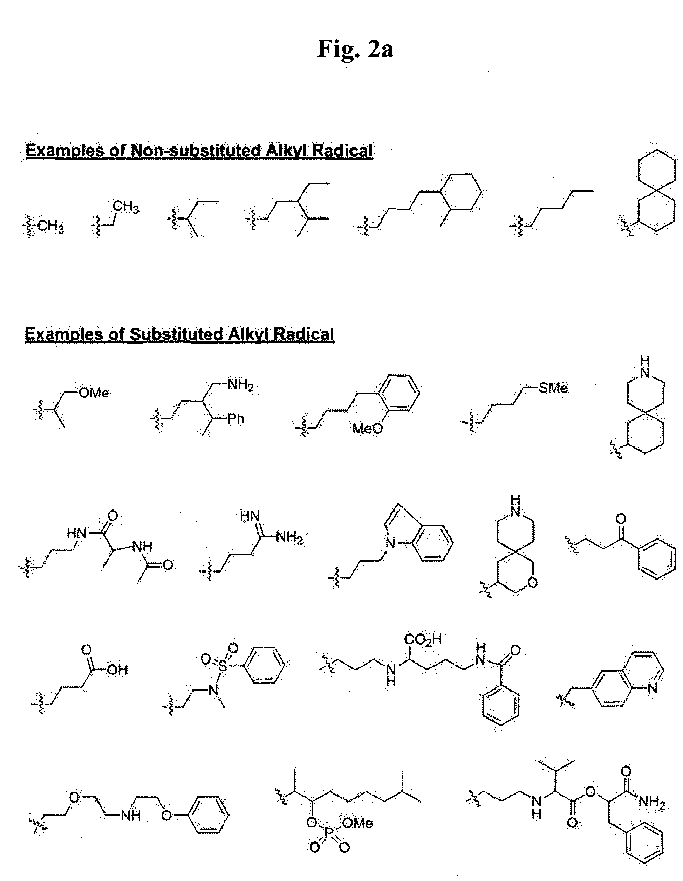

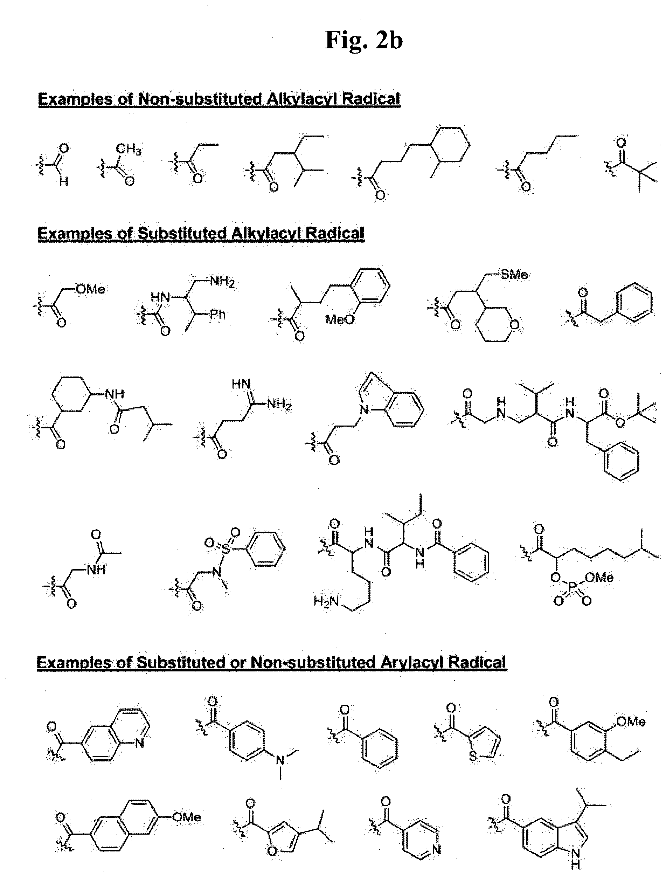

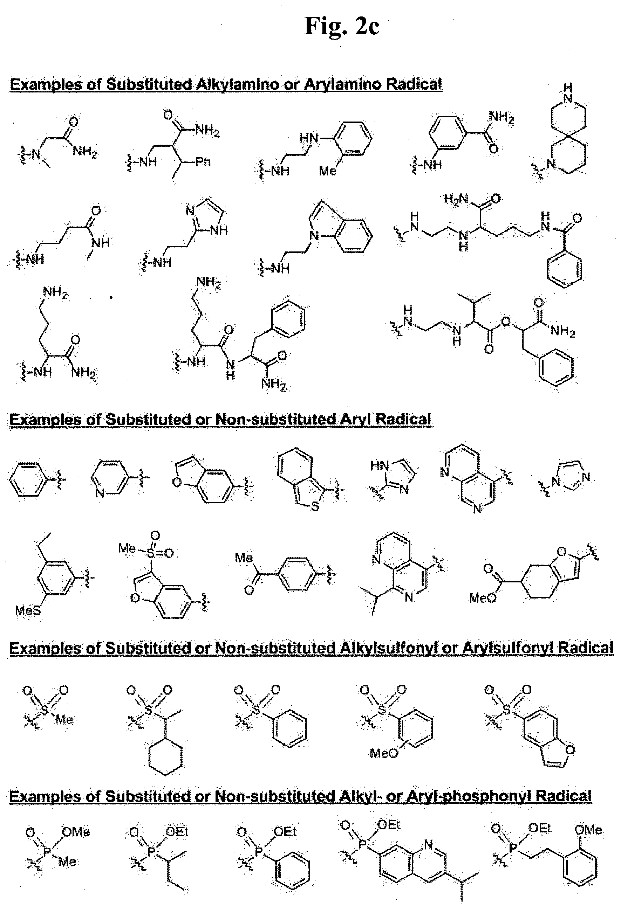

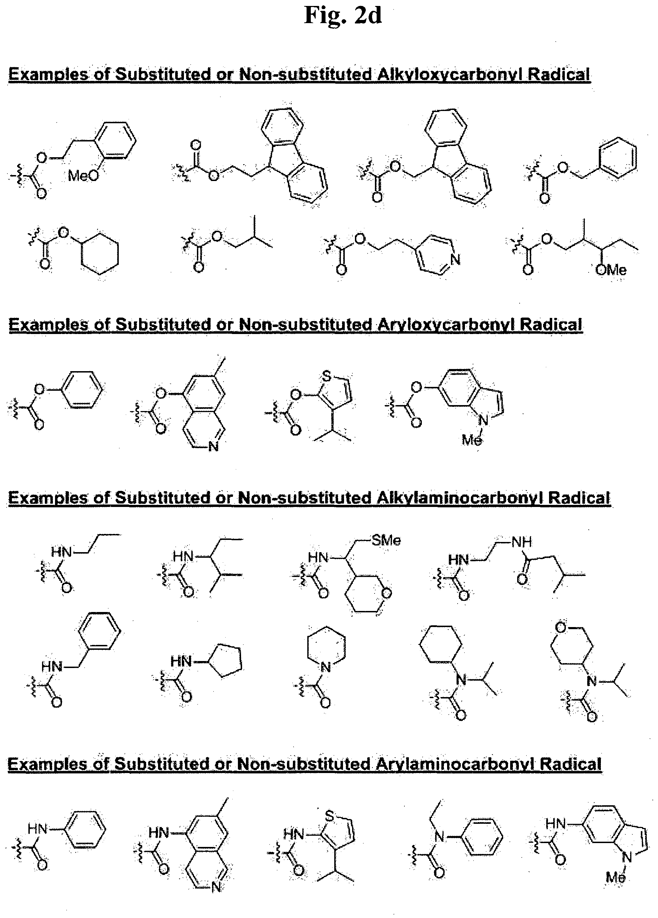

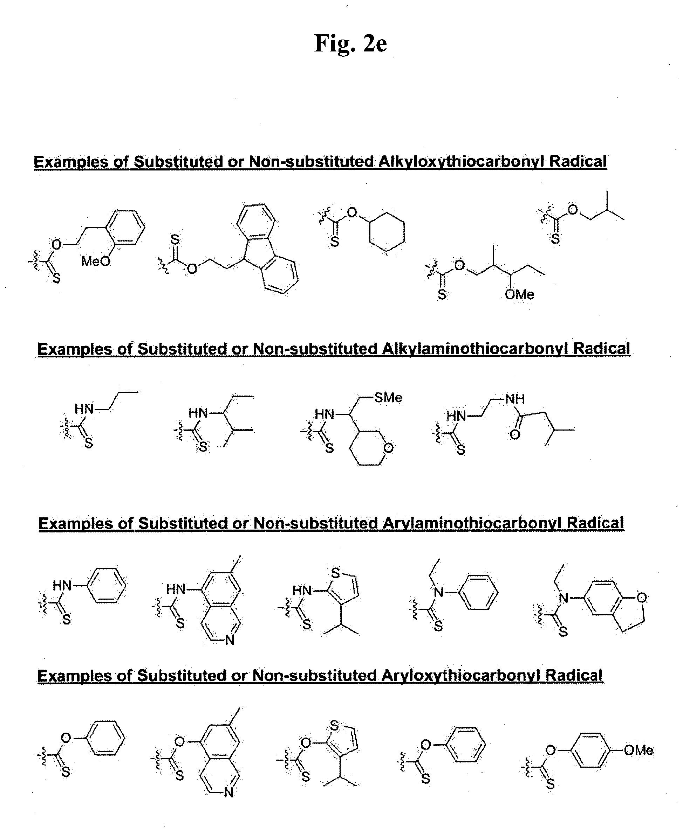

The substituents adopted to describe the PNA derivative of **Formula I** are exemplified in Figures 2a-2e. Figure 2a provides examples for substituted or non-substituted alkyl radicals. Substituted or non-substituted alkylacyl and substituted or non-substituted alkylacyl arylacyl radicals are exemplified in Figure 2b. Figure 2c illustrates examples for substituted or non-substituted alkylamino, substituted or non-substituted arylamino, substituted or non-substituted aryl, substituted or non-substituted alkylsulfonyl or arylsulfonyl, and substituted or non-substituted alkylphosphonyl or arylphosphonyl radicals. Figure 2d provides examples for substituted or non-substituted alkyloxycarbonyl or aryloxycarbonyl, substituted or non-substituted alkyl aminocarbonyl or arylaminocarbonyl radicals. In Figure 2e are provided examples for substituted or non-substituted alkylaminothiocarbonyl, substituted or non-substituted arylaminothiocarbonyl, substituted or non-substituted alkyloxythiocarbonyl, and substituted or non-substituted aryloxythiocarbonyl radicals. Provision of such exemplary substituents is to illustrate the diversity of allowable substituents, and therefore should not be interpreted to limit the scope of the present invention. A skilled person in the field may easily figure out that oligonucleotide sequence is the overriding factor for sequence specific binding of oligonucleotide to the target pre-mRNA sequence over substituents in the N-terminus or C-terminus.

The compound of **Formula I** tightly binds to the complementary DNA as exemplified in the prior art [PCT/KR2009/001256]. The duplex between the PNA derivative of **Formula I** and its full-length complementary DNA or RNA possesses a Tₘ value too high to be reliably determined in aqueous buffer. The PNA compound of **Formula I** yields high Tₘ values with complementary DNAs of shorter length.

The compound of **Formula I** complementarily binds to the 3' splice site of "exon 2" of the human TYR pre-mRNA. [NCBI Reference Sequence: NG_0008748] The 14-mer sequence of [(5' → 3') UGUACAGAUUGUCU] spans the junction of "intron 1" and "exon 2" in the human TYR pre-mRNA, and consists of 7-mer from "intron 1" and 7-mer from "exon 2". Thus the 14-mer pre-mRNA sequence may be conventionally denoted as [(5' → 3') uguacag | AUUGUCU], wherein the intron and exon sequence are provided as "small" and "capital" letters, respectively, and the intron-exon junction is expressed with " | ". The conventional denotation for pre-mRNA is further illustrated by a 30-mer sequence of [(5' → 3') ggguguuuuguacag | AUUGUCUGUAGCCGA] spanning the junction of "intron 1" and "exon 2" in the human TYR pre-mRNA. In addition, the 30-mer pre-mRNA sequence unequivocally defines the 3' splice site targeted by the compound of **Formula I** within the human TYR pre-mRNA, although the numbering of TYR exons may be reported otherwise.

The compound of **Formula I** tightly binds to the target 3' splice site of the human TYR pre-mRNA transcribed from the human TYR gene, and interferes with the formation of "splicesome early complex" involving the compound's target exon. Since the compound of the present invention sterically inhibits the formation of "splicesome early complex", TYR "exon 2" is spliced out to yield TYR mRNA splice variant(s) lacking "exon 2". Consequently the compound of the present invention induces the skipping of TYR "exon 2".

The strong RNA affinity allows the compound of **Formula I** to induce the skipping of TYR "exon 2", even when the PNA derivative possesses one or two mismatches with the target 3' splice site in the TYR pre-mRNA. Similary the PNA derivative of **Formula I** may still induce the skipping of TYR "exon 2" in a TYR mutant pre-mRNA possessing one or two SNPs (single nucleotide polymorphism) in the target splice site.

The compound of **Formula I** possesses good cell permeability and can be readily delivered into cell as "naked" oligonucleotide as exemplified in the prior art [PCT/KR2009/001256]. Thus the compound of the present invention induces the skipping of "exon 2" in the TYR pre-mRNA, and yields TYR mRNA splice variant(s) lacking TYR "exon 2" in cells treated with the compound of **Formula I** as "naked" oligonucleotide. The compound of **Formula I** does not require any means or formulations for delivery into cell to potently induce the skipping of the target exon in cells. The compound of **Formula I** readily induces the skipping of TYR "exon 2" in cells treated with the compound of the present invention as "naked" oligonucleotide at sub-femtomolar concentration.

Owing to the good cell or membrane permeability, the PNA derivative of **Formula I** can be topically administered as "naked" oligonucleotide to induce the skipping of TYR "exon 2" in the skin. The compound of **Formula I** does not require a formulation to increase trans-dermal delivery into target tissue for the intended therapeutic or biological activity. Usually the compound of **Formula I** is dissolved in water and co-solvent, and topically or trans-dermally administered at subpicomolar concentration to elicit the desired therapeutic or biological activity in target skin. The compound of the present invention does not need to be heavily or invasively formulated to elicit the topical therapeutic activity. Nevertheless, the PNA derivative of **Formula I** can be formulated with cosmetic ingredients or adjuvants as topical cream or lotion. Such topical cosmetic cream or lotion may be useful to treat facial hyper-pigmentation.

The compound of **Formula I** may be used as combined with a pharmaceutically acceptable acid or base including but not limited to sodium hydroxide, potassium hydroxide, hydrochloric acid, methanesulfonic acid, citric acid, trifluoroacetic acid, and so on.

The PNA derivative of **Formula I** or a pharmaceutically acceptable salt thereof can be administered to a subject in combination with a pharmaceutically acceptable adjuvant including but not limited to citric acid, hydrochloric acid, tartaric acid, stearic acid, polyethyleneglycol, polypropyleneglycol, ethanol, isopropanol, sodium bicarbonate, distilled water, preservative(s), and so on.

The compound of the present invention can be topically administered to a subject at a therapeutically or biologically effective concentration ranging from 1 aM to higher than 1 nM, which would vary depending on the dosing schedule, conditions or situations of the subject, and so on.

Preferred is a PNA derivative of **Formula I,** or a pharmaceutically acceptable salt thereof:
wherein,
n is an integer between 10 and 21;
the compound of **Formula I** possesses at least a 10-mer complementary overlap with the 14-mer pre-mRNA sequence of [(5' → 3') UGUACAGAUUGUCU] in the human TYR pre-mRNA;
the compound of **Formula I** is fully complementary to the human TYR pre-mRNA, or partially complementary to the human TYR pre-mRNA with one or two mismatches from the entire compound of Formula I;
S₁, S₂, ···, Sₙ₋₁, Sₙ, T₁, T₂, ···, Tₙ₋₁, and Tₙ independently represent deuterido or hydrido radical;
X and Y independently represent deuterido, hydrido, formyl, aminocarbonyl, aminothiocarbonyl, substituted or non-substituted alkyl, substituted or non-substituted aryl, substituted or non-substituted alkylacyl, substituted or non-substituted arylacyl, substituted or non-substituted alkyloxycarbonyl, substituted or non-substituted aryloxycarbonyl, substituted or non-substituted alkylaminocarbonyl, substituted or non-substituted arylaminocarbonyl, substituted or non-substituted alkylaminothiocarbonyl, substituted or non-substituted arylaminothiocarbonyl, substituted or non-substituted alkyloxythiocarbonyl, substituted or non-substituted aryloxythiocarbonyl, substituted or non-substituted alkylsulfonyl, substituted or non-substituted arylsulfonyl, substituted or non-substituted alkylphosphonyl radical, or substituted or non-substituted arylphosphonyl radical;
Z represents hydrido, hydroxy, substituted or non-substituted alkyloxy, substituted or non-substituted aryloxy, or substituted or non-substituted amino radical;
B₁, B₂, ···, Bₙ₋₁, and Bₙ are independently selected from natural nucleobases including adenine, thymine, guanine, cytosine and uracil, and unnatural nucleobases;
at least four of B₁, B₂, ···, Bₙ₋₁, and Bₙ are independently selected from unnatural nucleobases represented by **Formula II, Formula III,** or **Formula IV:** 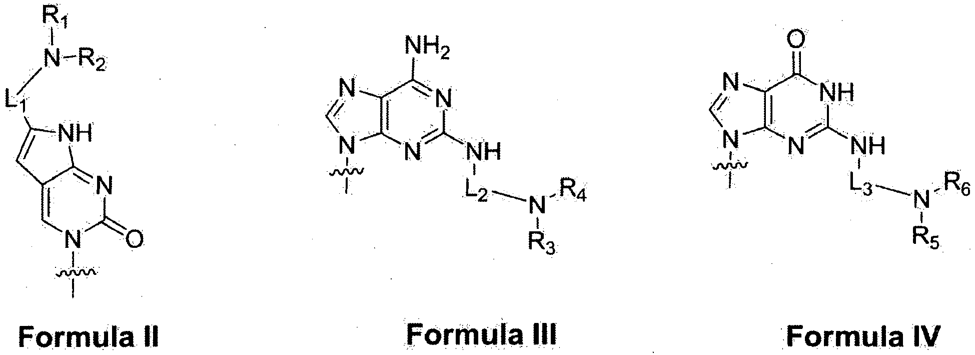
wherein,
R₁, R₂, R₃, R₄, R₅ and R₆ are independently selected from hydrido, and substituted or non-substituted alkyl radical;
- L₁, L₂ and L₃ are a covalent linker represented by **Formula V** covalently linking the basic amino group to the nucleobase moiety: 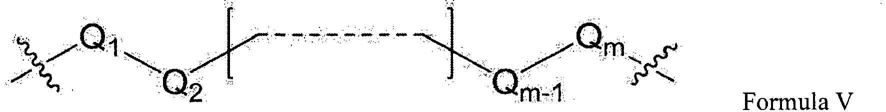
wherein,
Qₗ and Qₘ are substituted or non-substituted methylene (-CH₂-) radical, and Qₘ is directly linked to the basic amino group;
Q₂, Q₃, ···, and Qₘ₋₁ are independently selected from substituted or non-substituted methylene, oxygen (-O-), sulfur (-S-), and substituted or non-substituted amino radical [-N(H)-, or -N(substituent)-]; and,
m is an integer between 1 and 15.

Of interest is a PNA oligomer of **Formula I,** or a pharmaceutically acceptable salt thereof:
wherein,
n is an integer between 11 and 18;
the compound of **Formula I** possesses at least a 10-mer complementary overlap with the 14-mer pre-mRNA sequence of [(5' → 3') UGUACAGAUUGUCU] in the human TYR pre-mRNA;
the compound of **Formula I** is fully complementary to the human TYR pre-mRNA;
S₁, S₂, ···, Sₙ₋₁, Sₙ, T₁, T₂, ···, Tₙ₋₁, and Tₙ are hydrido radical;
X and Y independently represent hydrido, substituted or non-substituted alkyl, substituted or non-substituted aryl, substituted or non-substituted alkylacyl, substituted or non-substituted arylacyl, substituted or non-substituted alkyloxycarbonyl, or substituted or non-substituted aryloxycarbonyl radical;
Z represents substituted or non-substituted amino radical;
B₁, B₂, ···, Bₙ₋₁, and Bₙ are independently selected from natural nucleobases including adenine, thymine, guanine, cytosine and uracil, and unnatural nucleobases;
at least four of B₁, B₂, ···, Bₙ₋₁, and Bₙ are independently selected from unnatural nucleobases represented by **Formula II, Formula III,** or **Formula IV;**
R₁, R₂, R₃, R₄, R₅ and R₆ are independently selected from hydrido, and substituted or non-substituted alkyl radical;
Q₁ and Qₘ are substituted or non-substituted methylene radical, and Qₘ is directly linked to the basic amino group;
Q₂, Q₃, ···, and Qₘ₋₁ are independently selected from substituted or non-substituted methylene, oxygen, and amino radical; and,
m is an integer between 1 and 11.

Of particular interest is a PNA derivative of **Formula I,** or a pharmaceutically acceptable salt thereof:
wherein,
n is an integer between 11 and 16;
the compound of **Formula I** possesses at least a 10-mer complementary overlap with the 14-mer pre-mRNA sequence of [(5' → 3') UGUACAGAUUGUCU] in the human TYR pre-mRNA;
the compound of **Formula I** is fully complementary to the human TYR pre-mRNA;
S₁, S₂, ···, Sₙ₋₁, Sₙ, T₁, T₂, ···, Tₙ₋₁, and Tₙ are hydrido radical;
X and Y independently selected from hydrido, substituted or non-substituted alkylacyl, or substituted or non-substituted alkyloxycarbonyl radical;
Z represents substituted or non-substituted amino radical;
B₁, B₂, ···, Bₙ₋₁, and Bₙ are independently selected from natural nucleobases including adenine, thymine, guanine, cytosine and uracil, and unnatural nucleobases;
at least four of B₁, B₂, ···, Bₙ₋₁, and Bₙ are independently selected from unnatural nucleobases represented by **Formula II, Formula III,** or **Formula IV;**
R₁, R₂, R₃, R₄, R₅ and R₆ are independently selected from hydrido, and substituted or non-substituted alkyl radical;
Q₁ and Qₘ are methylene radical, and Qₘ is directly linked to the basic amino group;
Q₂, Q₃, ···, and Qₘ₋₁ are independently selected from methylene, oxygen, and amino radical; and,
m is an integer between 1 and 9.

Of high interest is a PNA oligomer of **Formula I,** or a pharmaceutically acceptable salt thereof:
wherein,
n is an integer between 11 and 16;
the compound of **Formula I** possesses at least a 10-mer complementary overlap with the 14-mer pre-mRNA sequence of [(5' → 3') UGUACAGAUUGUCU] in the human TYR pre-mRNA;
the compound of **Formula I** is fully complementary to the human TYR pre-mRNA;
S₁, S₂, ···, Sₙ₋₁, Sₙ, T₁, T₂, ···, Tₙ₋₁, and Tₙ are hydrido radical;
X and Y independently selected from hydrido, substituted or non-substituted alkylacyl, or substituted or non-substituted alkyloxycarbonyl radical;
Z represents substituted or non-substituted amino radical;
B₁, B₂, ···, Bₙ₋₁, and Bₙ are independently selected from natural nucleobases including adenine, thymine, guanine, cytosine and uracil, and unnatural nucleobases;
at least four of B₁, B₂, ···, Bₙ₋₁, and Bₙ are independently selected from unnatural nucleobases represented by **Formula II, Formula III,** or **Formula IV;**
R₁, R₃, and R₅ are hydrido radical, and R₂, R₄, and R₆ independently represent hydrido, or substituted or non-substituted alkyl radical;
Qₗ and Qₘ are methylene radical, and Qₘ is directly linked to the basic amino group;
Q₂, Q₃, ···, and Qₘ₋₁ are independently selected from methylene, oxygen radical; and, m is an integer between 1 and 9.

Of higher interest is a PNA derivative of **Formula I,** or a pharmaceutically acceptable salt thereof:
wherein,
n is an integer between 11 and 16;
the compound of **Formula I** possesses at least a 10-mer complementary overlap with the 14-mer pre-mRNA sequence of [(5' → 3') UGUACAGAUUGUCU] in the human TYR pre-mRNA;
the compound of **Formula I** is fully complementary to the human TYR pre-mRNA;
S₁, S₂, ···, Sₙ₋₁, Sₙ, T₁, T₂, ···, Tₙ₋₁, and Tₙ are hydrido radical;
X and Y independently selected from hydrido, substituted or non-substituted alkylacyl, or substituted or non-substituted alkyloxycarbonyl radical;
Z represents substituted or non-substituted amino radical;
B₁, B₂, ···, Bₙ₋₁, and Bₙ are independently selected from adenine, thymine, guanine, cytosine, and unnatural nucleobases;
at least five of B₁, B₂, ···, Bₙ₋₁, and Bₙ are independently selected from unnatural nucleobases represented by **Formula II, Formula III,** or **Formula IV;**
R₁, R₂ , R₃, R₄, R₅, and R₆ are hydrido radical;
Qₗ and Qₘ are methylene radical, and Qₘ is directly linked to the basic amino group;
Q₂, Q₃, ···, and Qₘ₋₁ are independently selected from methylene, and oxygen radical; and,
m is an integer between 1 and 9.

Of highest interest is a PNA derivative of **Formula I,** or a pharmaceutically acceptable salt thereof:
wherein,
n is an integer between 11 and 16;
the compound of **Formula I** possesses at least a 10-mer complementary overlap with the 14-mer pre-mRNA sequence of [(5' → 3') UGUACAGAUUGUCU] in the human TYR pre-mRNA;
the compound of **Formula I** is fully complementary to the human TYR pre-mRNA;
S₁, S₂, ···, Sₙ₋₁, Sₙ, T₁, T₂, ···, Tₙ₋₁, and Tₙ are hydrido radical;
X is hydrido radical;
Y represents substituted or non-substituted alkylacyl, or substituted or non-substituted alkyloxycarbonyl radical;
Z represents substituted or non-substituted amino radical;
B₁, B₂, ···, Bₙ₋₁, and Bₙ are independently selected from adenine, thymine, guanine, cytosine, and unnatural nucleobases;
at least five of B₁, B₂, ···, Bₙ₋₁, and Bₙ are independently selected from unnatural nucleobases represented by **Formula II, Formula III,** or **Formula IV;**
R₁, R₂, R₃, R₄, R₅, and R₆ are hydrido radical;
L₁ represents -(CH₂)₂-O-(CH₂)₂-, -CH₂-O-(CH₂)₂-, -CH₂-O-(CH₂)₃-, -CH₂-O-(CH₂)₄-, or -CH₂-O-(CH₂)₅- with the right end is directly linked to the basic amino group; and,
L₂ and L₃ are independently selected from -(CH₂)₂-O-(CH₂)₂-, -(CH₂)₃-O-(CH₂)₂-, -(CH₂)₂-O-(CH₂)₃-, -(CH₂)₂-, -(CH₂)₃-, -(CH₂)₄-, -(CH₂)₅-, -(CH₂)₆-, -(CH₂)₇-, and -(CH₂)₈- with the right end is directly linked to the basic amino group.

Of specific interest is a PNA derivative of **Formula I** which is selected from the group of compounds provided below, or a pharmaceutically acceptable salt thereof:
(N→C) Fethoc-CA(5)G-ACA(5)-ATC(1O2)-TG(6)T-A(5)-NH₂;
(N→C) Fethoc-AC(1/2)A(5)-GA(5)C-AA(5)T-CTG(6)-C(1/2)C-NH₂;
(N→C) Ac-CA(5)G-ACA(5)-ATC(1O2)-TG(6)T-A(5)-NH₂;
(N→C) Benzoyl-CA(5)G-ACA(5)-ATC(1O2)-TG(6)T-A(5)-NH₂;
(N→C) Piv-CA(5)G-ACA(5)-ATC(1O2)-TG(6)T-A(5)-NH₂;
(N→C) Methyl-CA(5)G-ACA(5)-ATC(1O2)-TG(6)T-A(5)-NH₂;
(N→C) n-Propyl-CA(5)G-ACA(5)-ATC(1O2)-TG(6)T-A(5)-NH₂;
(N→C) Fmoc-CA(5)G-ACA(5)-ATC(1O2)-TG(6)T-A(5)-NH₂;
(N→C) p-Toluenesulfonyl-CA(5)G-ACA(5)-ATC(1O2)-TG(6)T-A(5)-NH₂;
(N→C) H-Lys-CA(5)G-ACA(5)-ATC(1O2)-TG(6)T-A(5)-NH₂;
(N→C) Fethoc-Lys-Gly-CA(5)G-ACA(5)-ATC(1O2)-TG(6)T-A(5)-NH₂;
(N→C) Fethoc-CA(5)G-ACA(5)-ATC(1O2)-TG(6)T-A(5)-Lys-NH₂;
(N→C) Fethoc-Val-Gly-CA(5)G-ACA(5)-ATC(1O2)-TG(6)T-A(5)-NH₂;
(N→C) Ac-Arg-CA(5)G-ACA(5)-ATC(1O2)-TG(6)T-A(5)-NH₂;
(N→C) Benzyl-CA(5)G-ACA(5)-ATC(1O2)-TG(6)T-A(5)-NH₂;
(N→C) Phenyl-CA(5)G-ACA(5)-ATC(1O2)-TG(6)T-A(5)-NH₂;
(N→C) [N-(2-Phenylethyl)amino]carbonyl-CA(5)G-ACA(5)-ATC(1O2)-TG(6)T-A(5)-NH₂;
(N→C) Benzoyl-Leu-CA(5)G-ACA(5)-ATC(1O2)-TG(6)T-A(5)-NH₂;
(N→C) Piv-Lys-CA(5)G-ACA(5)-ATC(1O2)-TG(6)T-A(5)-Val-NH₂;
(N→C) Fethoc-CA(7)G-AC(2O2)A-A(4)TC(1O2)-TG(6)T-A-NH₂;
(N→C) Fethoc-CTG(6)-ACA(5)-ATC(1O2)-TG(6)T-A(5)-NH₂;
(N→C) Fethoc-CA(5)G-ATA(5)-ATC(1O2)-TG(5)T-A(5)-NH₂;
(N→C) Fethoc-TA(5)G-ACA(5)-ATC(1O2)-TG(6)T-A(5)-NH₂;
(N→C) Fethoc-TCA(3)-GAC(1O5)-A(5)AT-C(102)TG(5)-TA(5)-NH₂;
(N→C) Fethoc-CA(5)G-ACA(5)-ATC(1O2)-TG(6)-T-A(5)C-NH₂;
(N→C) Fethoc-AG(6)A-CA(5)A-TC(1O2)T-G(6)TA(5)-C-NH₂;
(N→C) Fethoc-AG(6)A-CA(5)A-TC(1O2)T-G(6)TA(5)-CA(2O2)A-NH₂;
(N→C) Fethoc-AC(1O2)A-GA(6)C-AA(6)T-CTG(6)-TA(6)C(1O2)-AA(6)-NH₂;
(N→C) Fethoc-AC(1O3)T-GA(6)C-TA(6)T-CTG(6)-TAC(1O5)-A(4)A-NH₂;
(N→C) Fethoc-GA(6)C-AA(6)T-CTG(6)-TA(6)C(1O2)-AA(6)-NH₂;
(N→C) Fethoc-AC(1O2)A-GA(6)C-AA(6)T-CTG(6)-TA(6)C-A(5)AA(2O2)-A-NH₂;
(N→C) Fethoc-AA(6)T-CTG(6)-TA(6)C-A(5)AA(2O2)-A-NH₂;
(N→C) Fethoe-AC(1O2)A-GA(6)C-AA(6)T-CTG(6)-TA(6)C-A(5)AA(2O3)-A-NH₂;
(N→C) Fethoc-GC(104)T-AC(1O2)A-GA(4)C-AAT-CTG(6)-TA(6)C-NH₂;
(N→C) Fethoc-GC(1O2)T-A(3)CA-GA(4)C-AAT-C(1O2)TG-NH₂;
(N→C) Fethoc-GC(1O2)T-A(3)CA-G(2O2)AC-A(5)AT-C(1O2)TG-NH₂; and
(N→C) Fethoc-GC(1O2)T-A(3)CA-GA(4)C-A(2O2)AT-C(1O2)TG-NH₂:
   wherein,
   A, G, T, and C are PNA monomers with a natural nucleobase of adenine, guanine, thymine, and cytosine, respectively;
   C(pOq), A(p), A(pOq), G(p), and G(pOq) are PNA monomers with an unnatural nucleobase represented by **Formula VI, Formula VII, Formula VIII, Formula IX,** and **Formula X,** respectively; 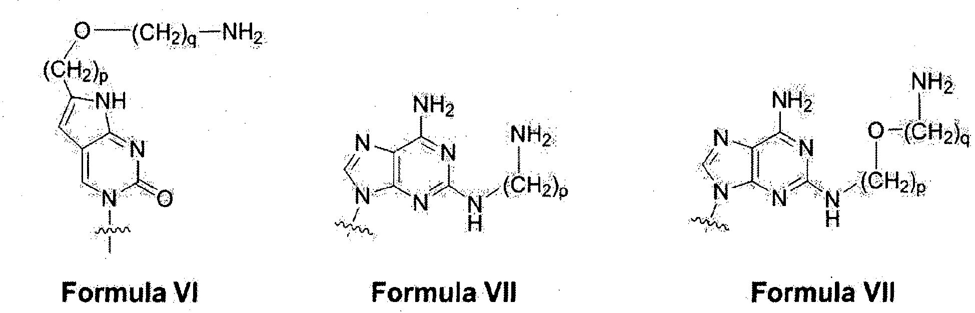 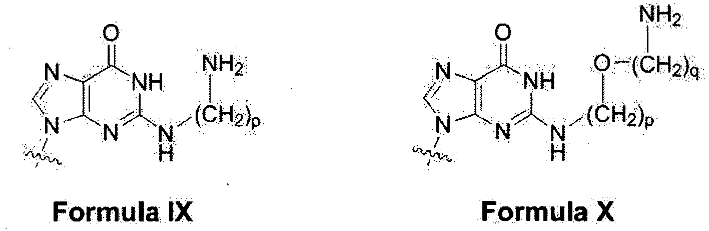 wherein,
      p and q are integers; and,
      the abbreviations for the N- and C-terminus substituents are as specifically described as follows: "Fmoc-" is the abbreviation for "[(9-fluorenyl)methyloxy]carbonyl-"; "Fethoc-" for "[2-(9-fluorenyl)ethyl-1-oxy]carbonyl"; "Ac-" for "acetyl-"; "Benzoyl-" for "benzenecabonyl-"; "Piv-" for "pivalyl-"; "Methyl-" for "methyl-"; "n-Propyl-" for "1-(n-propyl)-"; "H-" for "hydrido-" group; "p-Toluenesulfonyl" for "(4-methylbenzene)-1-sulfonyl-"; "-Lys-" for amino acid residue "lysine"; "-Val-" for amino acid residue "valine"; "-Leu-" for amino acid residue "leucine"; "-Arg-" for amino acid residue "arginine"; "-Gly-" for amino acid residue "glycine"; "[N-(2-Phenylethyl)amino]carbonyl-" for "[N-1-(2-phenylethyl)amino]carbonyl-"; "Benzyl-" for "1-(phenyl)methyl-"; "Phenyl-" for "phenyl-"; "Me-" for "methyl-"; and "-NH₂" for non-subsituted "-amino" group.

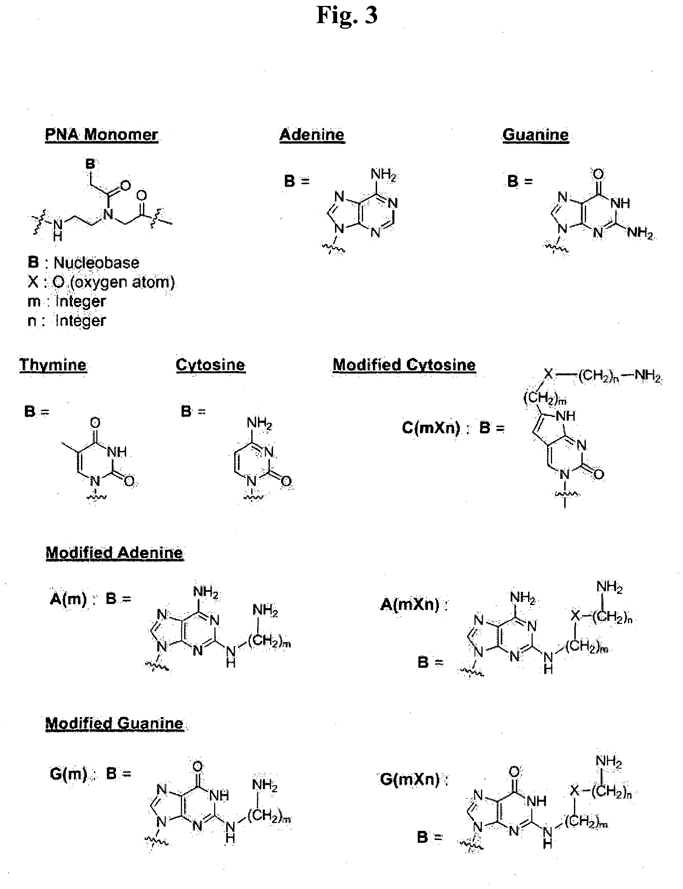

Figure 3 collectively and unambiguously provides the chemical structures for the PNA monomers abbreviated as A, G, T, C, C(pOq), A(p), A(pOq), G(p), and G(pOq). As discussed in the prior art [PCT/KR2009/001256], C(pOq) is regarded as a "modified cytosine" PNA monomer due to its hybridization for "guanine". A(p) and A(pOq) are taken as "modified adenine" PNA monomers for their hybridization for "thymine". Likewise G(p) and G(pOq) are considered to be "modified guanine" PNA monomers for their base pairing with "cytosine".

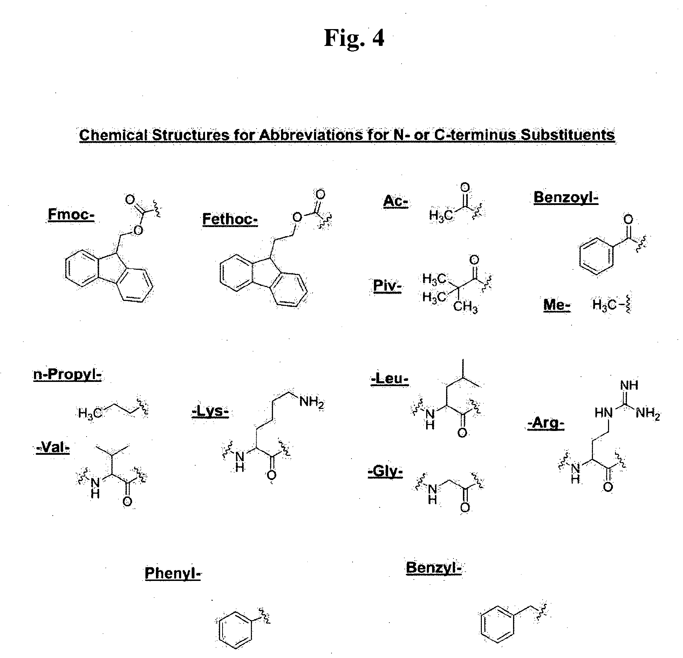

Figure 4 unequivocally illustrates the chemical structures for a variety of abbreviations for substituents used for diversifying the N-terminus or C-terminus of the PNA derivative of **Formula I** in the present invention.

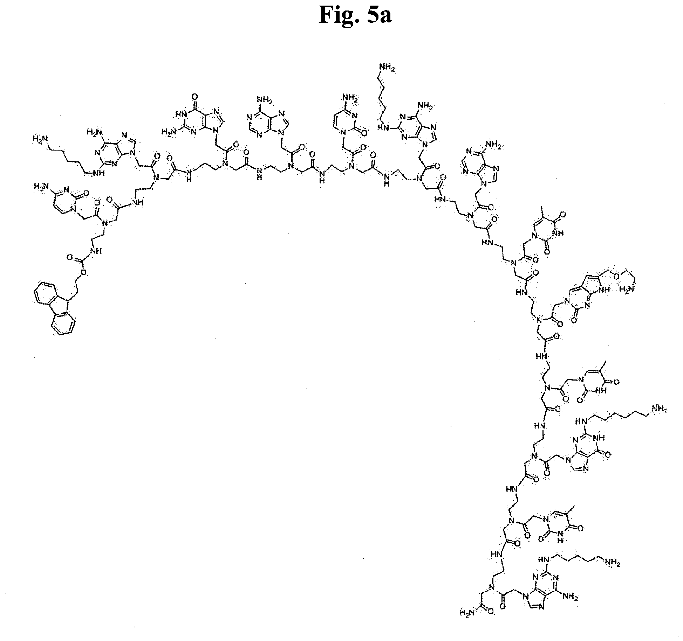

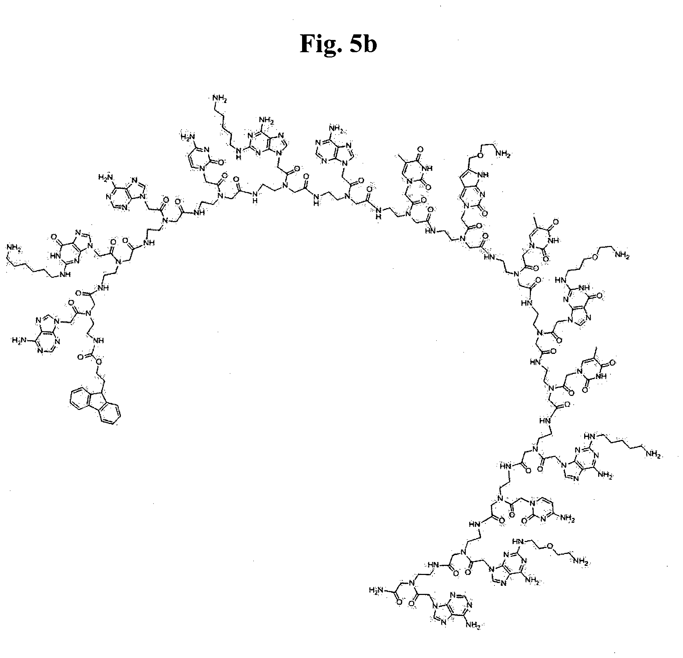

In order to illustrate the abbreviations employed for such PNA derivatives, the chemical structure for a 13-mer PNA derivative abbreviated as "(N→C) Fethoc-CA(5)G-ACA(5)-ATC(1O2)-TG(6)T-A(5)-NH₂" is provided in Figures 5a. As another illustration, the chemical structure for a 15-mer PNA derivative abbreviated as "(N→C) Fethoc-AG(6)A-CA(5)A-TC(1O2) T-G(3O2) TA(5)-CA(2O2)A-NH₂" is provided in Figures 5b.

The 13-mer PNA sequence of "(N→C) Fethoc-CA(5)G-ACA(5)-ATC(1O2)-TG(6)T-A(5)-NH₂" specified in Figures 5a is equivalent to the DNA sequence of "(5' → 3') CAG-ACA-ATC-TGT-A" for complementary binding to pre-mRNA. The 13-mer PNA has a 13-mer complementary overlap with the 13-mer sequence marked "bold" and "underlined" within the 30-mer RNA sequence of [(5' → 3') ggguguuuug**uacag** | **AUUGUCUG**UAGCCGA] spanning the junction of "intron 1" and "exon 2" in the human TYR pre-mRNA.

The 15-mer PNA sequence of "(N→C) Fethoc-AG(6)A-CA(5)A-TC(1O2)T-G(3O2)TA(5)-CA(2O2)A-NH₂" specified in Figures 5b is equivalent to the DNA sequence of "(5' → 3') AGA-CAA-TCT-GTA-CAA" for complementary binding to pre-mRNA. The 15-mer PNA has a 15-mer complementary overlap with the 15-mer sequence marked "bold" and "underlined" within the 30-mer RNA sequence of [(5' → 3') ggguguuuug**uacag** | **AUUGUCU**GUAGCCGA] spanning the junction of "intron 1" and "exon 2" in the human TYR pre-mRNA.

A 17-mer PNA sequence of "(N→C) Fethoc-AC(103)T-GA(6)C-TA(6)T-CTG(6)-TAC(1O5)-A(4)A-NH₂" is equivalent to the DNA sequence of "(5' → 3') ACT-GAC-TAT-CTG-TAC-AA" for complementary binding to pre-mRNA. The 17-mer PNA has a 15-mer complementary overlap with the 15-mer sequence marked "bold" and "underlined" within the 30-mer RNA sequence of [(5' → 3') ggguguuuug**uacag** | **AU**"U"**GUC**"U"**GU**AGCCGA] spanning the junction of "intron 1" and "exon 2" in the human TYR pre-mRNA. The two mismatches were marked with a quote sign, i.e. " ".

An aspect of the present invention provides a pharmaceutical composition for treating diseases or conditions involving the expression of the human tyrosinase gene, comprising the

An aspect of the present invention provides a pharmaceutical composition for treating hyper-pigmentation, comprising the peptide nucleic acid derivative according to the present invention. Examples of hyper-pigmentation caused by melanin overproduction include melasma, acne scarring, liver spots, freckle, age spots and actinic damage.

Also disclosed herein is a method to treat diseases or conditions involving the expression of the human tyrosinase gene, comprising the administration of the peptide nucleic acid derivative according to the present invention. The route of the administration may be, for example, a topical administration.

Also disclosed herein is a method to treat hyper-pigmentation, comprising the administration of the peptide nucleic acid derivative according to the present invention. The route of the administration may be, for example, a topical administration.

An aspect of the present invention provides a use of the peptide nucleic acid derivative according to the present invention in the preparation of a medicament for treatment of diseases or conditions involving the expression of the human tyrosinase gene.

An aspect of the present invention provides a use of the peptide nucleic acid derivative according to the present invention in the preparation of a medicament for treatment of hyper-pigmentation.

### Brief Explanation of Drawings

**Figures 1a-1c****.** Examples of natural or unnatural (modified) nucleobases selectable for the peptide nucleic acid derivative of **Formula I.**
**Figures 2a-2e****.** Examples of substituents selectable for the peptide nucleic acid derivative of **Formula I.**
**Figure 3****.** Chemical structures of PNA monomers with natural or modified nucleobase.
**Figure 4****.** Chemical structures for abbreviations of N- or C-terminus substituents.
**Figures 5a****.** Chemical structure of 13-mer PNA derivative "(N→C) Fethoc-CA(5)G-ACA(5)-ATC(1O2)-TG(6)T-A(5)-NH₂".
**Figures 5b****.** Chemical structure of 15-mer PNA derivative "(N→C) Fethoc-AG(6)A-CA(5)A-TC(1O2)T-G(3O2)TA(5)-CA(2O2)A-NH₂".
**Figure 6****.** Chemical structures of Fmoc-PNA monomers used to synthesize the PNA derivatives of the present invention.
**Figures 7a-7b****.** C₁₈-reverse phase HPLC chromatograms of "ASO 1" before and after HPLC purification, respectively.
**Figure 8****.** ESI-TOF mass spectrum of "ASO 1" purified by C₁₈-RP prep HPLC.
**Figure 9a****.** Electrophoretic analysis of the nested PCR products in B16F10 mouse melanoma cells treated with 0 (negative control), 1, 10 or 1,000 aM "ASO 1M".
**Figure 9b****.** Sanger sequencing data for the PCR product assignable to the skipping of exons (2-3).
**Figure 10a****.** Changes in the full-length TYR mRNA level by qPCR in B16F10 mouse melanoma cells treated with "ASO 1M" at 0 (negative control), 1, 10, 100 or 1,000 aM. (error bar by standard error)
**Figure 10b****.** TYR western blot data in B16F10 mouse melanoma cells treated for 24 hours with "ASO 1M" at 0 zM (negative control), 10 zM, 100 zM, 1 aM, or 10 aM (i.e. 10,000 zM).
**Figure 11****.** Changes in the melanin content in B16F10 mouse melanoma cells treated either with "ASO 1M" at 1, 10, 100 or 1,000 aM, or with 10 *µ*g/mL or 100 *µ*g/mL arbutin. (error bar by standard error)
**Figure 12****.** Changes in the full-length TYR mRNA level by qPCR in human primary epithelial melanocytes treated with "ASO 1" at 0 zM (negative control), 1 zM, 100 zM or 10 aM. (error bar by standard error)

### Best mode for carrying out the invention

### General Procedures for Preparation of PNA Oligomers

PNA oligomers were synthesized by solid phase peptide synthesis (SPPS) based on Fmoc-chemistry according to the method disclosed in the prior art [US6,133,444; WO96/40685] with minor but due modifications. The solid support employed in this study was H-Rink Amide-ChemMatrix purchased from PCAS BioMatrix Inc. (Quebec, Canada). Fmoc-PNA monomers with a modified nucleobase were synthesized as described in the prior art [PCT/KR 2009/001256] or with minor modifications. Such Fmoc-PNA monomers with a modified nucleobase and Fmoc-PNA monomers with a naturally occurring nucleobase were used to synthesize the PNA derivatives of the present invention. PNA oligomers were purified by C₁₈-reverse phase HPLC (watre/acetonitrile or water/methanol with 0.1% TFA) and characterized by mass spectrometry including ESI/TOF/MS.

Scheme 2 illustrates a typical monomer elongation cycle adopted in the SPPS of this study, and the synthetic details are provided as below. To a skilled person in the field, however, there are lots of minor variations obviously possible in effectively running such SPPS reactions on an automatic peptide synthesizer or manual peptide synthesizer. Each reaction step in Scheme 2 is briefly provided as follows.

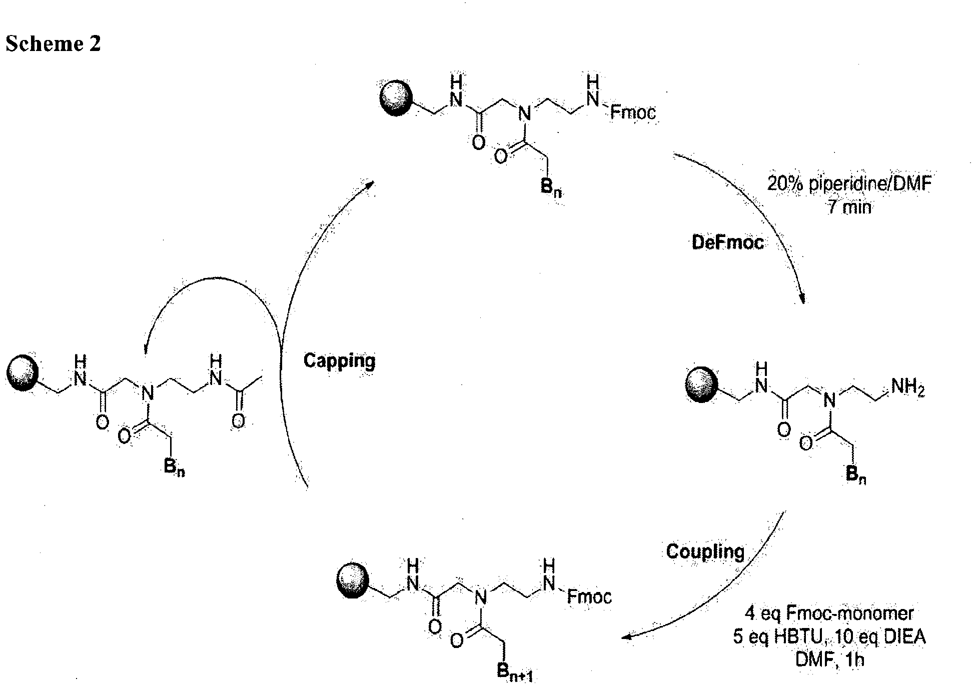

[Activation of H-Rink-ChemMatrix Resin] 0.01 mmol (ca 20 mg resin) of the ChemMatrix resin in 1.5 mL 20% piperidine/DMF was vortexed in a libra tube for 20 min, and the DeFmoc solution was filtered off. The resin was washed for 30 sec each in series with 1.5 mL methylene chloride (MC), 1.5 mL dimethylformamide (DMF), 1.5 mL MC, 1.5 mL DMF, and 1.5 mL MC. The resulting free amines on the solid support were subjected to coupling either with an Fmoc-PNA monomer or with an Fmoc-protected amino acid derivative.

[DeFmoc] The resin was vortexed in 1.5 mL 20% piperidine/DMF for 7 min, and the DeFmoc solution was filtered off. The resin was washed for 30 sec each in series with 1.5 mL MC, 1.5 mL DMF, 1.5 mL MC, 1.5 mL DMF, and 1.5 mL MC. The resulting free amines on the solid support were immediately subjected to coupling with an Fmoc-PNA monomer.

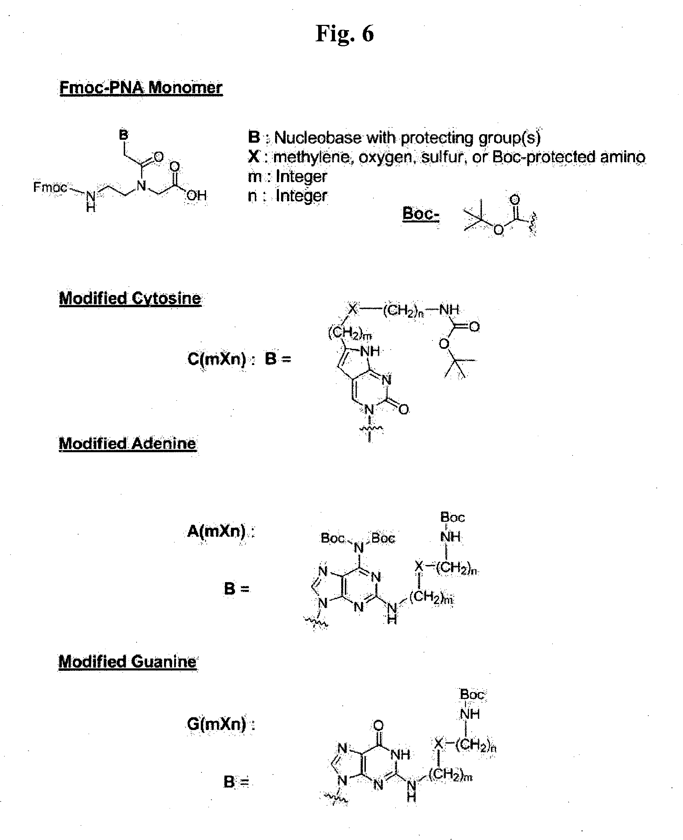

[Coupling with Fmoc-PNA Monomer] The free amines on the solid support were coupled with an Fmoc-PNA monomer as follows. 0.04 mmol of PNA monomer, 0.05 mmol HBTU, and 10 mmol DIEA were incubated for 2 min in 1 mL anhydrous DMF, and added to the resin with free amines. The resin solution was vortexed for 1 hour and the reaction medium was filtered off. Then the resin was washed for 30 sec each in series with 1.5 mL MC, 1.5 mL DMF, and 1.5 mL MC. The chemical structures of Fmoc-PNA monomers with a modified nucleobase used in the present invention are provided in Figure 6. The Fmoc-PNA monomers with a modified nucleobase are provided in Figure 6 should be taken as examples, and therefore should not be taken to limit the scope of the present invention. A skilled person in the field may easily figure out a number of variations in Fmoc-PNA monomers to synthesize the PNA derivative of **Formula I.**

[Capping] Following the coupling reaction, the unreacted free amines were capped by shaking for 5 min in 1.5 mL capping solution (5% acetic anhydride and 6% 2,6-leutidine in DMF). Then the capping solution was filtered off and washed for 30 sec each in series with 1.5 mL MC, 1.5 mL DMF, and 1.5 mL MC.

[Introduction of "Fethoc-" Radical in N-Terminus] "Fethoc-" radical was introduced to the N-terminus by reacting the free amine on the resin with "Fethoc-OSu" under basic coupling conditions. The chemical structure of "Fethoc-OSu" [CAS No. 179337-69-0, C₂₀H₁₇NO₅, MW 351.36] is provided as follows.

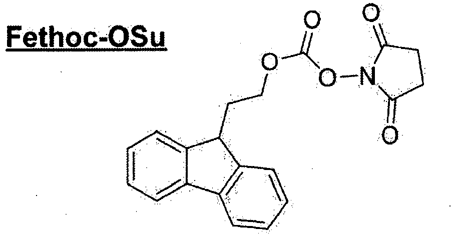

[Cleavage from Resin] PNA oligomers bound to the resin were cleaved from the resin by shaking for 3 hours in 1.5 mL cleavage solution (2.5% tri-isopropylsilane and 2.5% water in trifluoroacetic acid). The resin was filtered off and the filtrate was concentrated under reduced pressure. The resulting residue was triturated with diethylether and the resulting precipitate was collected by filtration for purification by reverse phase HPLC.

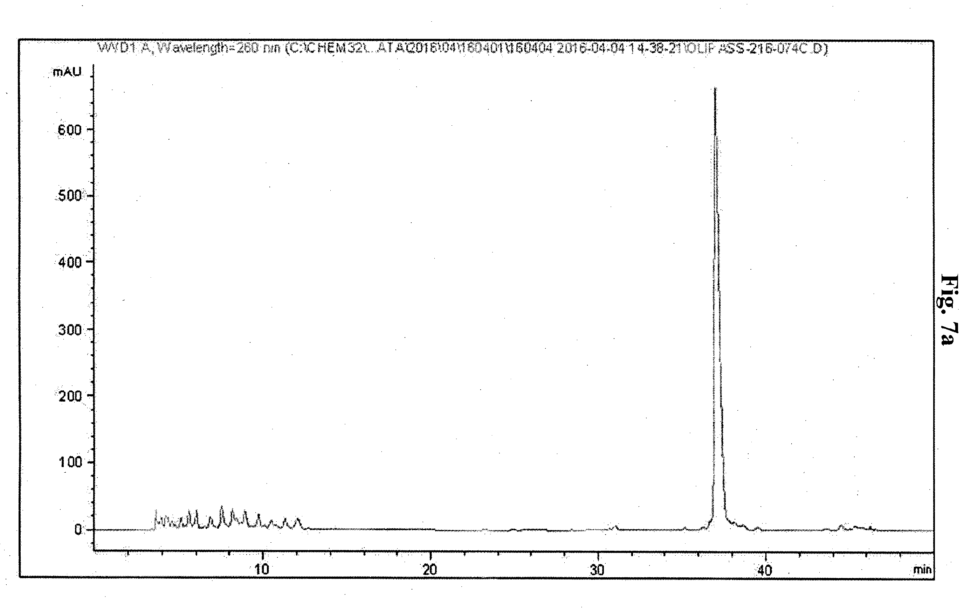

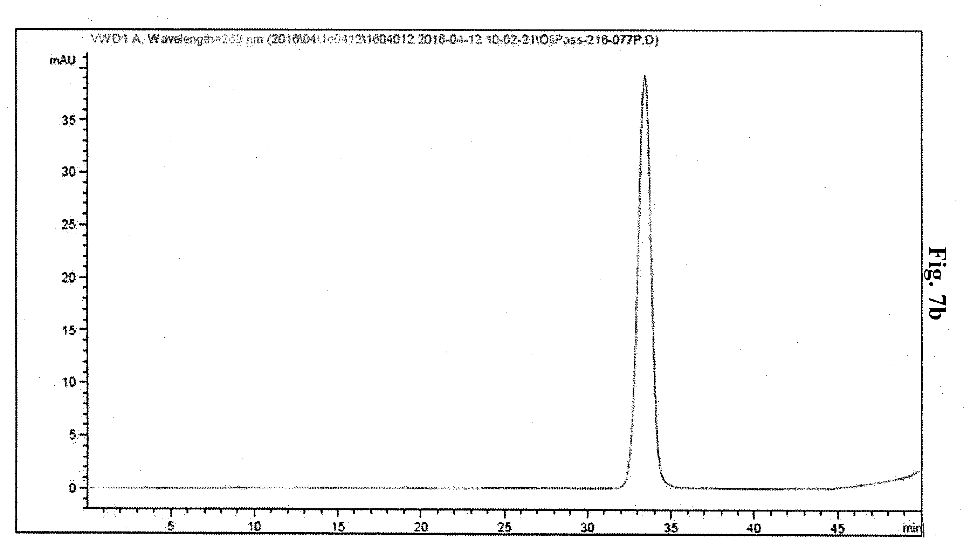

[HPLC Analysis and Purification] Following a cleavage from resin, the crude product of a PNA derivative was purified by C₁₈-reverse phase HPLC eluting water/acetonitrile or water/methanol (gradient method) containing 0.1% TFA. Figures 7a and 7b are exemplary HPLC chromatograms for "ASO 1" before and after HPLC purification, respectively. The oligomer sequence of "ASO 1" is as provided in Table 1.

### Synthetic Examples for PNA Derivative of Formula I

In order to complementarily target the 3' splice site of "exon 2" in the human TYR pre-mRNA, PNA derivatives of the present invention were prepared according to the synthetic procedures provided above or with minor modifications. Provision of such PNA derivatives targeting the human TYR pre-mRNA is to exemplify the PNA derivatives of **Formula I,** and should not be interpreted to limit the scope of the present invention.

**Table 1. PNA derivatives complementarily targeting the 3' splice site of "exon 2" in the human TYR pre-mRNA along with structural characterization data by mass spectrometry.**

| PNA Example | PNA Sequence (N→C) | Exact Mass, m/z | |
|---|---|---|---|
| | | theor.^{a} | obs.^{b} |
| ASO 1 | Fethoc-C A(5)G-ACA(5)-ATC(1O2)-_{T}G(6)T-A(5)-NH: | 4258.96 | 4260.99 |
| ASO 2 | Fethoc-AC(12)A-GA(5)C-AA(5)T-CTG(6)-TA(5)C(1O2)-AA(5)-NH₂ | 5532.55 | 5532.54 |
| ASO 3 | Fethoc-AC(1O2)A(5)-GA(5)C-AA(5)T-CTG(6)-C(1O2)C-NH₂. | 4592.11 | 4592.11 |

| | | | |
|---|---|---|---|
| ^{a)} theoretical exact mass, ^{b)} observed exact mass | | | |

Table 1 provides PNA derivatives complementarily targeting the 3' splice site of "exon 2" in the human TYR pre-mRNA read out from the human TYR gene [NCBI Reference Sequence: NG_0008748] along with structural characterization data by mass spectrometry. Provision of the TYR ASOs in Table 1 is to exemplify the PNA derivatives of **Formula I,** and should not be interpreted to limit the scope of the present invention.

"ASO 1" has a 13-mer complementary overlap with the 13-mer sequence marked "bold" and "underlined" within the 30-mer RNA sequence of [(5' → 3') ggguguuuug**uacag** | **AUUGU-CUG**UAGCCGA] spanning the junction of "intron 1" and "exon 2" in the human TYR pre-mRNA. Thus "ASO 1" possesses a 5-mer overlap with "intron 1" and a 8-mer overlap with "exon 2" within the human TYR pre-mRNA.

Table 2 provides "ASO 1M" complementarily targeting the 3' splice site of "exon 2" in the mouse TYR pre-mRNA read out from the mouse genomic DNA [accessed from NCBI Reference Sequence: NC_000073] along with structural characterization data by mass spectrometry. Provision of "ASO 1M" is to evaluate the antisense activity in cells of mouse origin.

**Table 2. PNA derivatives complementarily targeting the 3' splice site of "exon 2" in the mouse TYR pre-mRNA along with structural characterization data by mass spectrometry.**

| PNA Example | Exact Mass, m/z PNA Sequence (N→C) theor.^{a} obs.^{b} |
|---|---|
| ASO 1M | Fethoc-CA(5)A-A(5)TG-A(5)TC(102)-TG(6)T-G-NH₂ 4289.95 4289.96 |

| | |
|---|---|
| ^{a)} theoretical exact mass, ^{b)} observed exact mass | |

A 30-mer RNA sequence of [(5' → 3') aauuguuuuucacag | AUCAUUUGUAGCAGA] spanning the junction of intron 1 and exon 2 in the mouse TYR pre-mRNA was read out from the mouse TYR gene [NCBI Gene Accession Number: NC_000073].

"ASO 1M" is equivalent to the DNA sequence of "(5' → 3') CAA-ATG-ATC-TGT-G" for complementary binding to the pre-mRNA. "ASO 1M" has a 13-mer complementary overlap with the 13-mer sequence marked "bold" and "underlined" within the 30-mer RNA sequence of [(5' → 3') aauuguuuuu**cacag** | **AUCAUUUG**UAGCAGA] spanning the junction of "intron 1" and "exon 2" in the mouse TYR pre-mRNA.

Like "ASO 1" against the human TYR pre-mRNA, "ASO 1M" possesses a 5-mer overlap with "intron 1" and a 8-mer overlap with "exon 2" within the mouse TYR pre-mRNA. "ASO 1M" may serve as a good surrogate compound for the antisense activity of "ASO 1" in cells of human origin.

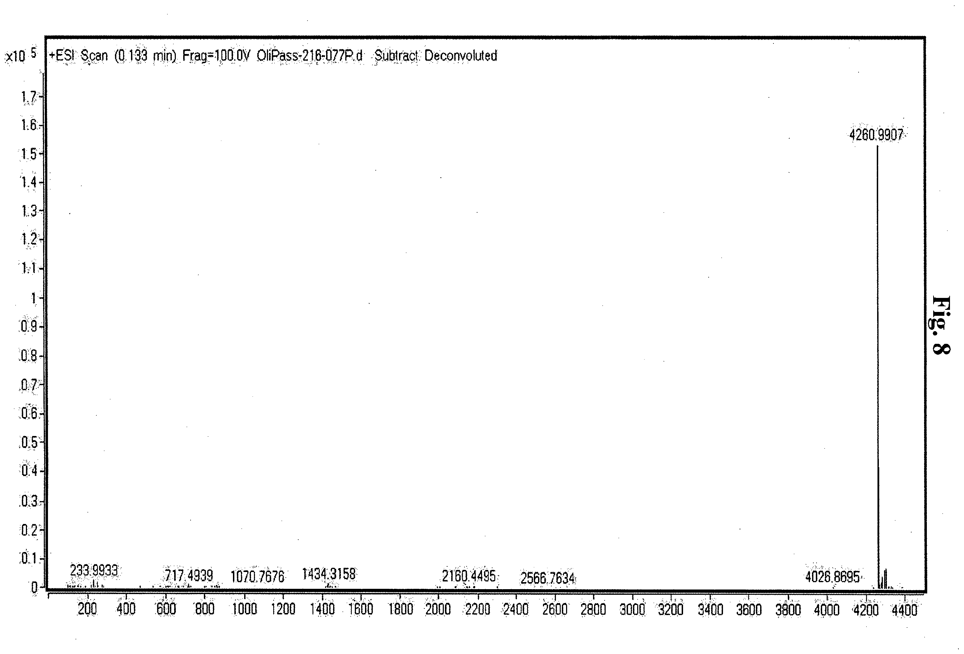

Figure 7a is a HPLC chromatogram obtained with a crude product of "ASO 1". The crude product was purified by C₁₈-RP preparatory HPLC. Figure 7b is a HPLC chromatogram for a purified product of "ASO 1". The purity of "ASO 1" improved markedly following the preparatory HPLC purification. Figure 8 provides a ESI-TOF mass spectrum obtained with the purified product of "ASO 1". Provision of the analysis data for "ASO 1" is to illustrate how the PNA derivatives of **Formula I** were purified and identified in the present invention, and should not be interpreted to limit the scope of the present invention.

### Binding Affinity of Model PNA Derivatives for Complementary DNA

The PNA derivatives in Tables 1 and 2 were evaluated for their binding affinity for 10-mer DNAs complementarily targeting either the N-terminal or C-terminal. The binding affinity was assessed by Tₘ value for the duplex between PNA and 10-mer complementary DNA. The duplex between PNA derivatives and fully complementary DNAs show Tₘ values too high to be reliably determined in aqueous buffer solution, since the buffer solution tends to boil during the Tₘ measurement.

Tₘ values were determined on a UV/Vis spectrometer as follows. A mixed solution of 4 µM PNA oligomer and 4 µM complementary 10-mer DNA in 4 mL aqueous buffer (pH 7.16, 10 mM sodium phosphate, 100 mM NaCl) in 15 mL polypropylene falcon tube was incubated at 90°C for a minute and slowly cooled down to ambient temperature. Then the solution was transferred into a 3 mL quartz UV cuvette equipped with an air-tight cap, and subjected to a Tₘ measurement at 260 nm on a UV/Visible spectrophotometer as described in the prior art [PCT/KR2009/001256] or with minor modifications. The 10-mer complementary DNAs for Tₘ measurement were purchased from Bioneer (www.bioneer.com, Dajeon, Republic of Korea) and used without further purification.

Observed Tₘ values of the PNA derivatives of **Formula I** are very high for a complementary binding to 10-mer DNA, and provided in Table 3. For example, "ASO 1" showed a Tₘ value of 78.0°C for the duplex with the 10-mer complementary DNA targeting the N-terminal 10-mer in the PNA as marked "bold" and "underlined" in [(N → C) Fethoc-**CA(5)G-ACA(5)-ATC(102)-T**G(6)T-A(5)-NH₂. In the meantime, "ASO 1" showed a Tₘ of 73.0°C for the duplex with the 10-mer complementary DNA targeting the C-terminal 10-mer in the PNA as marked "bold" and "underlined" in [(N → C) Fethoc-CA(5)G-**ACA(5)-ATC(102)-TG(6)T-A(5)**-NH₂].

**Table 3. Tₘ values between PNAs and 10-mer complementary DNA targeting either the N-terminal or the C-terminal of PNA.**

| PNA | °C Tₘ Value, °C | |
|---|---|---|
| | 10-mer DNA against N-Terminal | 10-mer DNA against C-Terminal. |
| ASO 1 | 78.0 | 73.0 |
| ASO 1M | 72.0 | 72.0 |

### Examples for Biological Activities of PNA Derivatives of Formula I

PNA derivatives in the present invention were evaluated for in vitro TYR antisense activities in B16F10 mouse melanoma cells and human melanocytes. The biological examples were provided as examples to illustrate the biological profiles of the PNA derivatives of **Formula I,** and therefore should not be interpreted to limit the scope of the current invention. **Example 1.** Exon Skipping Induced by "ASO 1M".

"ASO 1M" specified in Table 2 has a 13-mer complementary overlap with the 13-mer sequence marked "bold" and "underlined" within the 30-mer RNA sequence of [(5' → 3') aauuguuuuu**cacag** | **AUCAUUUG**UAGCAGA] spanning the junction of "intron 1" and "exon 2" in the mouse TYR pre-mRNA. In the meantime, "ASO 1M" possesses a 9-mer complementary along with 4 mismatches as marked "bold" and "underlined" within the 30-mer RNA sequence of [(5' → 3') ggguguuuug"u"**acag** | **AU**"UG"**U**"C"**UG**UAGCCGA] spanning the junction of "intron 1" and "exon 2" in the human TYR pre-mRNA. The 4 mismatches were marked with quote sign (" ").

"ASO 1M" was evaluated for its ability to induce the skipping of TYR "exon 2" in mouse B16F10 melanoma cells, even though "ASO 1M" possesses 4 mismatches with the 3' splice site of "exon 2" in the human TYR pre-mRNA. "ASO 1M", which is fully complementary to the mouse TYR pre-mRNA, may serve as a good surrogate compound for "ASO 1" which is fully complementary to the human TYR pre-mRNA.

[Cell Culture & ASO Treatment] B16F10 mouse melanoma cells (Cat. Number CRL-6475, ATCC) were maintained in DMEM (Dulbecco's modified Eagle's essential minimum medium) supplemented with 10% FBS, 1% streptomycin/penicillin, and 0.01 mg/ml bovine insulin. B16F10 cells grown in 60 mm culture dish containing 5 mL DMEM were incubated for 5 hours with "ASO 1M" at 0 (negative control), 1, 10,100 or 1000 aM.

[RNA Extraction & cDNA Synthesis by One-step PCR] Following an incubation for 5 hours, total RNA was extracted using "Universal RNA Extraction Kit" (Cat. Number 9767, Takara) according to the manufacturer's instructions. 200 ng of RNA template was used for a 25 *µ*ℓ reverse transcription reaction using Super Script^{®} One-Step RT-PCR kit with Platinum^{®} Taq polymerase (Cat. No. 10928-042, Invitrogen) against a set of exon-specific primers of [exon 1_forward: (5' → 3') GTAAGTTTGGATTTGGGG; and exon 4_reverse: (5' → 3') AGAGC-GGTATGAAAGGAA] according to the following cycle conditions: 50°C for 30 min and 94°C for 2 min, followed by 15 cycles of 30 sec at 94°C, 30 sec at 52°C, and 40 sec at 72°C.

[Nested PCR Amplification] 1 *µ*ℓ of cDNA was further amplified in a 20 *µ*ℓ nested PCR reaction (Cat. No. K2612, Bioneer) against a set of primers of [exon ln_forward: (5' → 3') GAGAACTAACTGGGGATGA; and exon 4n_reverse: (5' → 3') CGATAGGTGCATTGGCTT] according to the cycle conditions specified as follows: 95°C for 5 min followed by 30 cycles of 30 sec at 95°C, 30 sec at 52°C, and 40 sec at 72°C.

[Identification of Exon Skipping Products] The PCR products were subjected to electrophoretic separation on a 2% agarose gel. The bands of target size were collected and analyzed by Sanger Sequencing.

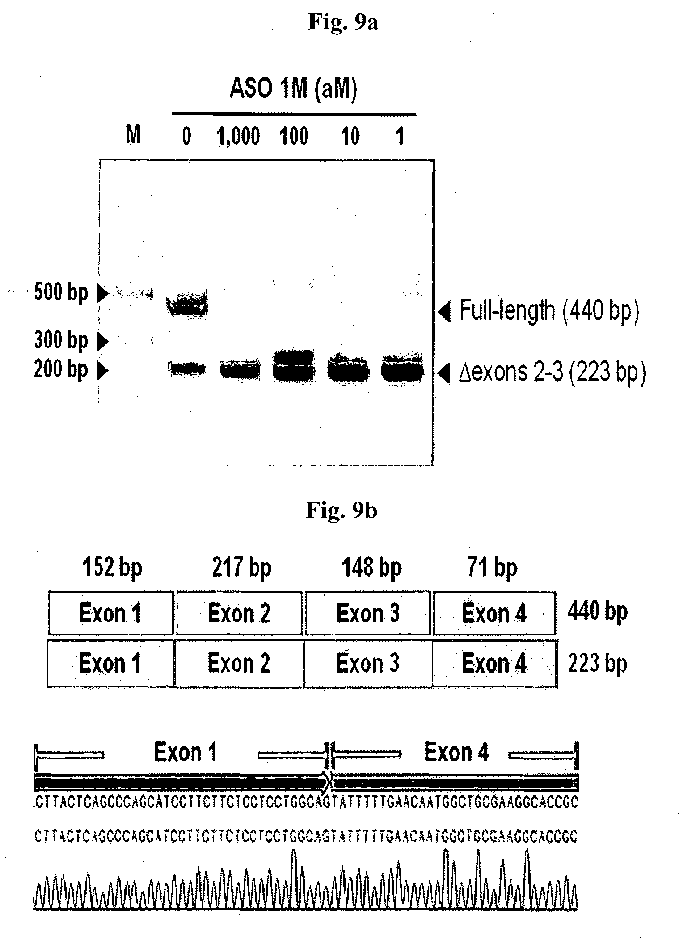

Figure 9a provides the electrophoresis data of the PCR products. The cells without "ASO 1M" treatment yielded two strong PCR bands, one for the full-length TYR mRNA and the other for the splice variant TYR mRNA lacking exons 2 and 3. Thus the skipping of exons 2-3 is considered to spontaneously occur. The cells treated with "ASO 1M" at 1 to 1,000 aM, however, yielded only the splice variant TYR mRNA lacking exons 2 and 3. Thus "ASO 1M" increases the propensity of the exons 2-3 skipping in B16F10 melanoma cells. The exon skipping PCR product was sequenced to be the skipping of exons 2-3 as shown in Figure 9b.

### Example 2. qPCR for TYR mRNA in B16F10 Mouse Melanoma Cells Treated with "ASO 1M".

"ASO 1M" was evaluated by TYR nested qPCR for its ability to induce changes in the mouse TYR mRNA level in B16F10 cells as described below.

[Cell Culture & ASO Treatment] B16F10 cells grown in 60 mm culture dish containing 5 mL DMEM were treated with "ASO 1M" at 0 (negative control), 1, 10,100 or 1000 aM. (2 culture dishes per dose)

[RNA Extraction & cDNA Synthesis by One-step PCR] Following an incubation with "ASO 1M" for 5 hours, total RNA was extracted from cells using "Universal RNA Extraction Kit" (Cat. No. 9767, Takara) according to the manufacturer's instructions. 200 ng of RNA template were used for a 25 *µ*ℓ reverse transcription reaction using One Step RT-PCR kit (Invitrogen, USA) against a set of exon specific primers of [exon 1_forward: (5' → 3') GTAAGTTTGGATTTGGGG; and exon 4_reverse: (5' → 3') AGAGCGGTATGAAAGGAA] according to the following cycle conditions: 50°C for 30 min and 94°C for 2 min, followed by 15 cycles of 30 sec at 94°C, 30 sec at 52°C, and 40 sec at 72°C.

[Nested qPCR Amplification] The cDNA solutions were diluted by 100 times, and 1 *µ*ℓ of each diluted PCR product was subjected to a 20 *µ*ℓ Real-Time PCR reaction with a Taqman probe set spanning the junction of exon 2 and exon 3 (Cat. No. Mm00495818_ml, Thermo Fisher Scientific) according to the following cycle conditions: 95°C for 3 min followed by 30 cycles 10 sec at 95°C, and 30 sec at 60°C.

[Statistical Analysis] The nested qPCR experiment was repeated independently four times, and individual mRNA levels from each experiment were normalized against the mRNA level without "ASO 1M" treatment. The mRNA levels obtained from all the 4 separate experiments were pooled for statistical analysis by student's t-test. Thus the number of RNA samples is 8 per concentration.

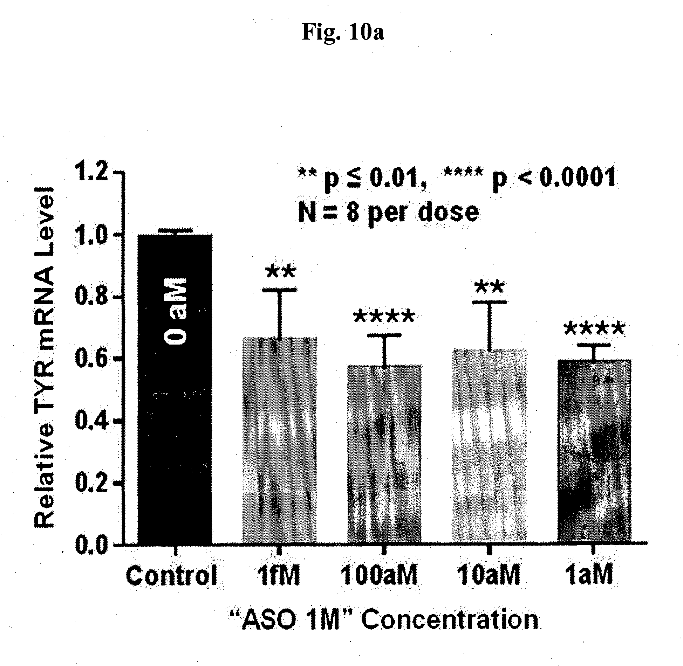

Figure 10a provides the pooled qPCR data, in which the full-length mRNA level significantly decreased by ca 40% in the cells treated with "ASO 1M" at 1 to 1,000 aM.

### Example 3. Inhibition of TYR Protein Expression in B16F10 Melanoma Cells by "ASO 1M".

"ASO 1M" was evaluated for its ability to down-regulate the TYR protein expression in B16F10 mouse melanoma cells as described below.

B16F10 cells grown in 60 mm culture dish containing 5 mL DMEM were treated with "ASO 1M" at 0 zM (negative control), 10 zM, 100 zM, 1 aM or 10 aM. 24 hours later, cells were washed 2 times with cold PBS (phosphate buffered saline), and then subjected to lysis with 200 *µ*ℓ 1X cell lysis buffer (Cat. No. 9803, Cell Signaling Tech) supplemented with 1X protease inhibitors cocktail (Cat. No. P8340, Sigma). 200 *µ*ℓ of each lysate in 1.5 mL e-tube was mixed with 100 *µ*ℓ 5X sample buffer, and boiled at 100°C for 5 min. 20 *µ*ℓ of the lysate was subjected to electrophoretic separation on a 4-15% gradient TGX gel (Cat No. 456-1086, Bio-Rad), and protein transfer on a 0.45 µm PVDF membrane. The membrane was probed with an anti-TYR antibody (Cat. No. 9319, Cell Signaling Tech) and anti-β-actin antibody (Cat. No. a3845, Sigma).

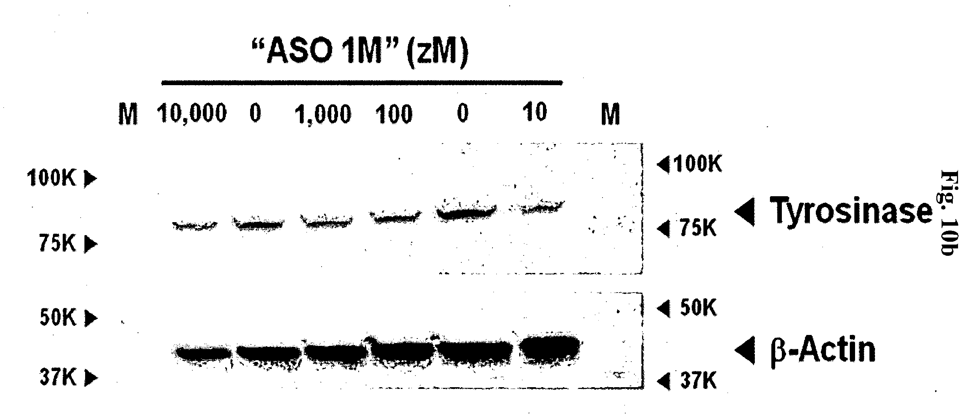

Figure 10b provides the western blot data for the TYR protein expression in B16F10 cells. The band intensity of TYR expression was considerably weaker in the lysates with "ASO 1M" treatment than in the lysates without "ASO 1M" treatment, i.e. negative control. The TYR protein and mRNA level decreased comparably by the ASO treatment. (cf. "Example 2")

### Example 4. Inhibition of Melanogenesis in B16F10 Melanoma Cells by "ASO 1M".

"ASO 1M" was evaluated for its ability to inhibit the melanogenesis in B16F10 mouse melanoma cells as described below.

B16F10 cells sub-cultured in 60 mm culture dish containing 5 mL DMEM were treated with nothing (negative control), with "ASO 1M" at 1 to 1,000 aM, or with 10 or 100 µg/mL arbutin as the positive control. (2 culture dishes per dose) 24 hours later, cells were washed 2 times with cold PBS, and subjected to lysis with 200 *µ*ℓ IN NaOH. Each lysate was collected in 1.5 mL e-tube, and kept overnight at room temperature. The melanin content in each lysate was determined by absorbance at 475 nm on an ELISA reader. The same experiment was repeated four times using cells at different passage. The four sets of melanin content data were pooled for statistical analysis by student's t-test against the melanin content without treatment (negative control).

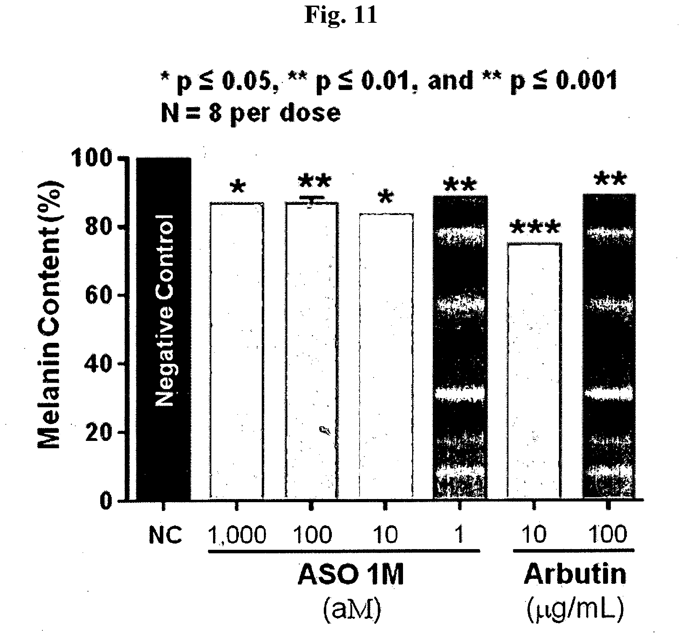

Figure 11 summarizes the changes of the melanin content in B16F10 cells following a 24 hours incubation either with "ASO 1M" or with arbutin. The melanin content significantly decreased ca by 15% and 25% in the cells treated with 10 µg/mL and 100 µg/mL arbutin, respectively. In case of the cells treated with "ASO 1M", the melanin content significantly decreased by ca 15% without much dose dependency. The inhibitory activity of "ASO 1M" was comparable to that of 10 µg/mL arbutin.

### Example 5. Preparation of Topical Cream Containing Compound of Formula I.

A compound of **Formula I,** for example "ASO 2" was formulated as a cream for topical application to subjects. The topical cream was preparation as described below. Given that there are lots of variations of topical cream possible, this preparation should be taken as an example and should not be interpreted to limit the scope of the current invention.

[Preparation of Solution A] In a beaker, were mixed deionized water (196 g), EDTA disodium (0.06 g), glycerin (15 g), dipropylene glycol (30 g), phenoxyethanol (0.9 g), ethylhexylglycerin (0.9 g), hydroxyethyl acrylate/sodium acryloyldimethyl taurate copolymer (2.4 g), and 1 nM aqueous solution of "ASO 2" (0.3 mL). The mixture was subjected to homogenization at 70 ∼ 75°C using a homo mixer.

[Preparation of Solution B] In another beaker, were mixed at 70 ∼ 75°C cetearyl ethylhexanoate (12 g), dimethicone (6 g), stearic acid/hydrogenated vegetable oil/behenyl alcohol/cetearyl alcohol (15 g), butyrospermum parkii (Shea) butter (9 g), and polyglyceryl-3-methylglucose distearate/glyceryl stearate SE/methyl glucose sesquistearate (6 g).

[Preparation of Topical Cream] "Solution A" and "Solution B" were mixed subjected to homogenization at 7,200 rpm and 70 ∼ 75°C. for 3 min using a homo mixer. The mixture was slowly cooled to room temperature to yield a topical cream containing 1 pM of "ASO 2".

### Example 6. qPCR for TYR mRNA in Human Melanocytes Treated with "ASO 1".

"ASO 1" specified in Table 1 is a 13-mer TYR ASO fully complementary to the 3' splice site spanning the junction of intron 1 and exon 2 in the human TYR as marked "bold" and "underlined" in the 30-mer human TYR pre-mRNA sequence of [(5' → 3') ggguguuuug**uacag** | **AUUGUCUG**UAGCCGA].

"ASO 1" was evaluated by TYR nested qPCR for its ability to induce changes in the TYR mRNA level in human primary epidermal melanocytes as follows.

[Cell Culture & ASO Treatment] Primary epidermal melanocytes (Cat. Number PCS-200-013, ATCC) cells were maintained in Dermal Cell Basal Medium (Cat Number PCS-200-030, ATCC) supplemented with Adult Melanocyte Growth Kit Component (Cat. Number PCS-200-042, ATCC). Melanocytes grown in 60 mm culture dish containing 5 mL culture medium were treated with "ASO 1" at 0 zM (negative control), 1 zM, 100 zM, or 10 aM. (3 culture dishes per concentration)

[RNA Extraction & cDNA Synthesis by One-step PCR] Following an incubation with "ASO 1" for 5 hours, total RNA was extracted using "RNeasy Mini Kit" (Cat. Number 74106, Qiagen) according to the manufacturer's instructions. 200 ng of RNA template was subjected to a 25 µL reverse transcription reaction using Super Script^{®} One-Step RT-PCR kit with Platinum^{®} Taq polymerase (Cat. No. 10928-042, Invitrogen) against a set of exon-specific primers of [exon 1_forward(2): (5' → 3') CTCTTTGTCTGGATGCATT; and exon 5_reverse: (5' → 3') CTGTGGTAATCCTCTTTCT] according to the following cycle conditions specified: 50 °C for 30 min and 94 °C for 2 min, which was followed by 15 cycles of 30 sec at 94 °C, 30 sec at 50 °C, and 1 min at 72 °C.

[Nested PCR Amplification] 1 µL of cDNA was further amplified in a 20 µL nested PCR reaction (Cat. No. K2612, Bioneer) against a set of exon-specific primers of [exon 2n_forward: (5' → 3') GATAAAGCTGCCAATTTC; and exon 3n_reverse: (5' → 3') TTGTGCATGCTGCTTTGA] against a Taqman probe [(5' → 3') 5,6-FAM-CACTGG-ZEN-AAGGATTTGCTAGTCCAC-3IABkFQ]. Cycle Conditions: 95 °C for 3 min followed by 40 cycles 10 sec at 95 °C, and 30 sec at 60 °C.

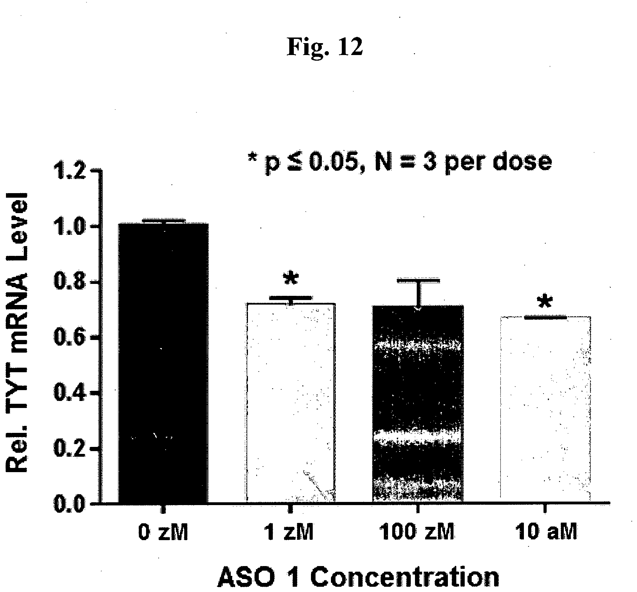

Figure 12 provides the qPCR data, in which the full-length TYR mRNA level decreased by ca 30% in the human melanocytes treated with "ASO 1" at 1 zM to 10 aM. The observed decreases were significant (student's t-test) in the cells treated with "ASO 1" at 1 zM and 10 aM.

## Claims

1. A peptide nucleic acid derivative represented by Formula I, or a pharmaceutically acceptable salt thereof: 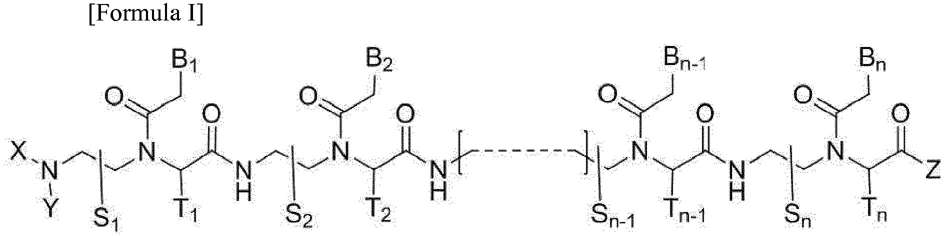 wherein,
n is an integer between 10 and 21;
the compound of **Formula I** possesses at least a 10-mer complementary overlap with the 14-mer pre-mRNA sequence of [(5' → 3') UGUACAGAUUGUCU] in the human tyrosinase pre-mRNA;
the compound of **Formula I** is fully complementary to the human tyrosinase pre-mRNA, or partially complementary to the human tyrosinase pre-mRNA with one or two mismatches from the entire compound of **Formula I;**
S₁, S₂, ···, Sₙ₋₁, Sₙ, T₁, T₂, ···, Tₙ₋₁, and Tₙ independently represent deuterido, hydrido, substituted or non-substituted alkyl, or substituted or non-substituted aryl radical;
X and Y independently represent deuterido, hydrido [H], formyl [H-C(=O)-], aminocarbonyl [NH₂-C(=O)-], aminothiocarbonyl [NH₂-C(=S)-], substituted or non-substituted alkyl, substituted or non-substituted aryl, substituted or non-substituted alkylacyl, substituted or non-substituted arylacyl, substituted or non-substituted alkyloxycarbonyl, substituted or non-substituted aryloxycarbonyl, substituted or non-substituted alkylaminocarbonyl, substituted or non-substituted arylaminocarbonyl, substituted or non-substituted alkylaminothiocarbonyl, substituted or non-substituted arylaminothiocarbonyl, substituted or non-substituted alkyloxythiocarbonyl, substituted or non-substituted aryloxythiocarbonyl, substituted or non-substituted alkylsulfonyl, substituted or non-substituted arylsulfonyl, substituted or non-substituted alkylphosphonyl radical, or substituted or non-substituted arylphosphonyl radical;
Z represents hydrido, hydroxy, substituted or non-substituted alkyloxy, substituted or non-substituted aryloxy, substituted or non-substituted amino, substituted or non-substituted alkyl, or substituted or non-substituted aryl radical;
B₁, B₂, ···, Bₙ₋₁, and Bₙ are independently selected from natural nucleobases including adenine, thymine, guanine, cytosine and uracil, and unnatural nucleobases; and,
at least four of B₁, B₂, ···, Bₙ₋₁, and Bₙ are independently selected from unnatural nucleobases with a substituted or non-substituted amino radical covalently linked to the nucleobase moiety.

2. The peptide nucleic acid derivative according to claim 1, or a pharmaceutically acceptable salt thereof:
wherein,
n is an integer between 10 and 21;
the compound of **Formula I** possesses at least a 10-mer complementary overlap with the 14-mer pre-mRNA sequence of [(5' → 3') UGUACAGAUUGUCU] in the human tyrosinase pre-mRNA;
the compound of **Formula I** is fully complementary to the human tyrosinase pre-mRNA, or partially complementary to the human tyrosinase pre-mRNA with one or two mismatches from the entire compound of Formula I;
S₁, S₂, ···, Sₙ₋₁, Sₙ, T₁, T₂, ···, Tₙ₋₁, and Tₙ independently represent deuterido or hydrido radical;
X and Y independently represent deuterido, hydrido, formyl, aminocarbonyl, aminothiocarbonyl, substituted or non-substituted alkyl, substituted or non-substituted aryl, substituted or non-substituted alkylacyl, substituted or non-substituted arylacyl, substituted or non-substituted alkyloxycarbonyl, substituted or non-substituted aryloxycarbonyl, substituted or non-substituted alkylaminocarbonyl, substituted or non-substituted arylaminocarbonyl, substituted or non-substituted alkylaminothiocarbonyl, substituted or non-substituted arylaminothiocarbonyl, substituted or non-substituted alkyloxythiocarbonyl, substituted or non-substituted aryloxythiocarbonyl, substituted or non-substituted alkylsulfonyl, substituted or non-substituted arylsulfonyl, substituted or non-substituted alkylphosphonyl radical, or substituted or non-substituted arylphosphonyl radical;
Z represents hydrido, hydroxy, substituted or non-substituted alkyloxy, substituted or non-substituted aryloxy, or substituted or non-substituted amino radical;
B₁, B₂, ···, Bₙ₋₁, and Bₙ are independently selected from natural nucleobases including adenine, thymine, guanine, cytosine and uracil, and unnatural nucleobases;
at least four of B₁, B₂, ···, Bₙ₋₁, and Bₙ are independently selected from unnatural nucleobases represented by **Formula II, Formula III,** or **Formula IV:** 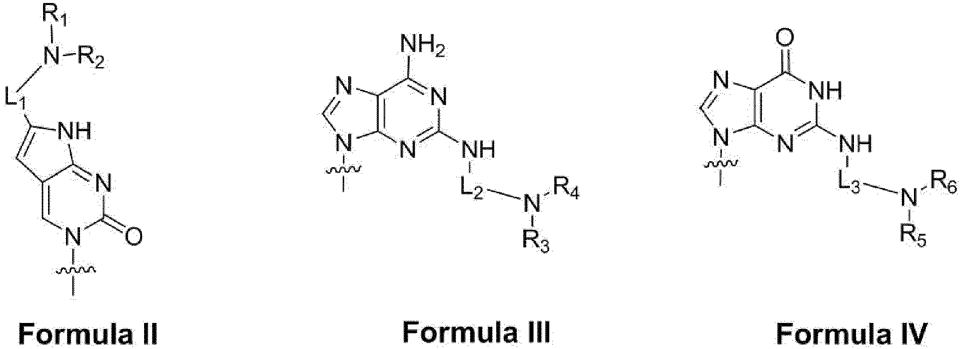
wherein,
R₁, R₂, R₃, R₄, R₅ and R₆ are independently selected from hydrido, and substituted or non-substituted alkyl radical;
L₁, L₂ and L₃ are a covalent linker represented by **Formula V** covalently linking the basic amino group to the nucleobase moiety: 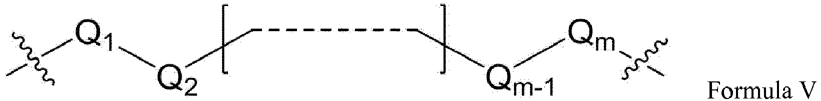
wherein,
Q₁ and Qₘ are substituted or non-substituted methylene (-CH₂-) radical, and Qₘ is directly linked to the basic amino group;
Q₂, Q₃, ···, and Qₘ₋₁ are independently selected from substituted or non-substituted methylene, oxygen (-O-), sulfur (-S-), and substituted or non-substituted amino radical [-N(H)-, or -N(substituent)-]; and,
m is an integer between 1 and 15.

3. The peptide nucleic acid derivative according to claim 2, or a pharmaceutically acceptable salt thereof:
wherein,
n is an integer between 11 and 18;
the compound of **Formula I** possesses at least a 10-mer complementary overlap with the 14-mer pre-mRNA sequence of [(5' → 3') UGUACAGAUUGUCU] in the human tyrosinase pre-mRNA;
the compound of **Formula I** is fully complementary to the human tyrosinase pre-mRNA;
S₁, S2, ···, Sₙ₋₁, Sₙ, T₁, T₂, ···, Tₙ₋₁, and Tn are hydrido radical;
X and Y independently represent hydrido, substituted or non-substituted alkyl, substituted or non-substituted aryl, substituted or non-substituted alkylacyl, substituted or non-substituted arylacyl, substituted or non-substituted alkyloxycarbonyl, or substituted or non-substituted aryloxycarbonyl radical;
Z represents substituted or non-substituted amino radical;
B₁, B₂, ···, Bₙ₋₁, and are independently selected from natural nucleobases including adenine, thymine, guanine, cytosine and uracil, and unnatural nucleobases;
at least four of B₁, B₂, ···, Bₙ₋₁, and are independently selected from unnatural nucleobases represented by **Formula II, Formula III,** or **Formula IV;**
R₁, R₂, R₃, R₄, R₅ and R₆ are independently selected from hydrido, and substituted or non-substituted alkyl radical;
Q₁ and Qₘ are substituted or non-substituted methylene radical, and Qₘ is directly linked to the basic amino group;
Q₂, Q₃, ···, and Qₘ₋₁ are independently selected from substituted or non-substituted methylene, oxygen, and amino radical; and,
m is an integer between 1 and 11.

4. The peptide nucleic acid derivative according to claim 3, or a pharmaceutically acceptable salt thereof:
wherein,
n is an integer between 11 and 16;
the compound of **Formula I** possesses at least a 10-mer complementary overlap with the 14-mer pre-mRNA sequence of [(5' → 3') UGUACAGAUUGUCU] in the human tyrosinase pre-mRNA;
the compound of **Formula I** is fully complementary to the human tyrosinase pre-mRNA;
S₁, S₂, ···, Sₙ₋₁, Sₙ, T₁, T₂, ···, Tₙ₋₁, and Tₙ are hydrido radical;
X and Y independently selected from hydrido, substituted or non-substituted alkylacyl, or substituted or non-substituted alkyloxycarbonyl radical;
Z represents substituted or non-substituted amino radical;
B₁, B₂, ..., Bₙ₋₁, and are independently selected from natural nucleobases including adenine, thymine, guanine, cytosine and uracil, and unnatural nucleobases;
at least four of B₁, B₂, ···, Bₙ₋₁, and are independently selected from unnatural nucleobases represented by **Formula II, Formula III,** or **Formula IV;**
R₁, R₂, R₃, R₄, R₅ and R₆ are independently selected from hydrido, and substituted or non-substituted alkyl radical;
Q₁ and Qₘ are methylene radical, and Qₘ is directly linked to the basic amino group;
Q₂, Q₃, ···, and Qₘ₋₁ are independently selected from methylene, oxygen, and amino radical; and,
m is an integer between 1 and 9.

5. The peptide nucleic acid derivative according to claim 4, or a pharmaceutically acceptable salt thereof:
wherein,
n is an integer between 11 and 16;
the compound of **Formula I** possesses at least a 10-mer complementary overlap with the 14-mer pre-mRNA sequence of [(5' → 3') UGUACAGAUUGUCU] in the human tyrosinase pre-mRNA;
the compound of **Formula I** is fully complementary to the human tyrosinase pre-mRNA;
S₁, S₂, ···, Sₙ₋₁, Sₙ, T₁, T₂, ···, Tₙ₋₁, and Tn are hydrido radical;
X and Y independently selected from hydrido, substituted or non-substituted alkylacyl, or substituted or non-substituted alkyloxycarbonyl radical;
Z represents substituted or non-substituted amino radical;
B₁, B₂, ···, Bₙ₋₁, and Bₙ are independently selected from natural nucleobases including adenine, thymine, guanine, cytosine and uracil, and unnatural nucleobases;
at least four of B₁, B₂, ···, Bₙ₋₁, and Bₙ are independently selected from unnatural nucleobases represented by **Formula II, Formula III,** or **Formula IV;**
R₁, R₃, and R₅ are hydrido radical, and R₂, R₄, and R₆ independently represent hydrido, or substituted or non-substituted alkyl radical;
Q₁ and Qₘ are methylene radical, and Qₘ is directly linked to the basic amino group;
Q₂, Q₃, ···, and Qₘ₋₁ are independently selected from methylene, oxygen radical; and, m is an integer between 1 and 9.

6. The peptide nucleic acid derivative according to claim 5, or a pharmaceutically acceptable salt thereof:
wherein,
n is an integer between 11 and 16;
the compound of **Formula I** possesses at least a 10-mer complementary overlap with the 14-mer pre-mRNA sequence of [(5' → 3') UGUACAGAUUGUCU] in the human tyrosinase pre-mRNA;
the compound of **Formula I** is fully complementary to the human tyrosinase pre-mRNA;
S₁, S₂, ···, Sₙ₋₁, Sₙ, T₁, T₂, ···, Tₙ₋₁, and Tn are hydrido radical;
X and Y independently selected from hydrido, substituted or non-substituted alkylacyl, or substituted or non-substituted alkyloxycarbonyl radical;
Z represents substituted or non-substituted amino radical;
B₁, B₂, ···, Bₙ₋₁, and Bₙ are independently selected from adenine, thymine, guanine, cytosine, and unnatural nucleobases;
at least five of B₁, B₂, ···, Bₙ₋₁, and Bₙ are independently selected from unnatural nucleobases represented by **Formula II, Formula III,** or **Formula IV;**
R₁, R₂ , R₃, R₄, R₅, and R₆ are hydrido radical;
Q₁ and Qₘ are methylene radical, and Qₘ is directly linked to the basic amino group;
Q₂, Q₃, ···, and Qₘ₋₁ are independently selected from methylene, and oxygen radical; and,
m is an integer between 1 and 9.

7. The peptide nucleic acid derivative according to claim 6, or a pharmaceutically acceptable salt thereof:
wherein,
n is an integer between 11 and 16;
the compound of **Formula I** possesses at least a 10-mer complementary overlap with the 14-mer pre-mRNA sequence of [(5' → 3') UGUACAGAUUGUCU] in the human tyrosinase pre-mRNA;
the compound of **Formula I** is fully complementary to the human tyrosinase pre-mRNA;
S₁, S₂, ···, Sₙ₋₁, Sₙ, T₁, T₂, ···, Tₙ₋₁, and Tn are hydrido radical;
X is hydrido radical;
Y represents substituted or non-substituted alkylacyl, or substituted or non-substituted alkyloxycarbonyl radical;
Z represents substituted or non-substituted amino radical;
B₁, B₂, ···, Bₙ₋₁, and Bₙ are independently selected from adenine, thymine, guanine, cytosine, and unnatural nucleobases;
at least five of B₁, B₂, ···, Bₙ₋₁, and Bₙ are independently selected from unnatural nucleobases represented by **Formula II, Formula III,** or **Formula IV;**
R₁, R₂, R₃, R₄, Rₛ, and R₆ are hydrido radical;
L₁ represents -(CH₂)₂-O-(CH₂)₂-, -CH₂-O-(CH₂)₂-, -CH₂-O-(CH₂)₃-, -CH₂-O-(CH₂)₄-, or -CH₂-O-(CH₂)₅- with the right end is directly linked to the basic amino group; and,
L₂ and L₃ are independently selected from -(CH₂)₂-O-(CH₂)₂-, -(CH₂)₃-O-(CH₂)₂-, -(CH₂)₂-O-(CH₂)₃-, -(CH₂)₂-, -(CH₂)₃-, -(CH₂)₄-, -(CH₂)₅-, -(CH₂)₆-, -(CH₂)₇-, and -(CH₂)₈- with the right end is directly linked to the basic amino group.

8. The peptide nucleic acid derivative according to claim 1, which is selected from the group of peptide nucleic acid derivatives provided below, or a pharmaceutically acceptable salt thereof:
(N→C) Fethoc-CA(5)G-ACA(5)-ATC(1O2)-TG(6)T-A(5)-NH₂;
(N→C) Fethoc-AC(1/2)A(5)-GA(5)C-AA(5)T-CTG(6)-C(1/2)C-NH₂;
(N→C) Ac-CA(5)G-ACA(5)-ATC(1O2)-TG(6)T-A(5)-NH₂;
(N→C)Benzoyl-CA(5)G-ACA(5)-ATC(1O2)-TG(6)T-A(5)-NH₂;
(N→C) Piv-CA(5)G-ACA(5)-ATC(1O2)-TG(6)T-A(5)-NH₂;
(N→C) Methyl-CA(5)G-ACA(5)-ATC(1O2)-TG(6)T-A(5)-NH₂;
(N→C) n-Propyl-CA(5)G-ACA(5)-ATC(1O2)-TG(6)T-A(5)-NH₂;
(N→C) Fmoc-CA(5)G-ACA(5)-ATC(1O2)-TG(6)T-A(5)-NH₂;
(N→C) p-Toluenesulfonyl-CA(5)G-ACA(5)-ATC(1O2)-TG(6)T-A(5)-NH₂;
(N→C) H-Lys-CA(5)G-ACA(5)-ATC(1O2)-TG(6)T-A(5)-NH₂;
(N→C) Fethoc-Lys-Gly-CA(5)G-ACA(5)-ATC(1O2)-TG(6)T-A(5)-NH₂;
(N→C) Fethoc-CA(5)G-ACA(5)-ATC(1O2)-TG(6)T-A(5)-Lys-NH₂;
(N→C) Fethoc-Val-Gly-CA(5)G-ACA(5)-ATC(1O2)-TG(6)T-A(5)-NH₂;
(N→C) Ac-Arg-CA(5)G-ACA(5)-ATC(1O2)-TG(6)T-A(5)-NH₂;
(N→C) Benzyl-CA(5)G-ACA(5)-ATC(1O2)-TG(6)T-A(5)-NH₂;
(N→C) Phenyl-CA(5)G-ACA(5)-ATC(1O2)-TG(6)T-A(5)-NH₂;
) [N-(2-Phenylethyl)amino]carbonyl-CA(5)G-ACA(5)-ATC(1O2)-TG(6)T-A(5)-NH₂;
(N→C) Benzoyl-Leu-CA(5)G-ACA(5)-ATC(1O2)-TG(6)T-A(5)-NH₂;
(N→C) Piv-Lys-CA(5)G-ACA(5)-ATC(1O2)-TG(6)T-A(5)-Val-NH₂;
(N→C) Fethoc-CA(7)G-AC(2O2)A-A(4)TC(1O2)-TG(6)T-A-NH₂;
) Fethoc-CTG(6)-ACA(5)-ATC(1O2)-TG(6)T-A(5)-NH₂;
(N→C) Fethoc-CA(5)G-ATA(5)-ATC(1O2)-TG(5)T-A(5)-NH₂;
(N→C) Fethoc-TA(5)G-ACA(5)-ATC(1O2)-TG(6)T-A(5)-NH₂;
(N→C)Fethoc-TCA(3)-GAC(1O5)-A(5)AT-C(1O2)TG(5)-TA(5)-NH₂;
(N→C) Fethoc-CA(5)G-ACA(5)-ATC(1O2)-TG(6)T-A(5)C-NH₂;
(N→C) Fethoc-AG(6)A-CA(5)A-TC(1O2)T-G(6)TA(5)-C-NH₂;
(N→C) Fethoc-AG(6)A-CA(5)A-TC(1O2)T-G(6)TA(5)-CA(2O2)A-NH₂;
(N→C) Fethoc-AC(1O2)A-GA(6)C-AA(6)T-CTG(6)-TA(6)C(1O2)-AA(6)-NH₂;
(N→C)Fethoc-AC(1O3)T-GA(6)C-TA(6)T-CTG(6)-TAC(1O5)-A(4)A-NH₂;
(N→C)Fethoc-GA(6)C-AA(6)T-CTG(6)-TA(6)C(1O2)-AA(6)-NH₂;
(N→C)Fethoc-AC(1O2)A-GA(6)C-AA(6)T-CTG(6)-TA(6)C-A(5)AA(2O2)-A-NH₂;
(N→C) Fethoc-AA(6)T-CTG(6)-TA(6)C-A(5)AA(2O2)-A-NH₂;
(N→C)Fethoc-AC(1O2)A-GA(6)C-AA(6)T-CTG(6)-TA(6)C-A(5)AA(2O3)-A-NH₂;
(N→C) Fethoc-GC(1O4)T-AC(1O2)A-GA(4)C-AAT-CTG(6)-TA(6)C-NH₂;
(N→C) Fethoc-GC(1O2)T-A(3)CA-GA(4)C-AAT-C(1O2)TG-NH₂;
(N→C) Fethoc-GC(1O2)T-A(3)CA-G(2O2)AC-A(5)AT-C(1O2)TG-NH₂; and
(N →C) Fethoc-GC(1O2)T-A(3)CA-GA(4)C-A(2O2)AT-C(1O2)TG-NH₂:
wherein,
A, G, T, and C are PNA monomers with a natural nucleobase of adenine, guanine, thymine, and cytosine, respectively;
C(pOq), A(p), A(pOq), G(p), and G(pOq) are PNA monomers with an unnatural nucleobase represented by **Formula VI, Formula VII, Formula VIII, Formula IX,** and **Formula X,** respectively; 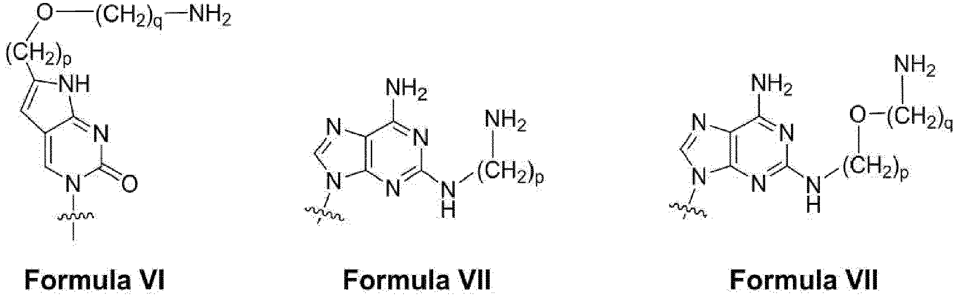 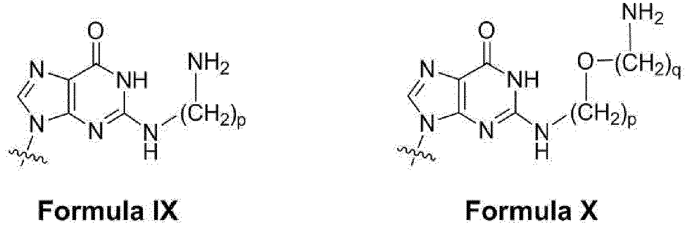
wherein,
p and q are integers; and,
the abbreviations for the N- and C-terminus substituents are as specifically described as follows: "Fmoc-" is the abbreviation for "[(9-fluorenyl)methyloxy]carbonyl-"; "Fethoc-" for "[2-(9-fluorenyl)ethyl-1-oxy]carbonyl"; "Ac-" for "acetyl-"; "Benzoyl-" for "benzenecabonyl-"; "Piv-" for "pivalyl-"; "Methyl-" for "methyl-"; "n-Propyl-" for "1-(n-propyl)-"; "H-" for "hydrido-" group; "p-Toluenesulfonyl" for "(4-methylbenzene)-1-sulfonyl-"; "-Lys-" for amino acid residue "lysine"; "-Val-" for amino acid residue "valine"; "-Leu-" for amino acid residue "leucine"; "-Arg-" for amino acid residue "arginine"; "-Gly-" for amino acid residue "glycine"; "[N-(2-Phenylethyl)amino]carbonyl-" for "[N-1-(2-phenylethyl)amino]carbonyl-"; "Benzyl-" for "1-(phenyl)methyl-"; "Phenyl-" for "phenyl-"; "Me-" for "methyl-"; and "-NH₂" for non-subsituted "-amino" group.

9. A pharmaceutical composition for treating diseases or conditions involving the expression of the human tyrosinase gene, comprising the peptide nucleic acid derivative according to claim 1, or a pharmaceutically acceptable salt thereof.

10. A pharmaceutical composition for treating hyper-pigmentation, comprising the peptide nucleic acid derivative according to claim 1, or a pharmaceutically acceptable salt thereof.

11. A peptide nucleic acid derivative according to claim 1 or a pharmaceutically acceptable salt thereof for use in a method to treat diseases or conditions involving the expression of the human tyrosinase gene, comprising the administration of said peptide nucleic acid derivative or said pharmaceutically acceptable salt thereof.

12. A peptide nucleic acid derivative according to claim 1 or a pharmaceutically acceptable salt thereof for use in a method to treat hyper-pigmentation, comprising the administration of said peptide nucleic acid derivative or said pharmaceutically acceptable salt thereof.

13. A use of the peptide nucleic acid derivative according to claim 1, or a pharmaceutically acceptable salt thereof, in the preparation of a medicament for treatment of diseases or conditions involving the expression of the human tyrosinase gene.

14. A use of the peptide nucleic acid derivative according to claim 1, or a pharmaceutically acceptable salt thereof, in the preparation of a medicament for treatment of hyper-pigmentation.

## Patentansprüche

1. Peptidnukleinsäurederivat, dargestellt durch die Formel I oder ein pharmazeutisch verträgliches Salz davon: 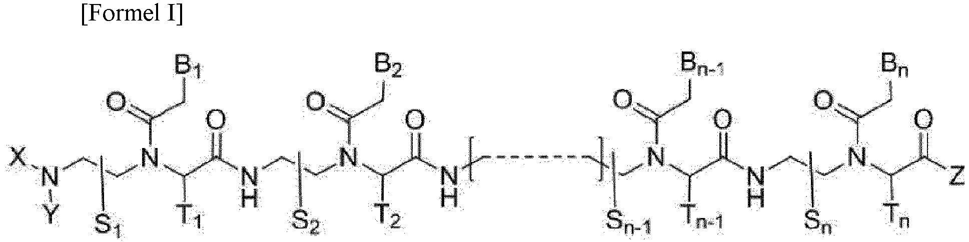 wobei,
n eine ganze Zahl zwischen 10 und 21 ist;
die Verbindung der **Formel I** mindestens eine komplementäre 10-mer-Überlappung mit der 14-mer-prä-mRNA-Sequenz von [(5' → 3')UGUACAGAUUGUCU] in der Human-Tyrosinase-prä-mRNA aufweist;
die Verbindung der **Formel I** vollständig komplementär zu der Human-Tyrosinase-prä-mRNA oder teilweise komplementär zu der Human-Tyrosinase-prä-mRNA mit einer oder zwei Fehlpaarungen gegenüber der gesamten Verbindung der **Formel I** ist;
S₁, S₂, ..., Sₙ₋₁, Sₙ, T₁, T₂, ..., Tₙ₋₁ und Tn unabhängig für einen Deuterido-, Hydrido-, substituierten oder nicht-substituierten Alkyl- oder substituierten oder nicht-substituierten Arylrest stehen;
X und Y unabhängig für einen Deuterido-, Hydrido- [H-], Formyl- [HC(=O)-], Aminocarbonyl- [NH₂-C(=O)-], Aminothiocarbonyl- [NH₂-C(=S)-], substituierten oder nicht-substituierten Alkyl-, substituierten oder nicht-substituierten Aryl-, substituierten oder nicht-substituierten Alkylacyl-, substituierten oder nicht-substituierten Arylacyl-, substituierten oder nicht-substituierten Alkyloxycarbonyl-, substituierten oder nicht-substituierten Aryloxycarbonyl-, substituierten oder nicht-substituierten Alkylaminocarbonyl-, substituierten oder nicht-substituierten Arylaminocarbonyl-, substituierten oder nicht-substituierten Alkylaminothiocarbonyl-, substituierten oder nicht-substituierten Arylaminothiocarbonyl-, substituierten oder nicht-substituierten Alkyloxythiocarbonyl-, substituierten oder nicht-substituierten Aryloxythiocarbonyl-, substituierten oder nicht-substituierten Alkylsulfonyl-, substituierten oder nicht-substituierten Arylsulfonyl-, substituierten oder nicht-substituierten Alkylphosphonylrest oder substituierten oder nicht-substituierten Arylphosphonylrest stehen;
Z für einen Hydrido-, Hydroxy-, substituierten oder nicht-substituierten Alkyloxy-, substituierten oder nicht substituierten Aryloxy-, substituierten oder nicht-substituierten Amino-, substituierten oder nicht-substituierten Alkyl- oder substituierten oder nicht-substituierten Arylrest steht;
B₁, B₂, ..., Bₙ₋₁ und Bₙ unabhängig aus natürlichen Nukleobasen, einschließlich Adenin, Thymin, Guanin, Cytosin und Uracil, und unnatürlichen Nukleobasen ausgewählt sind; und
mindestens vier von B₁, B₂, ..., Bₙ₋₁ und Bₙ unabhängig aus unnatürlichen Nukleobasen mit einem substituierten oder nicht-substituierten Aminorest, der kovalent an die Nukleobasegruppierung gebunden ist, ausgewählt sind.

2. Peptidnukleinsäurederivat nach Anspruch 1 oder ein pharmazeutisch verträgliches Salz davon:
wobei,
n eine ganze Zahl zwischen 10 und 21 ist;
die Verbindung der **Formel I** mindestens eine komplementäre 10-mer-Überlappung mit der 14-mer-prä-mRNA-Sequenz von [(5' → 3')UGUACAGAUUGUCU] in der Human-Tyrosinase-prä-mRNA aufweist;
die Verbindung der **Formel I** vollständig komplementär zu der Human-Tyrosinase-prä-mRNA oder teilweise komplementär zu der Human-Tyrosinase-prä-mRNA mit einer oder zwei Fehlpaarungen gegenüber der gesamten Verbindung der Formel I ist;
S₁, S₂, ..., Sₙ₋₁, Sₙ, T₁, T₂, ..., Tₙ₋₁ und Tn unabhängig für einen Deuterido- oder Hydridorest stehen;
X und Y unabhängig für einen Deuterido-, Hydrido-, Formyl-, Aminocarbonyl-, Aminothiocarbonyl, substituierten oder nicht-substituierten Alkyl-, substituierten oder nicht-substituierten Aryl-, substituierten oder nicht-substituierten Alkylacyl-, substituierten oder nicht-substituierten Arylacyl-, substituierten oder nicht-substituierten Alkyloxycarbonyl-, substituierten oder nicht-substituierten Aryloxycarbonyl-, substituierten oder nicht-substituierten Alkylaminocarbonyl-, substituierten oder nicht-substituierten Arylaminocarbonyl-, substituierten oder nicht-substituierten Alkylaminothiocarbonyl-, substituierten oder nicht-substituierten Arylaminothiocarbonyl-, substituierten oder nicht-substituierten Alkyloxythiocarbonyl-, substituierten oder nicht-substituierten Aryloxythiocarbonyl-, substituierten oder nicht-substituierten Alkylsulfonyl-, substituierten oder nicht-substituierten Arylsulfonyl-, substituierten oder nicht-substituierten Alkylphosphonylrest oder substituierten oder nicht-substituierten Arylphosphonylrest stehen;
Z für einen Hydrido-, Hydroxy-, substituierten oder nicht-substituierten Alkyloxy-, substituierten oder nicht substituierten Aryloxy- oder substituierten oder nicht-substituierten Aminorest steht;
B₁, B₂, ..., Bₙ₋₁ und Bₙ unabhängig aus natürlichen Nukleobasen, einschließlich Adenin, Thymin, Guanin, Cytosin und Uracil, und unnatürlichen Nukleobasen ausgewählt sind;
mindestens vier von B₁, B₂, ..., Bₙ₋₁ und Bₙ unabhängig aus unnatürlichen Nukleobasen dargestellt durch **Formel II, Formel III** oder **Formel IV** ausgewählt sind: 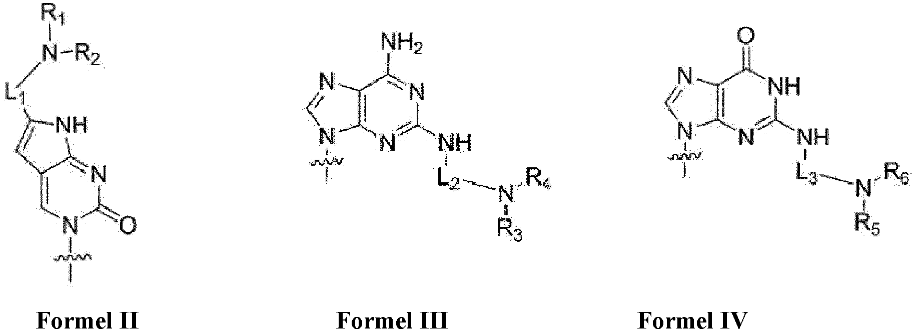
wobei,
R₁, R₂, R₃, R₄, R₅ und R₆ unabhängig aus einem Hydrido- und einem substituierten oder nicht-substituierten Alkylrest ausgewählt sind;
L₁, L₂ und L₃ ein kovalenter Linker sind, der durch **Formel V** dargestellt wird, der die basische Aminogruppe kovalent an die Nukleobasengruppierung bindet: 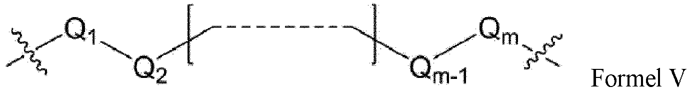
wobei,
Q₁ und Qₘ ein substituierter oder nicht-substituierter Methylen(-CH₂-)-Rest sind und Qₘ direkt an die basische Aminogruppe gebunden ist;
Q₂, Q₃, ... und Qₘ₋₁ unabhängig aus einem substituierten oder nicht-substituierten Methylen-, Sauerstoff- (-O-), Schwefel- (-S-) und substituierten oder nicht-substituierten Aminorest [-N(H)- oder -N(Substituent)-] ausgewählt sind; und
m eine ganze Zahl zwischen 1 und 15 ist.

3. Peptidnukleinsäurederivat nach Anspruch 2 oder ein pharmazeutisch verträgliches Salz davon:
wobei,
n eine ganze Zahl zwischen 11 und 18 ist;
die Verbindung der **Formel I** mindestens eine komplementäre 10-mer-Überlappung mit der 14-mer-prä-mRNA-Sequenz von [(5' → 3')UGUACAGAUUGUCU] in der Human-Tyrosinase-prä-mRNA aufweist;
die Verbindung der **Formel I** vollständig komplementär zu der Human-Tyrosinase-prä-mRNA ist;
S₁, S₂, ..., Sₙ₋₁, Sₙ, T₁, T₂, ..., Tₙ₋₁ und Tn ein Hydridorest sind;
X und Y unabhängig für einen Hydrido-, substituierten oder nicht-substituierten Alkyl-, substituierten oder nicht-substituierten Aryl-, substituierten oder nicht-substituierten Alkylacyl-, substituierten oder nicht-substituierten Arylacyl-, substituierten oder nicht-substituierten Alkyloxycarbonyl- oder substituierten oder nicht-substituierten Aryloxycarbonylrest stehen;
Z für einen substituierten oder nicht-substituierten Aminorest steht;
B₁, B₂, ..., Bₙ₋₁ und Bₙ unabhängig aus natürlichen Nukleobasen, einschließlich Adenin, Thymin, Guanin, Cytosin und Uracil, und unnatürlichen Nukleobasen ausgewählt sind;
mindestens vier von B₁, B₂, ..., Bₙ₋₁ und Bₙ unabhängig aus unnatürlichen Nukleobasen dargestellt durch **Formel II, Formel III** oder **Formel IV** ausgewählt sind;
R₁, R₂, R₃, R₄, R₅ und R₆ unabhängig aus einem Hydrido- und einem substituierten oder nicht-substituierten Alkylrest ausgewählt sind;
Q₁ und Qₘ ein substituierter oder nicht-substituierter Methylenrest sind und Qₘ direkt an die basische Aminogruppe gebunden ist;
Q₂, Q₃, ... und Qₘ₋₁ unabhängig aus einem substituierten oder nicht-substituierten Methylen-, Sauerstoff- und Aminorest ausgewählt sind; und
m eine ganze Zahl zwischen 1 und 11 ist.

4. Peptidnukleinsäurederivat nach Anspruch 3 oder ein pharmazeutisch verträgliches Salz davon:
wobei,
n eine ganze Zahl zwischen 11 und 16 ist;
die Verbindung der **Formel I** mindestens eine komplementäre 10-mer-Überlappung mit der 14-mer-prä-mRNA-Sequenz von [(5' → 3')UGUACAGAUUGUCU] in der Human-Tyrosinase-prä-mRNA aufweist;
die Verbindung der **Formel I** vollständig komplementär zu der Human-Tyrosinase-prä-mRNA ist;
S₁, S₂, ..., Sₙ₋₁, Sₙ, T₁, T₂, ..., Tₙ₋₁ und Tn ein Hydridorest sind;
X und Y unabhängig aus einem Hydrido-, substituierten oder nicht-substituierten Alkylacyl-oder substituierten oder nicht-substituierten Alkyloxycarbonylrest ausgewählt sind;
Z für einen substituierten oder nicht-substituierten Aminorest steht;
B₁, B₂, ..., Bₙ₋₁ und Bₙ unabhängig aus natürlichen Nukleobasen, einschließlich Adenin, Thymin, Guanin, Cytosin und Uracil, und unnatürlichen Nukleobasen ausgewählt sind;
mindestens vier von B₁, B₂, ..., Bₙ₋₁ und Bₙ unabhängig aus unnatürlichen Nukleobasen dargestellt durch **Formel II, Formel III** oder **Formel IV** ausgewählt sind;
R₁, R₂, R₃, R₄, R₅ und R₆ unabhängig aus einem Hydrido- und einem substituierten oder nicht-substituierten Alkylrest ausgewählt sind;
Q₁ und Qₘ ein Methylenrest sind und Qₘ direkt an die basische Aminogruppe gebunden ist;
Q₂, Q₃, ... und Qₘ₋₁ unabhängig aus einem Methylen-, Sauerstoff- und Aminorest ausgewählt sind; und
m eine ganze Zahl zwischen 1 und 9 ist.

5. Peptidnukleinsäurederivat nach Anspruch 4 oder ein pharmazeutisch verträgliches Salz davon:
wobei,
n eine ganze Zahl zwischen 11 und 16 ist;
die Verbindung der **Formel I** mindestens eine komplementäre 10-mer-Überlappung mit der 14-mer-prä-mRNA-Sequenz von [(5' → 3')UGUACAGAUUGUCU] in der Human-Tyrosinase-prä-mRNA aufweist;
die Verbindung der **Formel I** vollständig komplementär zu der Human-Tyrosinase-prä-mRNA ist;
S₁, S₂, ..., Sₙ₋₁, Sₙ, T₁, T₂, ..., Tₙ₋₁ und Tn ein Hydridorest sind;
X und Y unabhängig aus einem Hydrido-, substituierten oder nicht-substituierten Alkylacyl-oder substituierten oder nicht-substituierten Alkyloxycarbonylrest ausgewählt sind;
Z für einen substituierten oder nicht-substituierten Aminorest steht;
B₁, B₂, ..., Bₙ₋₁ und Bₙ unabhängig aus natürlichen Nukleobasen, einschließlich Adenin, Thymin, Guanin, Cytosin und Uracil, und unnatürlichen Nukleobasen ausgewählt sind;
mindestens vier von B₁, B₂, ..., Bₙ₋₁ und Bₙ unabhängig aus unnatürlichen Nukleobasen dargestellt durch **Formel II, Formel III** oder **Formel IV** ausgewählt sind;
R₁, R₃ und R₅ ein Hydridorest sind, und R₂, R₄ und R₆ unabhängig für einen Hydrido- oder einen substituierten oder nicht-substituierten Alkylrest stehen;
Q₁ und Qₘ ein Methylenrest sind und Qₘ direkt an die basische Aminogruppe gebunden ist;
Q₂, Q₃, ... und Qₘ₋₁ unabhängig aus einem Methylen-, Sauerstoffrest ausgewählt sind; und m eine ganze Zahl zwischen 1 und 9 ist.

6. Peptidnukleinsäurederivat nach Anspruch 5 oder ein pharmazeutisch verträgliches Salz davon:
wobei,
n eine ganze Zahl zwischen 11 und 16 ist;
die Verbindung der **Formel I** mindestens eine komplementäre 10-mer-Überlappung mit der 14-mer-prä-mRNA-Sequenz von [(5' → 3')UGUACAGAUUGUCU] in der Human-Tyrosinase-prä-mRNA aufweist;
die Verbindung der **Formel I** vollständig komplementär zu der Human-Tyrosinase-prä-mRNA ist;
S₁, S₂, ..., Sₙ₋₁, Sₙ, T₁, T₂, ..., Tₙ₋₁ und Tn ein Hydridorest sind;
X und Y unabhängig aus einem Hydrido-, substituierten oder nicht-substituierten Alkylacyl-oder substituierten oder nicht-substituierten Alkyloxycarbonylrest ausgewählt sind;
Z für einen substituierten oder nicht-substituierten Aminorest steht;
B₁, B₂, ..., Bₙ₋₁ und Bn unabhängig aus Adenin, Thymin, Guanin, Cytosin und unnatürlichen Nukleobasen ausgewählt sind;
mindestens fünf von B₁, B₂, ..., Bₙ₋₁ und Bₙ unabhängig aus unnatürlichen Nukleobasen dargestellt durch **Formel II, Formel III** oder **Formel IV** ausgewählt sind;
R₁, R₂, R₃, R₄, R₅ und R₆ ein Hydridorest sind;
Q₁ und Qₘ ein Methylenrest sind und Qₘ direkt an die basische Aminogruppe gebunden ist;
Q₂, Q₃, ... und Qₘ₋₁ unabhängig aus einem Methylen- und einem Sauerstoffrest ausgewählt sind; und
m eine ganze Zahl zwischen 1 und 9 ist.

7. Peptidnukleinsäurederivat nach Anspruch 6 oder ein pharmazeutisch verträgliches Salz davon:
wobei,
n eine ganze Zahl zwischen 11 und 16 ist;
die Verbindung der **Formel I** mindestens eine komplementäre 10-mer-Überlappung mit der 14-mer-prä-mRNA-Sequenz von [(5' → 3')UGUACAGAUUGUCU] in der Human-Tyrosinase-prä-mRNA aufweist;
die Verbindung der **Formel I** vollständig komplementär zu der Human-Tyrosinase-prä-mRNA ist;
S₁, S₂, ..., Sₙ₋₁, Sₙ, T₁, T₂, ..., Tₙ₋₁ und Tn ein Hydridorest sind;
X ein Hydridorest ist;
Y für einen substituierten oder nicht-substituierten Alkylacyl- oder substituierten oder nicht-substituierten Alkyloxycarbonylrest steht;
Z für einen substituierten oder nicht-substituierten Aminorest steht;
B₁, B₂, ..., Bₙ₋₁ und Bₙ unabhängig aus Adenin, Thymin, Guanin, Cytosin und unnatürlichen Nukleobasen ausgewählt sind;
mindestens fünf von B₁, B₂, ..., Bₙ₋₁ und Bₙ unabhängig aus unnatürlichen Nukleobasen dargestellt durch **Formel II, Formel III** oder **Formel IV** ausgewählt sind;
R₁, R₂, R₃, R₄, R₅ und R₆ ein Hydridorest sind;
L₁ für -(CH₂)₂-O-(CH₂)₂-, -CH₂-O-(CH₂)₂-, -CH₂-O-(CH₂)₃-, -CH₂-O-(CH₂)₄- oder -CH₂-O-(CH₂)₅- steht, wobei das rechte Ende direkt an die basische Aminogruppe gebunden ist; und
L₂ und L₃ unabhängig aus -(CH₂)₂-O-(CH₂)₂-, -(CH₂)₃-O-(CH₂)₂-,-(CH₂)₂-O-(CH₂)₃-, - (CH₂)₂-, -(CH₂)₃-, -(CH₂)₄-, -(CH₂)₅-, -(CH₂)₆-, -(CH₂)₇- und -(CH₂)₈- ausgewählt sind, wobei das rechte Ende direkt an die basische Aminogruppe gebunden ist.

8. Peptidnukleinsäurederivat nach Anspruch 1, das aus der unten bereitgestellten Gruppe von Peptidnukleinsäurederivaten ausgewählt ist, oder ein pharmazeutisch verträgliches Salz davon:
(N→C) Fethoc-CA(5)G-ACA(5)-ATC(1O2)-TG(6)T-A(5)-NH₂;
(N→C) Fethoc-AC(1/2)A(5)-GA(5)C-AA(5)T-CTG(6)-C(1/2)C-NH₂;
(N→C) Ac-CA(5)G-ACA(5)-ATC(1O2)-TG(6)T-A(5)-NH₂;
(N→C) Benzoyl-CA(5)G-ACA(5)-ATC(1O2)-TG(6)T-A(5)-NH₂;
(N→C)Piv-CA(5)G-ACA(5)-ATC(1O2)-TG(6)T-A(5)-NH₂;
(N→C) Methyl-CA(5)G-ACA(5)-ATC(1O2)-TG(6)T-A(5)-NH₂;
(N→C) n-Propyl-CA(5)G-ACA(5)-ATC(1O2)-TG(6)T-A(5)-NH₂;
(N→C) Fmoc-CA(5)G-ACA(5)-ATC(1O2)-TG(6)T-A(5)-NH₂;
(N→C) p-Toluolsulfonyl-CA(5)G-ACA(5)-ATC(1O2)-TG(6)T-A(5)-NH₂;
(N→C) H-Lys-CA(5)G-ACA(5)-ATC(1O2)-TG(6)T A(5)-NH₂;
(N→C) Fethoc-Lys-Gly-CA(5)G-ACA(5)-ATC(1O2)-TG(6)T-A(5)-NH₂;
(N→C) Fethoc-CA(5)G-ACA(5)-ATC(1O2)-TG(6)T-A(5)-Lys-NH₂;
(N→C) Fethoc-Val-Gly-CA(5)G-ACA(5)-ATC(1O2)-TG(6)T-A(5)-NH₂;
(N→C) Ac-Arg-CA(5)G-ACA(5)-ATC(1O2)-TG(6)T-A(5)-NH₂;
(N→C) Benzyl-CA(5)G-ACA(5)-ATC(1O2)-TG(6)T-A(5)-NH₂;
(N→C) Phenyl-CA(5)G-ACA(5)-ATC(1O2)-TG(6)T-A(5)-NH₂;
(N→C) [N-(2-Phenylethyl)amino]carbonyl-CA(5)G-ACA(5)-ATC(1O2)-TG(6)T-A(5)-NH₂;
(N→C) Benzoyl-Leu-CA(5)G-ACA(5)-ATC(102)-TG(6)T-A(5)-NH₂;
(N→C) Piv-Lys-CA(5)G-ACA(5)-ATC(1O2)-TG(6)T-A(5)-Val-NH₂;
(N→C) Fethoc-CA(7)G-AC(2O2)A-A(4)TC(1O2)-TG(6)T-A-NH₂;
(N→C) Fethoc-CTG(6)-ACA(5)-ATC(1O2)-TG(6)T-A(5)-NH₂;
(N→C) Fethoc-CA(5)G-ATA(5)-ATC(1O2)-TG(5)T-A(5)-NH₂;
(N→C) Fethoc-TA(5)G-ACA(5)-ATC(1O2)-TG(6)T-A(5)-NH₂;
(N→C) Fethoc-TCA(3)-GAC(1O5)-A(5)AT-C(1O2)TG(5)-TA(5)-NH₂;
(N→C) Fethoc-CA(5)G-ACA(5)-ATC(1O2)-TG(6)T-A(5)C-NH₂;
(N→C) Fethoc-AG(6)A-CA(5)A-TC(1O2)TG(6)TA(5)-C-NH₂;
(N→C) Fethoc-AG(6)A-CA(5)A-TC(1O2)TG(6)TA(5)-CA(2O2)A-NH₂;
(N→C) Fethoc-AC(1O2)A-GA(6)C-AA(6)T-CTG(6)-TA(6)C(1O2)-AA(6)-NH₂;
(N→C) Fethoc-AC(103)T-GA(6)C-TA(6)T-CTG(6)-TAC(105)-A(4)A-NH₂;
(N→C) Fethoc-GA(6)C-AA(6)T-CTG(6)-TA(6)C(1O2)-AA(6)-NH₂;
(N→C)Fethoc-AC(1O2)A-GA(6)C-AA(6)T-CTG(6)-TA(6)C-A(5)AA(2O2)-A-NH₂;
(N→C) Fethoc-AA(6)T-CTG(6)-TA(6)C-A(5)AA(2O2)-A-NH₂;
(N→C) Fethoc-AC(1O2)A-GA(6)C-AA(6)T-CTG(6)-TA(6)C-A(5)AA(2O3)-A-NH₂;
(N→C) Fethoc-GC(1O4)T-AC(1O2)A-GA(4)C-AAT-CTG(6)-TA(6)C-NH₂;
(N→C) Fethoc-GC(102)T-A(3)CA-GA(4)C-AAT-C(102)TG-NH₂;
(N→C) Fethoc-GC(1O2)T-A(3)CA-G(2O2)AC-A(5)AT-C(1O2)TG-NH₂; und
(N→C) Fethoc-GC(1O2)T-A(3)CA-GA(4)C-A(2O2)AT-C(1O2)TG-NH₂:
wobei,
A, G, T und C PNA-Monomere mit einer natürlichen Nukleobase von Adenin, Guanin, Thymin bzw. Cytosin sind;
C(pOq), A(p), A(pOq), G(p) und G(pOq) PNA-Monomere mit einer unnatürlichen Nukleobase sind, die durch die **Formel VI, Formel VII, Formel VIII, Formel IX** bzw. **Formel X** dargestellt ist; 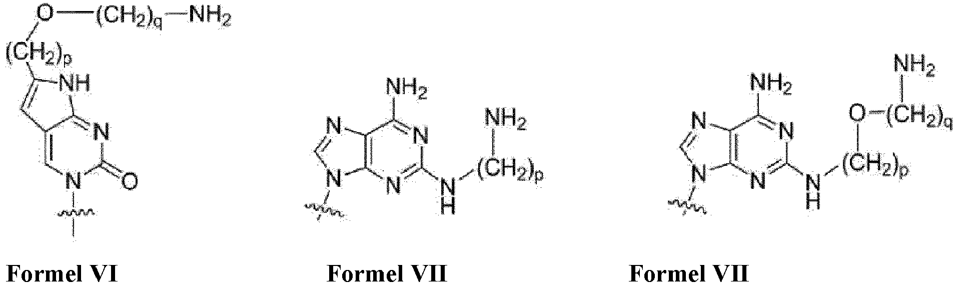 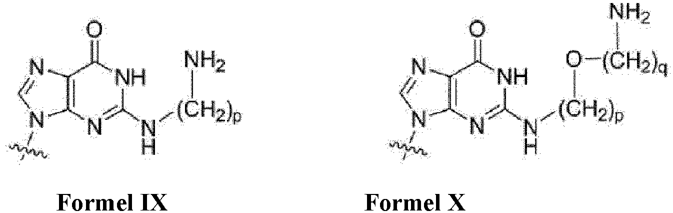
wobei,
p und q ganze Zahlen sind; und
die Abkürzungen für die N- und C-terminalen Substituenten konkret wie folgt beschrieben sind: "Fmoc-" ist die Abkürzung für "[(9-Fluorenyl)methyloxy]carbonyl-"; "Fethoc-" für "[2-(9-Fluorenyl)ethyl-1-oxy]carbonyl"; "Ac-" für "Acetyl-"; "Benzoyl-" für "Benzolcarbonyl-"; "Piv-" für "Pivalyl-"; "Methyl-" für "Methyl-" "; "n-Propyl-" für "1-(n-Propyl)-", "H-" für "Hydrido-"-Gruppe; "p-Toluensulfonyl" für "(4-Methylbenzol)-1-sulfonyl-"; "-Lys-" für den Aminosäurerest "Lysin"; "-Val-" für den Aminosäurerest "Valin"; "-Leu-" für den Aminosäurerest "Leucin"; "-Arg-" für den Aminosäurerest "Arginin "; "-Gly-" für den Aminosäurerest "Glycin"; "[N-(2-Phenylethyl)amino]carbonyl-" für "[N-1-(2-Phenylethyl)amino]carbonyl-", "Benzyl-" für "1-(Phenyl)methyl-", "Phenyl-" für "Phenyl-", "Me-" für "Methyl-" und "-NH₂" für nicht-substituierte "-Amino"-Gruppe.

9. Pharmazeutische Zusammensetzung zum Behandeln von Krankheiten oder Zuständen, welche die Expression des Human-Tyrosinase-Gens beinhalten, umfassend das Peptidnukleinsäurederivat nach Anspruch 1 oder ein pharmazeutisch verträgliches Salz davon.

10. Pharmazeutische Zusammensetzung zum Behandeln von Hyperpigmentierung, umfassend das Peptidnukleinsäurederivat nach Anspruch 1 oder ein pharmazeutisch verträgliches Salz davon.

11. Peptidnukleinsäurederivat nach Anspruch 1 oder ein pharmazeutisch verträgliches Salz davon zur Verwendung in einem Verfahren zum Behandeln von Krankheiten oder Zuständen, welche die Expression des Human-Tyrosinase-Gens beinhalten, umfassend die Verabreichung des Peptidnukleinsäurederivats oder des pharmazeutisch verträglichen Salzes davon.

12. Peptidnukleinsäurederivat nach Anspruch 1 oder ein pharmazeutisch verträgliches Salz davon zur Verwendung in einem Verfahren zum Behandeln von Hyperpigmentierung, umfassend die Verabreichung des Peptidnukleinsäurederivats oder des pharmazeutisch verträglichen Salzes davon.

13. Verwendung des Peptidnukleinsäurederivats nach Anspruch 1 oder eines pharmazeutisch verträglichen Salzes davon bei der Herstellung eines Medikaments zur Behandlung von Krankheiten oder Zuständen, welche die Expression des Human-Tyrosinase-Gens beinhalten.

14. Verwendung des Peptidnukleinsäurederivats nach Anspruch 1 oder eines pharmazeutisch verträglichen Salzes davon zur Herstellung eines Medikaments zur Behandlung von Hyperpigmentierung.

## Revendications

1. Dérivé d'acide nucléique peptidique représenté par la **Formule I,** ou sel pharmaceutiquement acceptable de celui-ci : 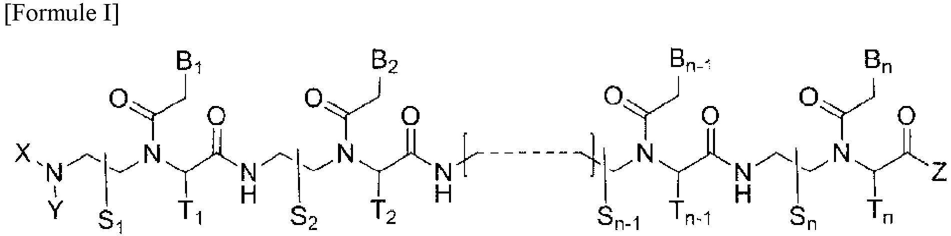 où
n est un nombre entier entre 10 et 21 ;
le composé de **Formule I** possède au moins un chevauchement complémentaire de 10-mer avec la séquence d'un pré-ARNm de 14-mer de [(5' → 3') UGUACAGAUUGUCU] dans le pré-ARNm de tyrosinase humaine ;
le composé de **Formule I** est totalement complémentaire du pré-ARNm de tyrosinase humaine, ou partiellement complémentaire du pré-ARNm de tyrosinase humaine avec un ou deux mésappariements du composé entier de **Formule I** ;
S₁, S₂, ···, Sₙ₋₁, Sₙ, T₁, T₂, ···, Tₙ₋₁, et Tn représentent indépendamment deutérido, hydrido, alkyle substitué ou non substitué, ou un radical aryle substitué ou non substitué ;
X et Y représentent indépendamment deutérido, hydrido [H], formyle [H-C(=O)-], aminocarbonyle [NH₂-C(=O)-], aminothiocarbonyle [NH₂-C(=S)-], alkyle substitué ou non substitué, aryle substitué ou non substitué, alkylacyle substitué ou non substitué, arylacyle substitué ou non substitué, alkyloxycarbonyle substitué ou non substitué, aryloxycarbonyle substitué ou non substitué, alkylaminocarbonyle substitué ou non substitué, arylaminocarbonyle substitué ou non substitué, alkylaminothiocarbonyle substitué ou non substitué, arylaminothiocarbonyle substitué ou non substitué, alkyloxythiocarbonyle substitué ou non substitué, aryloxythiocarbonyle substitué ou non substitué, alkylsulfonyle substitué ou non substitué, arylsulfonyle substitué ou non substitué, radical alkylphosphonyle substitué ou non substitué, ou radical arylphosphonyle substitué ou non substitué ;
Z représente hydrido, hydroxy, alkyloxy substitué ou non substitué, aryloxy substitué ou non substitué, amino substitué ou non substitué, alkyle substitué ou non substitué, ou radical aryle substitué ou non substitué ;
B₁, B₂, ···, Bₙ₋₁, et Bₙ sont indépendamment choisis parmi des nucléobases naturelles comprenant adénine, thymine, guanine, cytosine et uracile, et des nucléobases non naturelles ; et,
au moins quatre de B₁, B₂, ···, Bₙ₋₁, et Bₙ sont indépendamment choisis parmi des nucléobases non naturelles avec un radical amino substitué ou non substitué lié par covalence à la partie nucléobase.

2. Dérivé d'acide nucléique peptidique selon la revendication 1, ou sel pharmaceutiquement acceptable de celui-ci :
où
n est un nombre entier entre 10 et 21 ;
le composé de **Formule I** possède au moins un chevauchement complémentaire de 10-mer avec la séquence d'un pré-ARNm de 14-mer de [(5' → 3') UGUACAGAUUGUCU] dans le pré-ARNm de tyrosinase humaine ;
le composé de **Formule I** est totalement complémentaire du pré-ARNm de tyrosinase humaine, ou partiellement complémentaire du pré-ARNm de tyrosinase humaine avec un ou deux mésappariements du composé entier de Formule I ;
S₁, S₂, ···, Sₙ₋₁, Sₙ, T₁, T₂, ···, Tₙ₋₁, et Tn représentent indépendamment deutérido ou radical hydrido ;
X et Y représentent indépendamment deutérido, hydrido, formyle, aminocarbonyle, aminothiocarbonyle, alkyle substitué ou non substitué, aryle substitué ou non substitué, alkylacyle substitué ou non substitué, arylacyle substitué ou non substitué, alkyloxycarbonyle substitué ou non substitué, aryloxycarbonyle substitué ou non substitué, alkylaminocarbonyle substitué ou non substitué, arylaminocarbonyle substitué ou non substitué, alkylaminothiocarbonyle substitué ou non substitué, arylaminothiocarbonyle substitué ou non substitué, alkyloxythiocarbonyle substitué ou non substitué, aryloxythiocarbonyle substitué ou non substitué, alkylsulfonyle substitué ou non substitué, arylsulfonyle substitué ou non substitué, radical alkylphosphonyle substitué ou non substitué, ou radical arylphosphonyle substitué ou non substitué ;
Z représente hydrido, hydroxy, alkyloxy substitué ou non substitué, aryloxy substitué ou non substitué, ou radical amino substitué ou non substitué ;
B₁, B₂, ···, Bₙ₋₁, et Bₙ sont indépendamment choisis parmi des nucléobases naturelles comprenant adénine, thymine, guanine, cytosine et uracile, et des nucléobases non naturelles ;
au moins quatre de B₁, B₂, ···, Bₙ₋₁, et Bₙ sont indépendamment choisis parmi des nucléobases non naturelles représentées par la **Formule II,** la **Formule III,** ou la **Formule IV** : 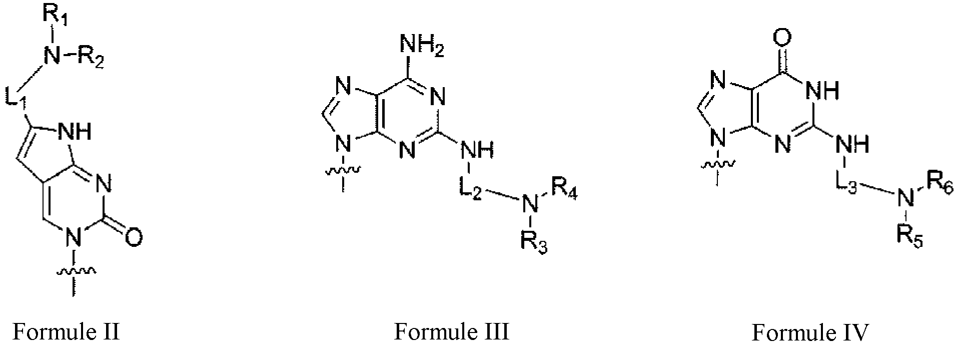
où
R₁, R₂, R₃, R₄, R₅ et R₆ sont indépendamment choisis parmi hydrido, et radical alkyle substitué ou non substitué ;
L₁, L₂ et L₃ sont un lieur covalent représenté par la **Formule V** liant de manière covalente le groupe amino basique à la partie nucléobase : 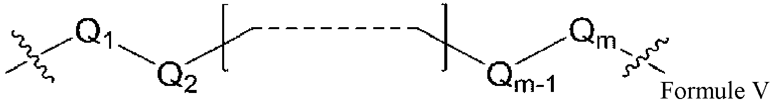
où
Q₁ et Qₘ sont un radical méthylène (-CH₂-) substitué ou non substitué, et Qₘ est directement lié au groupe amino basique ;
Q₂, Q₃, ···, et Qₘ₋₁ sont indépendamment choisis parmi méthylène substitué ou non substitué, oxygène (-O-), soufre (-S-), et radical amino substitué ou non substitué [-N(H)-, ou - N(substituent)-] ; et,
m est un nombre entier entre 1 et 15.

3. Dérivé d'acide nucléique peptidique selon la revendication 2, ou sel pharmaceutiquement acceptable de celui-ci :
où
n est un nombre entier entre 11 et 18 ;
le composé de **Formule I** possède au moins un chevauchement complémentaire de 10-mer avec la séquence d'un pré-ARNm de 14-mer de [(5' → 3') UGUACAGAUUGUCU] dans le pré-ARNm de tyrosinase humaine ;
le composé de **Formule I** est totalement complémentaire du pré-ARNm de tyrosinase humaine ;
S₁, S₂, ···, Sₙ₋₁, Sₙ, T₁, T₂, □, Tₙ₋₁, et Tn sont un radical hydrido ;
X et Y représentent indépendamment hydrido, alkyle substitué ou non substitué, aryle substitué ou non substitué, alkylacyle substitué ou non substitué, arylacyle substitué ou non substitué, alkyloxycarbonyle substitué ou non substitué, ou radical aryloxycarbonyle substitué ou non substitué ;
Z représente un radical amino substitué ou non substitué ;
B₁, B₂, ···, Bₙ₋₁, et Bₙ sont indépendamment choisis parmi des nucléobases naturelles comprenant adénine, thymine, guanine, cytosine et uracile, et des nucléobases non naturelles ;
au moins quatre de B₁, B₂, ···, Bₙ₋₁, et Bₙ sont indépendamment choisis parmi des nucléobases non naturelles représentées par la **Formule II,** la **Formule III,** ou la **Formule IV** ;
R₁, R₂, R₃, R₄, R₅ et R₆ sont indépendamment choisis parmi hydrido, et radical alkyle substitué ou non substitué ;
Q₁ et Qₘ sont un radical méthylène substitué ou non substitué, et Qₘ est directement lié au groupe amino basique ;
Q₂, Q₃, ···, et Qₘ₋₁ sont indépendamment choisis parmi méthylène substitué ou non substitué, oxygène, et radical amino ; et,
m est un nombre entier entre 1 et 11.

4. Dérivé d'acide nucléique peptidique selon la revendication 3, ou sel pharmaceutiquement acceptable de celui-ci :
où
n est un nombre entier entre 11 et 16 ;
le composé de **Formule I** possède au moins un chevauchement complémentaire de 10-mer avec la séquence d'un pré-ARNm de 14-mer de [(5' → 3') UGUACAGAUUGUCU] dans le pré-ARNm de tyrosinase humaine ;
le composé de **Formule I** est totalement complémentaire du pré-ARNm de tyrosinase humaine ;
S₁, S₂, ···, Sₙ₋₁, Sₙ, T₁, T₂, ···, Tₙ₋₁, et Tn sont un radical hydrido ;
X et Y sont indépendamment choisis parmi hydrido, alkylacyle substitué ou non substitué, ou radical alkyloxycarbonyle substitué ou non substitué ;
Z représente un radical amino substitué ou non substitué ;
B₁, B₂, ···, Bₙ₋₁, et Bₙ sont indépendamment choisis parmi des nucléobases naturelles comprenant adénine, thymine, guanine, cytosine et uracile, et des nucléobases non naturelles ;
au moins quatre de B₁, B₂, ···, Bₙ₋₁, et Bₙ sont indépendamment choisis parmi des nucléobases non naturelles représentées par la **Formule II,** la **Formule III,** ou la **Formule IV** ;
R₁, R₂, R₃, R₄, R₅ et R₆ sont indépendamment choisis parmi hydrido, et radical alkyle substitué ou non substitué ;
Q₁ et Qₘ sont un radical méthylène, et Qₘ est directement lié au groupe amino basique ;
Q₂, Q₃, ···, et Qₘ₋₁ sont indépendamment choisis parmi méthylène, oxygène, et radical amino ; et,
m est un nombre entier entre 1 et 9.

5. Le dérivé d'acide nucléique peptidique selon la revendication 4, ou sel pharmaceutiquement acceptable de celui-ci :
où
n est un nombre entier entre 11 et 16 ;
le composé de **Formule I** possède au moins un chevauchement complémentaire de 10-mer avec la séquence d'un pré-ARNm de 14-mer de [(5' → 3') UGUACAGAUUGUCU] dans le pré-ARNm de tyrosinase humaine ;
le composé de **Formule I** est totalement complémentaire du pré-ARNm de tyrosinase humaine ;
S₁, S₂, ···, Sₙ₋₁, Sₙ, T₁, T₂, □, Tₙ₋₁, et Tn sont un radical hydrido ;
X et Y sont indépendamment choisis parmi hydrido, alkylacyle substitué ou non substitué, ou radical alkyloxycarbonyle substitué ou non substitué ;
Z représente un radical amino substitué ou non substitué ;
B₁, B₂, ···, Bₙ₋₁, et Bₙ sont indépendamment choisis parmi des nucléobases naturelles comprenant adénine, thymine, guanine, cytosine et uracile, et des nucléobases non naturelles ;
au moins quatre de B₁, B₂, ···, Bₙ₋₁, et Bₙ sont indépendamment choisis parmi des nucléobases non naturelles représentées par la **Formule II,** la **Formule III,** ou la **Formule IV** ;
R₁, R₃, et R₅ sont un radical hydrido, et R₂, R₄, et R₆ représentent indépendamment hydrido, ou radical alkyle substitué ou non substitué ;
Q₁ et Qₘ sont un radical méthylène, et Qₘ est directement lié au groupe amino basique ; Q₂, Q₃, ···, et Qₘ₋₁ sont indépendamment choisis parmi méthylène, radical oxygène ; et, m est un nombre entier entre 1 et 9.

6. Dérivé d'acide nucléique peptidique selon la revendication 5, ou sel pharmaceutiquement acceptable de celui-ci :
où
n est un nombre entier entre 11 et 16 ;
le composé de **Formule I** possède au moins un chevauchement complémentaire de 10-mer avec la séquence d'un pré-ARNm de 14-mer de [(5' → 3') UGUACAGAUUGUCU] dans le pré-ARNm de tyrosinase humaine ;
le composé de **Formule I** est totalement complémentaire du pré-ARNm de tyrosinase humaine ;
S₁, S₂, ···, Sₙ₋₁, Sₙ, T₁, T₂, ···, Tₙ₋₁, et Tn sont un radical hydrido ;
X et Y sont indépendamment choisis parmi hydrido, alkylacyle substitué ou non substitué, ou radical alkyloxycarbonyle substitué ou non substitué ;
Z représente un radical amino substitué ou non substitué ;
B₁, B₂, ···, Bₙ₋₁, et Bₙ sont indépendamment choisis parmi adénine, thymine, guanine, cytosine et des nucléobases non naturelles ;
au moins cinq de B₁, B₂, ···, Bₙ₋₁, et Bₙ sont indépendamment choisis parmi des nucléobases non naturelles représentées par la **Formule II,** la **Formule III,** ou la **Formule IV ;**
R₁, R₂, R₃, R₄, R₅, et R₆ sont un radical hydrido ;
Q₁ et Qₘ sont un radical méthylène, et Qₘ est directement lié au groupe amino basique ;
Q₂, Q₃, ···, et Qₘ₋₁ sont indépendamment choisis parmi méthylène, et radical oxygène ; et,
m est un nombre entier entre 1 et 9.

7. Dérivé d'acide nucléique peptidique selon la revendication 6, ou sel pharmaceutiquement acceptable de celui-ci :
où
n est un nombre entier entre 11 et 16 ;
le composé de **Formule I** possède au moins un chevauchement complémentaire de 10-mer avec la séquence d'un pré-ARNm de 14-mer de [(5' → 3') UGUACAGAUUGUCU] dans le pré-ARNm de tyrosinase humaine ;
le composé de **Formule I** est totalement complémentaire du pré-ARNm de tyrosinase humaine ;
S₁, S₂, ···, Sₙ₋₁, Sₙ, T₁, T₂, □, Tₙ₋₁, et Tn sont un radical hydrido ;
X est un radical hydrido ;
Y représente alkylacyle substitué ou non substitué, ou radical alkyloxycarbonyle substitué ou non substitué ;
Z représente un radical amino substitué ou non substitué ;
B₁, B₂, ···, Bₙ₋₁, et Bₙ sont indépendamment choisis parmi adénine, thymine, guanine, cytosine et des nucléobases non naturelles ;
au moins cinq de B₁, B₂, ···, Bₙ₋₁, et Bₙ sont indépendamment choisis parmi des nucléobases non naturelles représentées par la **Formule II,** la **Formule III,** ou la **Formule IV ;**
R₁, R₂, R₃, R₄, R₅, et R₆ sont un radical hydrido ;
L₁ représente -(CH₂)₂-O-(CH₂)₂-, -CH₂-O-(CH₂)₂-, -CH₂-O-(CH₂)₃-, -CH₂-O-(CH₂)₄-, ou - CH₂-O-(CH₂)₅-, l'extrémité droite étant directement liée au groupe amino basique ; et,
L₂ et L₃ sont indépendamment choisis parmi -(CH₂)₂-O-(CH₂)₂-, -(CH₂)₃-O-(CH₂)₂-,-(CH₂)₂-O-(CH₂)₃-, -(CH₂)₂-, -(CH₂)₃-, -(CH₂)₄-, -(CH₂)₅-, -(CH₂)₆-, -(CH₂)₇-, et -(CH₂)₈-, l'extrémité droite étant directement liée au groupe amino basique.

8. Dérivé d'acide nucléique peptidique selon la revendication 1, choisi dans le groupe des dérivés d'acide nucléique peptidique indiqués ci-dessous, ou sel pharmaceutiquement acceptable de ceux-ci :
(N→C) Fethoc-CA(5)G-ACA(5)-ATC(1O2)-TG(6)T-A(5)-NH₂ ;
(N→C) Fethoc-AC(1/2)A(5)-GA(5)C-AA(5)T-CTG(6)-C(1/2)C-NH₂ ;
(N→C) Ac-CA(5)G-ACA(5)-ATC(1O2)-TG(6)T-A(5)-NH₂ ;
(N→C) Benzoyl-CA(5)G-ACA(5)-ATC(1O2)-TG(6)T-A(5)-NH₂ ;
(N→C) Piv-CA(5)G-ACA(5)-ATC(1O2)-TG(6)T-A(5)-NH₂ ;
(N→C) Methyl-CA(5)G-ACA(5)-ATC(1O2)-TG(6)T-A(5)-NH₂;
(N→C) n-Propyl-CA(5)G-ACA(5)-ATC(1O2)-TG(6)T-A(5)-NH₂;
(N→C) Fmoc-CA(5)G-ACA(5)-ATC(1O2)-TG(6)T-A(5)-NH₂;
(N→C) p-Toluènesulfonyl-CA(5)G-ACA(5)-ATC(1O2)-TG(6)T-A(5)-NH₂;
(N→C) H-Lys-CA(5)G-ACA(5)-ATC(1O2)-TG(6)T-A(5)-NH₂;
(N→C) Fethoc-Lys-Gly-CA(5)G-ACA(5)-ATC(1O2)-TG(6)T-A(5)-NH₂ ;
(N→C) Fethoc-CA(5)G-ACA(5)-ATC(1O2)-TG(6)T-A(5)-Lys-NH₂ ;
(N→C) Fethoc-Val-Gly-CA(5)G-ACA(5)-ATC(1O2)-TG(6)T-A(5)-NH₂ ;
(N→C) Ac-Arg-CA(5)G-ACA(5)-ATC(1O2)-TG(6)T-A(5)-NH₂ ;
(N→C) Benzyl-CA(5)G-ACA(5)-ATC(102)-TG(6)T-A(5)-NH₂ ;
(N→C) Phenyl-CA(5)G-ACA(5)-ATC(1O2)-TG(6)T-A(5)-NH₂ ;
(N→C) [N-(2-Phenylethyl)amino]carbonyl-CA(5)G-ACA(5)-ATC(1O2)-TG(6)T-A(5)-NH₂;
(N→C) Benzoyl-Leu-CA(5)G-ACA(5)-ATC(1O2)-TG(6)T-A(5)-NH₂ ;
(N→C) Piv-Lys-CA(5)G-ACA(5)-ATC(1O2)-TG(6)T-A(5)-Val-NH₂ ;
(N→C) Fethoc-CA(7)G-AC(2O2)A-A(4)TC(1O2)-TG(6)T-A-NH₂ ;
(N→C) Fethoc-CTG(6)-ACA(5)-ATC(1O2)-TG(6)T-A(5)-NH₂ ;
(N→C) Fethoc-CA(5)G-ATA(5)-ATC(1O2)-TG(5)T-A(5)-NH₂ ;
(N→C) Fethoc-TA(5)G-ACA(5)-ATC(1O2)-TG(6)T-A(5)-NH₂ ;
(N→C) Fethoc-TCA(3)-GAC(1O5)-A(5)AT-C(1O2)TG(5)-TA(5)-NH₂ ;
(N→C) Fethoc-CA(5)G-ACA(5)-ATC(1O2)-TG(6)T-A(5)C-NH₂ ;
(N→C) Fethoc-AG(6)A-CA(5)A-TC(1O2)T-G(6)TA(5)-C-NH₂ ;
(N→C) Fethoc-AG(6)A-CA(5)A-TC(1O2)T-G(6)TA(5)-CA(2O2)A-NH₂ ;
(N→C) Fethoc-AC(1O2)A-GA(6)C-AA(6)T-CTG(6)-TA(6)C(1O2)-AA(6)-NH₂;
(N→C) Fethoc-AC(1O3)T-GA(6)C-TA(6)T-CTG(6)-TAC(1O5)-A(4)A-NH₂ ;
(N→C) Fethoc-GA(6)C-AA(6)T-CTG(6)-TA(6)C(1O2)-AA(6)-NH₂ ;
(N→C) Fethoc-AC(1O2)A-GA(6)C-AA(6)T-CTG(6)-TA(6)C-A(5)AA(2O2)-A-NH₂;
(N→C) Fethoc-AA(6)T-CTG(6)-TA(6)C-A(5)AA(2O2)-A-NH₂ ;
(N→C) Fethoc-AC(1O2)A-GA(6)C-AA(6)T-CTG(6)-TA(6)C-A(5)AA(2O3)-A-NH₂;
(N→C) Fethoc-GC(1O4)T-AC(1O2)A-GA(4)C-AAT-CTG(6)-TA(6)C-NH₂ ;
(N→C) Fethoc-GC(1O2)T-A(3)CA-GA(4)C-AAT-C(1O2)TG-NH₂;
(N→C) Fethoc-GC(1O2)T-A(3)CA-G(2O2)AC-A(5)AT-C(1O2)TG-NH₂; et
(N→C) Fethoc-GC(1O2)T-A(3)CA-GA(4)C-A(2O2)AT-C(1O2)TG-NH₂:
où
A, G, T, et C sont des monomères PNA avec une nucléobase naturelle d'adénine, de guanine, de thymine, et de cytosine, respectivement ;
C(pOq), A(p), A(pOq), G(p), et G(pOq) sont des monomères PNA avec une nucléobase non naturelle représentés par la **Formule VI,** la **Formule VII,** la **Formule VIII,** la **Formule IX,** et la **Formule X,** respectivement ; 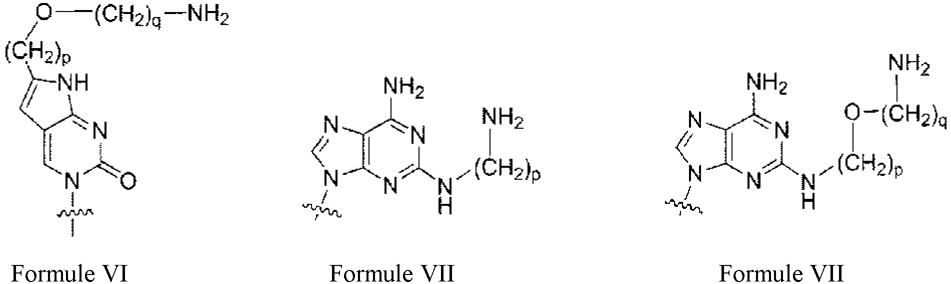 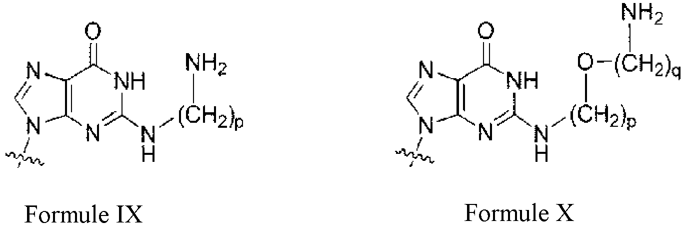
où
p et q sont des nombres entiers ; et,
les abréviations des substituants de terminaison N- et C- sont spécifiquement décrites comme suit : « Fmoc- » est l'abréviation utilisée pour « [(9-fluorényl)méthyloxy]carbonyl- » ; « Fethoc- » pour « [2-(9-fluorényl)éthyl-1-oxy]carbonyle »; « Ac- » pour « acétyl- »; « Benzoyl- » pour « benzenecabonyl- » ; « Piv- » pour « pivalyl- » ; « Méthyl- » pour « méthyl-» ; « n-Propyl- » pour « 1-(n-propyl)- » ; « H- » pour groupe « hydrido- » ; « p-Toluènesulfonyle » pour « (4-méthylbenzène)-1-sulfonyl- » ; « -Lys- » pour résidu d'acide aminé « lysine » ; « -Val-» pour résidu d'acide aminé « valine » ; « -Leu- » pour résidu d'acide aminé « leucine » ; « -Arg-» pour résidu d'acide aminé « arginine » ; « -Gly- » pour résidu d'acide aminé « glycine » ; « [N-(2-Phenylethyl)amino]carbonyl- » pour « [N-1-(2-phenylethyl)amino]carbonyl- » ; « Benzyl- » pour « 1-(phényl)méthyl- » ; « Phényl- » pour « phenyl- » ; « Me- » pour « méthyl- » ; et « -NH₂ » pour groupe « -amino » non substitué.

9. Composition pharmaceutique pour le traitement de maladies ou d'états impliquant l'expression du gène de la tyrosinase humaine, comprenant le dérivé d'acide nucléique peptidique selon la revendication 1, ou un sel pharmaceutiquement acceptable de celui-ci.

10. Composition pharmaceutique pour le traitement d'une hyper-pigmentation, comprenant le dérivé d'acide nucléique peptidique selon la revendication 1, ou un sel pharmaceutiquement acceptable de celui-ci.

11. Dérivé d'acide nucléique peptidique selon la revendication 1 ou sel pharmaceutiquement acceptable de celui-ci destiné à être utilisé dans un procédé de traitement de maladies ou états impliquant l'expression du gène de la tyrosinase humaine, comprenant l'administration dudit dérivé d'acide nucléique peptidique ou dudit sel pharmaceutiquement acceptable de celui-ci.

12. Dérivé d'acide nucléique peptidique selon la revendication 1 ou sel pharmaceutiquement acceptable de celui-ci destiné à être utilisé dans un procédé de traitement d'une hyper-pigmentation, comprenant l'administration dudit dérivé d'acide nucléique peptidique ou dudit sel pharmaceutiquement acceptable de celui-ci.

13. Utilisation du dérivé d'acide nucléique peptidique selon la revendication 1, ou d'un sel pharmaceutiquement acceptable de celui-ci, dans la préparation d'un médicament pour le traitement de maladies ou états impliquant l'expression du gène de la tyrosinase humaine.

14. Utilisation du dérivé d'acide nucléique peptidique selon la revendication 1, ou d'un sel pharmaceutiquement acceptable de celui-ci, dans la préparation d'un médicament pour le traitement d'une hyper-pigmentation.
